# EUROPEAN PATENT SPECIFICATION

(11) **EP 4 262 948 B1**
(45) Date of publication and mention of the grant of the patent: **09.09.2026**
(21) Application number: 21904659.6
(22) Date of filing: 16.12.2021
(51) Int. Cl.: A61M 16/06, A61M 16/08, A61M 16/16, A61M 16/20

(54) **PATIENT INTERFACE**
PATIENTENSCHNITTSTELLE
INTERFACE PATIENT

(30) Priority: 16.12.2020 AU 2020904681; 18.03.2021 AU 2021900782; 20.05.2021 AU 2021901506; 18.08.2021 AU 2021902571; 19.11.2021 AU 2021903730
(43) Date of publication of application: 25.10.2023
(73) Proprietor: ResMed Pty Ltd, Bella Vista, New South Wales 2153 (AU)
(72) Inventor: SCHEINER, Rupert, Christian, Bella Vista, New South Wales 2153 (AU)
(74) Representative: Vossius & Partner Patentanwälte Rechtsanwälte mbB
(86) International application number: PCT/AU2021/051498
(87) International publication number: WO 2022/126190

(56) References cited:
- WO-A1-2009/052560
- WO-A1-2020/165761
- WO-A1-2020/240495
- TW-A- 201 412 351
- US-A1- 2007 144 525
- US-A1- 2017 326 320
- US-A1- 2020 246 572
- US-B1- 6 431 172

## Description

### PATIENT INTERFACE

### 1 CROSS-REFERENCE TO RELATED APPLICATIONS

This application claims priority to Australian Provisional Patent Application No. 2020904681, filed December 16, 2020, Australian Provisional Patent Application No. 2021900782, filed 18 March 2021, Australian Provisional Patent Application No. 2021901506, filed 20 May 2021, Australian Provisional Patent Application No. 2021902571, filed 18 August 2021, Australian Provisional Patent Application No. 2021903730, filed 19 November 2021.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO2 to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design is able to fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.1.3 Pressurised Air Conduit

In one type of treatment system, a flow of pressurised air is provided to a patient interface through a conduit in an air circuit that fluidly connects to the patient interface so that, when the patient interface is positioned on the patient's face during use, the conduit extends out of the patient interface forwards away from the patient's face. This may sometimes be referred to as a "tube down" configuration.

Some patients find such interfaces to be unsightly or to create a feeling of claustrophobia and are consequently deterred from wearing them, reducing patient compliance. Additionally, conduits connecting to an interface at the front of a patient's face may sometimes be vulnerable to becoming tangled up in bed clothes.

### 2.2.3.1.4 Pressurised Air Conduit used for Positioning / Stabilising the Seal-Forming Structure

An alternative type of treatment system which seeks to address these problems comprises a patient interface in which a tube that delivers pressurised air to the patient's airways also functions as part of the headgear to position and stabilise the seal-forming portion of the patient interface at the appropriate part of the patient's face. This type of patient interface may be referred to as having "conduit headgear" or "headgear tubing". Such patient interfaces allow the conduit in the air circuit providing the flow of pressurised air from a respiratory pressure therapy device to connect to the patient interface in a position other than in front of the patient's face. One example of such a treatment system is disclosed in US Patent Publication No. US 2007/0246043, in which the conduit connects to a tube in the patient interface through a port positioned in use on top of the patient's head.

Patient interfaces incorporating headgear tubing may provide some advantages, for example avoiding a conduit connecting to the patient interface at the front of a patient's face, which may be unsightly and obtrusive. However, it is desirable for patient interfaces incorporating headgear tubing to be comfortable for a patient to wear over a prolonged duration when the patient is asleep, form an air-tight and stable seal with the patient's face, while also able to fit a range of patient head shapes and sizes.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 2.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of the present technology comprises a patient interface comprising:
a chassis portion at least partially forming a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure comprising a pair of nasal pillows, each nasal pillow being constructed and arranged to form a seal with a respective naris of the nose of a patient, each nasal pillow having a hole therein such that the flow of air at said therapeutic pressure is delivered to at least the patient's nares, the seal-forming structure constructed and arranged to maintain said therapeutic pressure in the plenum chamber throughout the patient's respiratory cycle in use;
a membrane portion at least partially forming the plenum chamber, the membrane portion connected to the chassis portion and being formed at least partially from a textile material, wherein the nasal pillows are supported on the membrane portion, and the membrane portion is constructed and arranged to be flexible to allow for relative movement between the nasal pillows and the chassis portion in use; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port, or the patient interface is configured to leave the patient's mouth uncovered.

In examples:
- the membrane portion is constructed and arranged to at least partially decouple movement of the nasal pillows from each other;
- the membrane portion is constructed and arranged to allow each of the nasal pillows to move to align to a respective one of the patient's nares in use;
- the membrane portion is constructed and arranged to be inflated upon pressurisation of the plenum chamber to the therapeutic pressure in use;
- the membrane portion is stretchable;
- the membrane portion is constructed and arranged to stretch during inflation upon pressurisation of the plenum chamber to the therapeutic pressure in use;
- the membrane portion is constructed and arranged to be taut in the absence of therapeutic pressure in the plenum chamber;
- the membrane portion is constructed and arranged to inflate to conform to one or more portions of the patient's nose in use;
- the membrane portion is constructed and arranged to inflate to urge each of the nasal pillows towards a respective one of the patient's nares in use;
- the membrane portion is constructed and arranged to resist separation of the nasal pillows from the patient's nares upon movement of the chassis portion in use;
- the membrane portion is configured to engage the patient's lip superior in use;
- the chassis portion is configured to not engage the patient's lip inferior in use;
- the membrane portion comprises a textile layer and an air impermeable layer;
- the textile layer provides an external surface of the membrane portion;
- the plenum chamber comprises a posterosuperior-facing side configured to face posteriorly and superiorly in use, the membrane portion and the seal-forming structure forming a majority of the posterosuperior-facing side;
- the membrane portion and seal-forming structure form substantially all of the posterosuperior-facing side;
- the plenum chamber comprises an anterosuperior-facing side configured to face anteriorly and superiorly in use, the membrane portion forming a majority of the anterosuperior-facing side;
- the plenum chamber comprises a posteroinferior-facing side configured to face posteriorly and inferiorly in use, the membrane portion forming a majority of the posteroinferior-facing side;
- the chassis portion forms a majority of an anteroinferior-facing side of the plenum chamber configured to face anteriorly and inferiorly in use;
- the chassis portion is formed from an elastomeric material;
- the chassis portion is formed from silicone or TPE;
- the chassis portion is configured to not engage the patient's lip superior;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- each of the nasal pillows is formed from an elastomeric material;
- each of the nasal pillows is formed from silicone or TPE;
- each nasal pillow is formed from a single wall;
- the nasal pillows are dual-wall nasal pillows;
- the seal-forming structure further comprises an oral portion configured to form a seal around the patient's mouth such that the flow of air at said therapeutic pressure is delivered to the patient's mouth;
- the membrane portion forms the oral portion of the seal-forming structure;
- the patient interface comprises a nasal cushion module and an oral cushion module, wherein the chassis portion is a nasal chassis portion forming part of the nasal cushion module, and wherein the oral cushion module comprises an oral chassis portion supporting the oral portion of the seal-forming structure;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber;
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient;
- the patient interface further comprises a vent module comprising the vent, the vent module provided to an anterior side of the chassis portion;
- the chassis portion comprises an anterior hole to receive the vent module;
- the vent module is configured to support a diffuser through which the continuous flow of gases exhaled by the patient is able to pass when flowing to ambient; and/or
- the vent module is configured to allow the continuous flow of gases exhaled by the patient from the interior of the plenum chamber to ambient to pass by the diffuser without flowing through the diffuser.

Another aspect of the present technology comprises a patient interface comprising:
a chassis portion at least partially forming a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure supported by the chassis portion and comprising a pair of nasal pillows, each nasal pillow being constructed and arranged to form a seal with a respective naris of the nose of a patient, each nasal pillow having a hole therein such that the flow of air at said therapeutic pressure is delivered to at least the patient's nares, the seal-forming structure constructed and arranged to maintain said therapeutic pressure in the plenum chamber throughout the patient's respiratory cycle in use;
a membrane portion at least partially forming the plenum chamber, the membrane portion being connected to the chassis portion and being formed at least partially from a textile material, wherein the nasal pillows are supported on the membrane portion and the membrane portion is constructed and arranged to inflate to conform to one or more portions of the patient's nose in use; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port, or the patient interface is configured to leave the patient's mouth uncovered.

In examples:
- the membrane portion is constructed and arranged to be flexible to allow for relative movement between the nasal pillows and the chassis portion in use;
- the membrane portion is constructed and arranged to at least partially decouple movement of the nasal pillows from each other;
- the membrane portion is constructed and arranged to allow each of the nasal pillows to move to align to a respective one of the patient's nares in use;
- the membrane portion is constructed and arranged to stretch and/or flex to allow each of the nasal pillows to move to align to the respective one of the patient's nares prior to pressure being applied;
- the membrane portion is constructed and arranged to be inflated upon pressurisation of the plenum chamber to the therapeutic pressure in use;
- the membrane portion is stretchable;
- the membrane portion is constructed and arranged to stretch during inflation upon pressurisation of the plenum chamber to the therapeutic pressure in use;
- the membrane portion is constructed and arranged to be taut in the absence of therapeutic pressure in the plenum chamber;
- the membrane portion is constructed and arranged to inflate to urge each of the nasal pillows towards a respective one of the patient's nares in use;
- the membrane portion is constructed and arranged to resist separation of the nasal pillows from the patient's nares upon movement of the chassis portion in use;
- the membrane portion is configured to engage the patient's lip superior in use;
- the chassis portion is configured to not engage the patient's lip inferior in use;
- the membrane portion comprises a textile layer and an air impermeable layer;
- the textile layer provides an external surface of the membrane portion;
- the plenum chamber comprises a posterosuperior-facing side configured to face posteriorly and superiorly in use, the membrane portion and the seal-forming structure forming a majority of the posterosuperior-facing side;
- the membrane portion and the seal-forming structure form substantially all of the posterosuperior-facing side;
- the plenum chamber comprises an anterosuperior-facing side configured to face anteriorly and superiorly in use, the membrane portion forming a majority of the anterosuperior-facing side;
- the plenum chamber comprises a posteroinferior-facing side configured to face posteriorly and inferiorly in use, the membrane portion forming a majority of the posteroinferior-facing side;
- the chassis portion forms a majority of an anteroinferior-facing side of the plenum chamber configured to face anteriorly and inferiorly in use;
- the chassis portion is formed from an elastomeric material;
- the chassis portion is formed from silicone or TPE;
- the chassis portion is configured to not engage the patient's lip superior;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- each of the nasal pillows is formed from an elastomeric material;
- each of the nasal pillows is formed from silicone or TPE;
- each nasal pillow is formed from a single wall;
- the nasal pillows are dual-wall nasal pillows;
- the seal-forming structure further comprises an oral portion configured to form a seal around the patient's mouth such that the flow of air at said therapeutic pressure is delivered to the patient's mouth;
- the membrane portion forms the oral portion of the seal-forming structure;
- the patient interface comprises a nasal cushion module and an oral cushion module, wherein the chassis portion is a nasal chassis portion forming part of the nasal cushion module, and wherein the oral cushion module comprises an oral chassis portion supporting the oral portion of the seal-forming structure;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber;
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient;
- the patient interface further comprises a vent module comprising the vent, the vent module provided to an anterior side of the chassis portion;
- the chassis portion comprises an anterior hole to receive the vent module;
- the vent module is configured to support a diffuser through which the continuous flow of gases exhaled by the patient is able to pass when flowing to ambient; and/or
- the vent module is configured to allow the continuous flow of gases exhaled by the patient from the interior of the plenum chamber to ambient to pass by the diffuser without flowing through the diffuser.

Another aspect of the present technology comprises a patient interface comprising:
a chassis portion at least partially forming a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use, the seal-forming structure comprising a nasal portion being formed from an elastomeric material;
a membrane portion at least partially forming the plenum chamber, the membrane portion being connected to the chassis portion and being at least partially formed from a textile material, wherein the nasal portion of the seal-forming structure is supported on the membrane portion; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port, or the patient interface is configured to leave the patient's mouth uncovered.

In examples:
- the membrane portion is constructed and arranged to be flexible to allow for relative movement between the seal-forming structure and the chassis portion in use;
- the membrane portion is constructed and arranged to be inflated upon pressurisation of the plenum chamber to the therapeutic pressure in use;
- the membrane portion is stretchable;
- the membrane portion is constructed and arranged to stretch during inflation upon pressurisation of the plenum chamber to the therapeutic pressure in use;
- the membrane portion is constructed and arranged to be taut in the absence of therapeutic pressure in the plenum chamber;
- the seal-forming structure comprises a pair of nasal pillows, each nasal pillow being constructed and arranged to form a seal with a respective naris of the nose of a patient, each nasal pillow having a hole therein to deliver the flow of air at said therapeutic pressure to the patient's nares;
- the membrane portion is constructed and arranged to be flexible to allow for relative movement between the nasal pillows and the chassis portion in use;
- the membrane portion is constructed and arranged to at least partially decouple movement of the nasal pillows from each other;
- the membrane portion is constructed and arranged to allow each of the nasal pillows to move to align to a respective one of the patient's nares in use;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- each of the nasal pillows is formed from an elastomeric material;
- each of the nasal pillows is formed from silicone or TPE;
- each nasal pillow is formed from a single wall;
- the nasal pillows are dual-wall nasal pillows;
- the membrane portion is constructed and arranged to inflate to conform to one or more portions of the patient's nose in use;
- the membrane portion is constructed and arranged to inflate to urge each of the nasal pillows towards a respective one of the patient's nares in use;
- the membrane portion is constructed and arranged to resist separation of the nasal pillows from the patient's nares upon movement of the chassis portion in use;
- the membrane portion is configured to engage the patient's lip superior in use;
- the chassis portion is configured to not engage the patient's lip inferior in use;
- the membrane portion comprises a textile layer and an air impermeable layer;
- the textile layer provides an external surface of the membrane portion;
- the plenum chamber comprises a posterosuperior-facing side configured to face posteriorly and superiorly in use, the membrane portion and the seal-forming structure forming a majority of the posterosuperior-facing side;
- the membrane portion and the seal-forming structure form substantially all of the posterosuperior-facing side;
- the plenum chamber comprises an anterosuperior-facing side configured to face anteriorly and superiorly in use, the membrane portion forming a majority of the anterosuperior-facing side;
- the plenum chamber comprises a posteroinferior-facing side configured to face posteriorly and inferiorly in use, the membrane portion forming a majority of the posteroinferior-facing side;
- the chassis portion forms a majority of an anteroinferior-facing side of the plenum chamber configured to face anteriorly and inferiorly in use;
- the chassis portion is formed from an elastomeric material;
- the chassis portion is formed from silicone or TPE;
- the chassis portion is configured to not engage the patient's lip superior;
- the seal-forming structure further comprises an oral portion configured to form a seal around the patient's mouth such that the flow of air at said therapeutic pressure is delivered to the patient's mouth;
- the membrane portion forms the oral portion of the seal-forming structure;
- the patient interface comprises a nasal cushion module and an oral cushion module, wherein the chassis portion is a nasal chassis portion forming part of the nasal cushion module, and wherein the oral cushion module comprises an oral chassis portion supporting the oral portion of the seal-forming structure;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber;
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient;
- the patient interface further comprises a vent module comprising the vent, the vent module provided to an anterior side of the chassis portion;
- the chassis portion comprises an anterior hole to receive the vent module;
- the vent module is configured to support a diffuser through which the continuous flow of gases exhaled by the patient is able to pass when flowing to ambient; and/or
- the vent module is configured to allow the continuous flow of gases exhaled by the patient from the interior of the plenum chamber to ambient to pass by the diffuser without flowing through the diffuser.

Another aspect of the present technology comprises a patient interface comprising:
a chassis portion at least partially forming a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure supported by the chassis portion and comprising a pair of nasal pillows, each nasal pillow being constructed and arranged to form a seal with a respective naris of the nose of a patient, each nasal pillow having a hole therein such that the flow of air at said therapeutic pressure is delivered to at least the patient's nares, the seal-forming structure constructed and arranged to maintain said therapeutic pressure in the plenum chamber throughout the patient's respiratory cycle in use;
a membrane portion at least partially forming the plenum chamber, the membrane portion being connected to the chassis portion, wherein the nasal pillows are supported on the membrane portion and the membrane portion is formed from a stretchable material able to stretch during inflation upon pressurisation of the plenum chamber to the therapeutic pressure in use; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port, or the patient interface is configured to leave the patient's mouth uncovered.

In examples:
- the membrane portion is constructed and arranged to be flexible to allow the nasal pillows to move with respect to the chassis portion in use;
- the membrane portion is constructed and arranged to at least partially decouple movement of the nasal pillows from each other;
- the membrane portion is constructed and arranged to allow each of the nasal pillows to move to align to a respective one of the patient's nares in use;
- the membrane portion is constructed and arranged to be taut in the absence of therapeutic pressure in the plenum chamber;
- the membrane portion is constructed and arranged to urge each of the nasal pillows towards a respective one of the patient's nares in use upon inflation;
- the membrane portion is constructed and arranged to resist separation of the nasal pillows from the patient's nares upon movement of the chassis portion in use;
- the membrane portion is constructed and arranged to inflate to conform to one or more portions of the patient's nose in use;
- the membrane portion is configured to engage the patient's lip superior in use;
- the chassis portion is configured to not engage the patient's lip inferior in use;
- the membrane portion is at least partially formed from a textile material;
- the membrane portion comprises a textile layer and an air impermeable layer;
- the textile layer provides an external surface of the membrane portion;
- the membrane portion is formed from silicone;
- the membrane portion is less than 0.25mm in thickness;
- the membrane portion is 0.2mm in thickness or less;
- the membrane portion is within the range of 0.1mm-0.19mm in thickness;
- the membrane portion is formed from silicone having a Durometer hardness in a range of D20-D40;
- the plenum chamber comprises a posterosuperior-facing side configured to face posteriorly and superiorly in use, the membrane portion and the seal-forming structure forming a majority of the posterosuperior-facing side;
- the membrane portion and the seal-forming structure form substantially all of the posterosuperior-facing side;
- the plenum chamber comprises an anterosuperior-facing side configured to face anteriorly and superiorly in use, the membrane portion forming a majority of the anterosuperior-facing side;
- the plenum chamber comprises a posteroinferior-facing side configured to face posteriorly and inferiorly in use, the membrane portion forming a majority of the posteroinferior-facing side;
- the chassis portion forms a majority of an anteroinferior-facing side of the plenum chamber configured to face anteriorly and inferiorly in use;
- the chassis portion is formed from an elastomeric material;
- the chassis portion is formed from silicone or TPE;
- the chassis portion is configured to not engage the patient's lip superior;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- each of the nasal pillows is formed from an elastomeric material;
- each of the nasal pillows is formed from silicone or TPE;
- each nasal pillow is formed from a single wall;
- the nasal pillows are dual-wall nasal pillows;
- the seal-forming structure further comprises an oral portion configured to form a seal around the patient's mouth such that the flow of air at said therapeutic pressure is delivered to the patient's mouth;
- the membrane portion forms the oral portion of the seal-forming structure;
- the patient interface comprises a nasal cushion module and an oral cushion module, wherein the chassis portion is a nasal chassis portion forming part of the nasal cushion module, and wherein the oral cushion module comprises an oral chassis portion supporting the oral portion of the seal-forming structure;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber;
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient;
- the patient interface further comprises a vent module comprising the vent, the vent module provided to an anterior side of the chassis portion;
- the chassis portion comprises an anterior hole to receive the vent module;
- the vent module is configured to support a diffuser through which the continuous flow of gases exhaled by the patient is able to pass when flowing to ambient; and/or
- the vent module is configured to allow the continuous flow of gases exhaled by the patient from the interior of the plenum chamber to ambient to pass by the diffuser without flowing through the diffuser.

Another aspect of the present technology comprises a patient interface comprising:
a chassis portion at least partially defining a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure comprising a pair of nasal pillows, each nasal pillow being constructed and arranged to form a seal with a respective naris of the nose of a patient, each nasal pillow having a hole therein such that the flow of air at said therapeutic pressure is delivered to the patient's nares, the seal-forming structure further comprising an oral portion configured to form a seal around the patient's mouth such that the flow of air at said therapeutic pressure is delivered to the patient's mouth, the seal-forming structure constructed and arranged to maintain said therapeutic pressure in the plenum chamber throughout the patient's respiratory cycle in use;
a membrane portion at least partially defining the plenum chamber, the membrane portion connected to the chassis portion and being more flexible than the chassis portion, wherein the nasal pillows of the seal-forming structure are supported on the membrane portion and the membrane portion forms the oral portion of the seal-forming structure; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port.

Another aspect of the present technology comprises a patient interface comprising:
a chassis portion at least partially defining a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways, said seal-forming structure having a hole therein such that the flow of air at said therapeutic pressure is delivered to at least an entrance to the patient's nares, the seal-forming structure constructed and arranged to maintain said therapeutic pressure in the plenum chamber throughout the patient's respiratory cycle in use, the seal-forming structure comprising a nasal portion and an oral portion;
a membrane portion at least partially defining the plenum chamber, the membrane portion connected to the chassis portion and being more flexible than the chassis portion, wherein the nasal portion of the seal-forming structure is connected to a substantially sheetlike nasal portion of the membrane portion, the nasal portion of the membrane portion surrounding the nasal portion of the seal-forming structure to space the nasal portion of the seal-forming structure from the chassis portion, the membrane portion allowing the nasal portion of the seal-forming structure to move with respect to the chassis portion, and wherein an oral portion of the membrane portion forms the oral portion of the seal-forming structure; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port.

In examples:
- the membrane portion is flexible to allow for relative movement between the nasal portion of the seal-forming structure and the chassis portion in use;
- the nasal portion of the seal-forming structure comprises a pair of nasal pillows supported on the membrane portion, each nasal pillow being constructed and arranged to form a seal with a respective naris of the nose of the patient;
- the membrane portion is constructed and arranged to at least partially decouple movement of the nasal pillows from each other;
- the membrane portion is constructed and arranged to allow each of the nasal pillows to move to align to a respective one of the patient's nares in use;
- the membrane portion is constructed and arranged to be inflated upon pressurisation of the plenum chamber to the therapeutic pressure in use;
- the membrane portion is stretchable;
- the membrane portion is constructed and arranged to stretch during inflation upon pressurisation of the plenum chamber to the therapeutic pressure in use;
- the membrane portion is constructed and arranged to be taut in the absence of therapeutic pressure in the plenum chamber;
- the membrane portion is constructed and arranged to inflate to conform to one or more portions of the patient's nose in use;
- the membrane portion is constructed and arranged to inflate to urge each of the nasal pillows towards a respective one of the patient's nares in use;
- the membrane portion is constructed and arranged to resist separation of the nasal pillows from the patient's nares upon movement of the chassis portion in use;
- the membrane portion is formed at least partially from a textile material;
- the membrane portion comprises a textile layer and an air impermeable layer;
- the textile layer provides an external surface of the membrane portion;
- the membrane portion is formed from an elastomeric material;
- the membrane portion is formed from silicone;
- the membrane portion is less than 0.25mm in thickness;
- the membrane portion is 0.2mm in thickness or less;
- the chassis portion and the membrane portion form a nasal portion of the plenum chamber and an oral portion of the plenum chamber;
- the nasal portion of the plenum chamber comprises a posterosuperior-facing side configured to face posteriorly and superiorly in use, the membrane portion and seal-forming structure forming a majority of the posterosuperior-facing side, the nasal pillows being positioned on the posterosuperior-facing side;
- the membrane portion and seal-forming structure form substantially all of the posterosuperior-facing side of the nasal portion of the plenum chamber;
- the nasal portion of the plenum chamber comprises an anterosuperior-facing side configured to face anteriorly and superiorly in use, the membrane portion forming a majority of the anterosuperior-facing side;
- the chassis portion forms a majority of an anterior-facing side of the oral portion of the plenum chamber;
- the chassis portion forms substantially all of the anterior-facing side of the oral portion of the plenum chamber;
- the chassis portion and oral portion of the seal-forming structure form substantially all of the posterior-facing side of the oral portion of the plenum chamber;
- the chassis portion is flexible to at least partially decouple the membrane portion from disruptive forces applied to the chassis portion in use;
- the chassis portion is formed from an elastomeric material;
- the chassis portion is formed from silicone or TPE;
- the chassis portion is shaped to curve from one lateral side of the patient's face in use to the other lateral side of the patient's face, the chassis portion being substantially flush with the patient's cheeks at each lateral side;
- the chassis portion is shaped substantially as a hyperbolic paraboloid or a parabolic cylinder;
- the membrane portion is shaped substantially as a hyperbolic paraboloid or a parabolic cylinder;
- the chassis portion and membrane portion are shaped substantially as hyperbolic paraboloids rotated 90 degrees with respect to each other;
- the patient interface comprises an undercushion attached to the chassis portion and configured to engage an internal surface of the membrane portion to support the membrane portion in use;
- the undercushion is configured to engage a chin region of the membrane portion configured to contact a chin region of the patient's face in use;
- the undercushion is configured to engage cheek regions of the membrane portion configured to contact the patient's cheeks in use;
- the undercushion is configured to engage a nose region of the membrane portion configured to contact the patient's nose in use;
- the undercushion is configured to engage the membrane portion around an entire periphery of the membrane portion;
- the undercushion comprises a pair of wing portions extending inwardly with respect to an outer periphery of the undercushion, each wing portion configured to urge the membrane portion to seal against the patient's face in a region proximate a respective one of the patient's nasal alae;
- the patient interface comprises a pair of ribs each extending between the chassis portion and a respective one of the wing portions to provide support for the respective wing portion;
- the undercushion comprises a rim provided along an outer periphery of the undercushion, the membrane portion being attached to the rim, the rim being structured to space the membrane portion from the undercushion at least at the periphery of the undercushion;
- the rim of the undercushion provides a connection surface at which the membrane portion is connected to the undercushion, the connection surface being proud of the remainder of the undercushion;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber;
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient;
- the positioning and stabilising structure comprises a pair of lower straps, each lower strap configured to be positioned on a respective side of the patient's head inferior to a respective otobasion inferior of the patient's head;
- the lower straps are elastically extendable;
- the lower straps are configured to releasably attach to the chassis portion;
- the positioning and stabilising structure comprises a pair of gas delivery tubes and the chassis portion comprises a pair of openings at which the gas delivery tubes are configured connect to the chassis portion, the openings being sized and structured to receive a flow of air from the gas delivery tubes at the therapeutic pressure for breathing by a patient;
- the positioning and stabilising structure comprises a pair of lower straps each configured to be positioned on a respective side of the patient's head inferior to a respective otobasion inferior of the patient's head;
- the lower straps are elastically extendable;
- the patient interface comprises a pair of lower arms extending from the chassis portion, each lower arm configured to attach to a respective one of the lower straps of the positioning and stabilising structure;
- on each lateral side of the chassis portion, the respective lower arm is connected to the chassis portion immediately adjacent to the respective gas delivery tube;
- the positioning and stabilising structure comprises a pair of upper straps, each upper strap configured to be positioned on a respective side of the patient's head superior to a respective otobasion superior of the patient's head;
- the patient interface comprises a pair of upper arms, each upper arm configured to attach to a respective one of the upper straps;
- each upper arm extends superiorly and posteriorly with respect to the chassis portion;
- each upper arm extends from the chassis portion;
- the patient interface comprises a pair of lower arms extending from the chassis portion, each lower arm configured to attach to a respective one of a pair of lower straps of the positioning and stabilising structure configured to be positioned on a respective side of the patient's head inferior to a respective otobasion inferior of the patient's head, each upper arm extending from a respective one of the lower arms;
- the patient interface further comprises a vent module comprising the vent, the vent module provided to an anterior side of the chassis portion; and/or
- the chassis portion comprises an anterior hole to receive the vent module.

Another aspect of the present technology comprises a patient interface comprising:
a chassis portion at least partially forming a plenum chamber pressurisable to a therapeutic pressure of at least 4 cmH₂O above ambient air pressure, said plenum chamber including a plenum chamber inlet port sized and structured to receive a flow of air at the therapeutic pressure for breathing by a patient;
a seal-forming structure comprising a pair of nasal pillows, each nasal pillow being constructed and arranged to form a seal with a respective naris of the nose of a patient, each nasal pillow having a hole therein such that the flow of air at said therapeutic pressure is delivered to at least the patient's nares, the seal-forming structure constructed and arranged to maintain said therapeutic pressure in the plenum chamber throughout the patient's respiratory cycle in use;
a membrane portion connected to the chassis portion and at least partially forming the plenum chamber, wherein the nasal pillows are supported on the membrane portion and the membrane portion is constructed and arranged to be flexible to allow for relative movement between the nasal pillows and the chassis portion in use, and wherein the membrane portion does not have a predetermined three-dimensional shape in the absence of positive pressure with respect to atmospheric pressure in the plenum chamber; and
a vent to allow a continuous flow of gases exhaled by the patient from an interior of the plenum chamber to ambient, said vent being sized and shaped to maintain the therapeutic pressure in the plenum chamber in use;
wherein the patient interface is configured to allow the patient to breathe from ambient through their mouth in the absence of a flow of pressurised air through the plenum chamber inlet port, or the patient interface is configured to leave the patient's mouth uncovered.

In examples:
- the membrane portion is constructed and arranged to at least partially decouple movement of the nasal pillows from each other;
- the membrane portion is constructed and arranged to allow each of the nasal pillows to move to align to a respective one of the patient's nares in use;
- the membrane portion is constructed and arranged to be inflated upon pressurisation of the plenum chamber to the therapeutic pressure in use;
- the membrane portion is stretchable;
- the membrane portion is constructed and arranged to stretch during inflation upon pressurisation of the plenum chamber to the therapeutic pressure in use;
- the membrane portion is constructed and arranged to be taut in the absence of therapeutic pressure in the plenum chamber;
- the membrane portion is constructed and arranged to inflate to conform to one or more portions of the patient's nose in use;
- the membrane portion is constructed and arranged to inflate to urge each of the nasal pillows towards a respective one of the patient's nares in use;
- the membrane portion is constructed and arranged to resist separation of the nasal pillows from the patient's nares upon movement of the chassis portion in use;
- the membrane portion is configured to engage the patient's lip superior in use;
- the membrane portion is formed at least partially from a textile material;
- the membrane portion comprises a textile layer and an air impermeable layer;
- the textile layer provides an external surface of the membrane portion;
- the membrane portion is formed from an elastomer;
- the membrane portion is formed from silicone;
- the membrane portion is in the form of a sheet connected to the chassis portion at edges of the membrane portion;
- the plenum chamber comprises a posterosuperior-facing side configured to face posteriorly and superiorly in use, the membrane portion and the seal-forming structure forming a majority of the posterosuperior-facing side;
- the membrane portion and seal-forming structure form substantially all of the posterosuperior-facing side;
- the plenum chamber comprises an anterosuperior-facing side configured to face anteriorly and superiorly in use, the membrane portion forming a majority of the anterosuperior-facing side;
- the plenum chamber comprises a posteroinferior-facing side configured to face posteriorly and inferiorly in use, the membrane portion forming a majority of the posteroinferior-facing side;
- the chassis portion forms a majority of an anteroinferior-facing side of the plenum chamber configured to face anteriorly and inferiorly in use;
- the chassis portion is formed from an elastomeric material;
- the chassis portion is formed from silicone or TPE;
- the chassis portion is configured to not engage the patient's lip superior;
- each of the nasal pillows is stalkless;
- each of the nasal pillows comprises a frustoconical portion having a tip and a base wider than the tip, the base being attached directly to the membrane portion;
- each of the nasal pillows is formed from a different material to the membrane portion;
- each of the nasal pillows is formed from an elastomeric material;
- each of the nasal pillows is formed from silicone or TPE;
- the seal-forming structure further comprises an oral portion configured to form a seal around the patient's mouth such that the flow of air at said therapeutic pressure is delivered to the patient's mouth;
- the membrane portion forms the oral portion of the seal-forming structure;
- the patient interface comprises a positioning and stabilising structure to provide a force to hold the seal-forming structure in a therapeutically effective position on the patient's head, the positioning and stabilising structure comprising a tie, the tie being constructed and arranged so that at least a portion overlies a region of the patient's head superior to an otobasion superior of the patient's head in use;
- the positioning and stabilising structure comprises one or more gas delivery tubes configured to provide the flow of air at the therapeutic pressure to the plenum chamber; and/or
- the positioning and stabilising structure comprises a pair of gas delivery tubes and the chassis portion comprises a pair of laterally projecting connection portions configured to connect to the gas delivery tubes and which are sized and structured to receive a flow of air at the therapeutic pressure for breathing by a patient.

One form of the present technology comprises a patient interface for sealed delivery of a flow of air at a continuously positive pressure with respect to ambient air pressure to an entrance to the patient's airways including the patient's nares and mouth, wherein the patient interface is configured to maintain a therapy pressure in a range of about 4 cmH2O to about 30 cmH2O above ambient air pressure in use, throughout the patient's respiratory cycle, while the patient is sleeping, to ameliorate sleep disordered breathing; the patient interface comprising:
a chassis portion formed from a textile material, the chassis portion having a predetermined three-dimensional shape that is maintained throughout the patient's respiratory cycle; and
a seal-forming structure provided to the chassis portion, the seal-forming structure comprising a foam undercushion and a textile membrane portion connected to the foam undercushion and configured to inflate, in use, to form a seal around an entrance to the patient's nares and around the patient's mouth.

In examples:
a) the textile membrane portion forms a seal around anterior and lateral portions of a periphery of the alar base of the patient's nose and from one side of the patient's nose, around the patient's mouth to the opposite side the patient's nose;
b) the textile membrane portion has an outer periphery and an inner periphery, wherein a width of the textile membrane portion, between the inner and outer peripheries, is greatest at a wing portion of the membrane portion configured to seal at the corners of the nose;
c) a connecting portion is provided between opposing wing portions of the textile membrane portion to prevent blowout;
d) the textile membrane portion forms a first seal around the entrance to the patient's nares and a separate second seal around the patient's mouth;
e) the textile membrane portion comprises a first hole for delivery of pressurised air to the patient's nares and a second hole for delivery of pressurised air to the patient's mouth;
f) the textile membrane portion has only two holes;
g) the entire textile membrane portion is integrally formed from a single sheet of material;
h) the foam undercushion comprises a pair of inwardly protruding portions configured to engage the patient's face at the corners of the patient's nose;
i) the chassis portion is provided with a plastically deformable element configured to allow a width of the frame to be adjusted;
j) the patient interface comprises an inlet port connector which extends through an inferior wall portion of the chassis portion, the inlet port connector having a central axis which extends in a substantially inferior direction in use;
k) the foam undercushion is in contact with an outer surface of the inlet port connector;
l) an anterior wall of the chassis portion is in contact with an outer surface of the inlet port connector;
m) the inlet port connector protrudes through the anterior wall of the chassis portion;
n) the chassis portion has a non-zero negative first principal curvature and a substantially zero second principal curvature, wherein the second principal curvature is substantially parallel, in use, to the patient's mid-sagittal plane;
o) an exterior surface of the chassis portion, immediately adjacent the inlet port connector, has a radius which is substantially equal to a radius of an immediately adjacent portion of the exterior of the inlet port connector;
p) the patient interface comprises a pair of superior headgear connectors and a pair of inferior headgear connectors, wherein at least one of the headgear connectors is releasably connected to the chassis portion;
q) the at least one headgear connector is connected to the chassis portion by a snap connector; and/or
r) the patient interface comprises a vent module which is releasably connected to the chassis portion.

Another form of the present technology comprises a patient interface for sealed delivery of a flow of air at a continuously positive pressure with respect to ambient air pressure to an entrance to the patient's airways including the patient's nares and mouth, wherein the patient interface is configured to maintain a therapy pressure in a range of about 4 cmH2O to about 30cmH2O above ambient air pressure in use, throughout the patient's respiratory cycle, while the patient is sleeping, to ameliorate sleep disordered breathing;
said patient interface comprising:
a chassis portion formed from a textile material, the chassis portion having a predetermined three-dimensional shape that is maintained throughout the patient's respiratory cycle; and
a seal-forming structure provided to the chassis portion, the seal-forming structure comprising a foam undercushion and a membrane portion connected to the foam undercushion and configured to inflate, in use, to form a seal around an entrance to the patient's nares and around the patient's mouth,
wherein the patient interface comprises an inlet port connector which extends through an inferior wall portion of the chassis portion, the inlet port connector having a central axis which extends in a substantially inferior direction in use.

In examples:
a) the foam undercushion is in contact with an outer surface of the inlet port connector;
b) an anterior wall of the chassis portion is in contact with an outer surface of the inlet port connector;
c) the inlet port connector protrudes through the anterior wall of the chassis portion;
d) the chassis portion has a non-zero negative first principal curvature and a substantially zero second principal curvature, wherein the second principal curvature is substantially parallel, in use, to the patient's sagittal plane;
e) an exterior surface of the chassis portion, immediately adjacent the inlet port connector, has a radius which is substantially equal to a radius of an immediately adjacent portion of the exterior of the connection port;
f) the patient interface comprises a pair of superior headgear connectors and a pair of inferior headgear connectors, wherein at least one of the headgear connectors is releasably connected to the chassis portion;
g) the at least one headgear connector is connected to the chassis portion by a snap connector;
h) the patient interface comprises a vent module which is releasably connected to the chassis portion; and/or
i) the membrane portion is formed from a textile.

One form of the present technology comprises a patient interface comprising:
a chassis portion formed from a flexible material and partially forming a plenum chamber pressurisable to a therapeutic pressure of at least 6 cmH2O above ambient air pressure, wherein the chassis portion has a non-zero negative first principal curvature and a substantially zero second principal curvature, wherein the second principal curvature is substantially parallel, in use, to the patient's sagittal plane;
an inlet port sized and structured to receive a flow of air at the therapeutic pressure for breathing by the patient;
a pair of superior headgear connectors connected to the chassis portion and a pair of inferior headgear connectors connected to the chassis portion;
a seal-forming structure provided to the chassis portion and partially forming the plenum chamber, the seal-forming structure being constructed and arranged to form a seal with a region of the patient's face surrounding an entrance to the patient's airways, the seal-forming structure having at least one hole therein such that the flow of air at the therapeutic pressure is delivered to an entrance to the patient's nares and the patient's mouth, the seal-forming structure constructed and arranged to maintain said therapeutic pressure in the plenum chamber throughout the patient's respiratory cycle in use,
wherein the seal-forming structure comprises an undercushion formed from foam and a textile membrane portion connected to the undercushion and configured to inflate, in use, to form a seal around anterior and lateral portions of a periphery of the alar base of the patient's nose, the seal-forming structure further configured to form a seal around the patient's mouth, and
wherein the chassis portion is configured to be flexible such that the magnitude of the first principal curvature can be varied when the patient interface is donned.

In examples:
a) the chassis portion flexes such that the first principal curvature has a larger magnitude when donned by a patient with a narrow face than when donned by a patient having a relatively wider face;
b) the chassis portion is formed from a polymer;
c) the chassis portion is formed from biaxially-oriented polyethylene terephthalate, for example Mylar ^{™};
d) the chassis portion is less than 0.5mm thick, for example substantially 0.25mm thick;
e) the chassis portion is formed from a sheet of material;
f) the chassis portion is stamped from a sheet of material;
g) the chassis portion is moulded to shape;
h) a patient facing side of the undercushion is configured to hold the textile membrane portion substantially taut when the patient interface is not in use;
i) the undercushion has a substantially "C" shaped cross-section;
j) a non-patient-facing side of the undercushion comprises a connection formation configured to connect the undercushion to the chassis portion;
k) the connection formation comprises a channel configured to receive an edge of the chassis portion;
1) the channel extends around an entire periphery of the non-patient-facing side of the undercushion;
m) the engagement between the channel and the chassis portion forms a seal;
n) the undercushion comprises a first portion of a snap fastener and the chassis portion comprises a second portion of the snap fastener;
o) the undercushion comprises a rigid clip formation;
p) the undercushion is formed from a polyurethane, for example a thermoplastic polyurethane, or from a soft thermoplastic elastomer;
q) the textile membrane portion is attached to the undercushion around an outer periphery of the textile membrane portion;
r) the textile membrane portion is bonded to the undercushion;
s) the textile membrane portion comprises a first hole through which air can flow to both the patient's nares, in use;
t) the textile membrane portion comprises a second hole through which air can flow to the patient's mouth, in use, wherein the textile membrane portion extends between the first and second holes;
u) the textile membrane portion comprises an air impermeable coating on a non-patient facing side thereof;
v) the air impermeable coating comprises a silicone layer;
w) the silicone layer is substantially 0.05mm thick;
x) the total thickness of the textile membrane portion is substantially 0.3mm;
y) the textile membrane portion is formed from a single sheet of textile;
z) each of the superior headgear connectors comprises a curved arm;
aa) each of the inferior headgear connectors comprises a magnetic connector; and/or
ab) the chassis portion, seal-forming structure and headgear connectors have a combined weight of 45g or less;

Another form of the present technology comprises a patient interface comprising:
a chassis portion formed from a flexible material and partially forming a plenum chamber pressurisable to a therapeutic pressure of at least 6 cmH2O above ambient air pressure,
an inlet port sized and structured to receive a flow of air at the therapeutic pressure for breathing by the patient;
a pair of superior headgear connectors connected to the chassis portion and a pair of inferior headgear connectors connected to the chassis portion;
a seal-forming structure releasably connectable to the chassis portion and partially forming the plenum chamber, the seal-forming structure being constructed and arranged to form a seal with a region of the patient's face surrounding an entrance to the patient's airways, the seal-forming structure having at least one hole therein such that the flow of air at the therapeutic pressure is delivered to an entrance to the patient's nares and the patient's mouth, the seal-forming structure constructed and arranged to maintain said therapeutic pressure in the plenum chamber throughout the patient's respiratory cycle in use,
wherein the seal-forming structure comprises an undercushion formed from foam and a textile membrane portion connected to the undercushion and configured to inflate, in use, to form a seal around anterior and lateral portions of a periphery of the alar base of the patient's nose, the seal-forming structure further configured to form a seal around the patient's mouth.

In examples:
a) a non-patient-facing side of the undercushion comprises a connection formation configured to connect the undercushion to the chassis portion;
b) the connection formation comprises a channel configured to receive an edge of the chassis portion;
c) the channel extends around an entire periphery of the non-patient-facing side of the undercushion;
d) the engagement between the channel and the chassis portion forms a seal;
e) the undercushion comprises a first portion of a snap fastener and the chassis portion comprises a second portion of the snap fastener;
f) the undercushion comprises a rigid clip formation;
g) the undercushion is formed from a polyurethane, for example a thermoplastic polyurethane, or from a soft thermoplastic elastomer;
h) a patient facing side of the undercushion is configured to hold the textile membrane portion substantially taut when the interface is not in use;
i) the undercushion has a substantially "C" shaped cross-section;
j) the textile membrane portion is attached to the undercushion around an outer periphery of the textile membrane portion;
k) the textile membrane portion is bonded to the undercushion;
l) the textile membrane portion comprises a first hole through which air can flow to both the patient's nares, in use;
m) the textile membrane portion comprises a second hole through which air can flow to the patient's mouth, in use, wherein the textile membrane portion extends between the first and second holes;
n) the textile membrane portion comprises an air impermeable coating on a non-patient facing side thereof;
o) the air impermeable coating comprises a silicone layer;
p) the silicone layer is substantially 0.05mm thick;
q) the total thickness of the textile membrane portion is substantially 0.3mm;
r) the textile membrane portion is formed from a single sheet of textile; and/or
s) the chassis portion has a non-zero negative first principal curvature and a substantially zero second principal curvature, wherein the second principal curvature is substantially parallel, in use, to the patient's sagittal plane.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.
Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

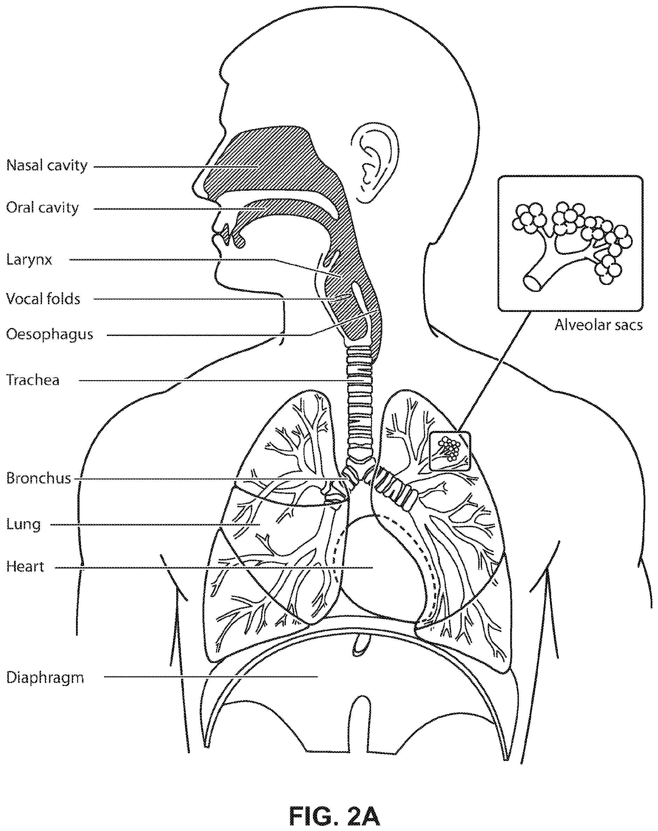

Fig. 2A shows an overview of a human respiratory system including the nasal and oral cavities, the larynx, vocal folds, oesophagus, trachea, bronchus, lung, alveolar sacs, heart and diaphragm.

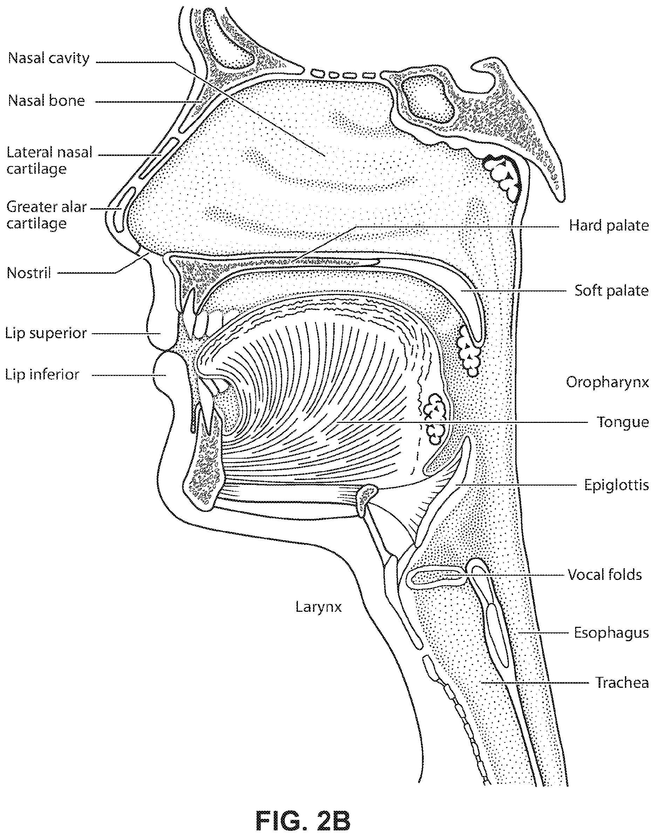

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

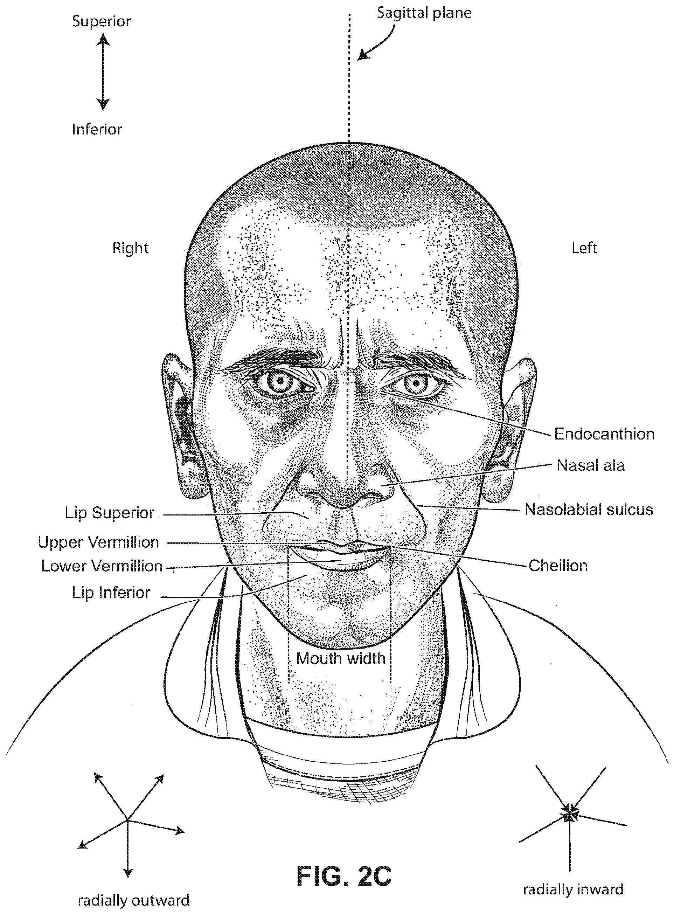

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

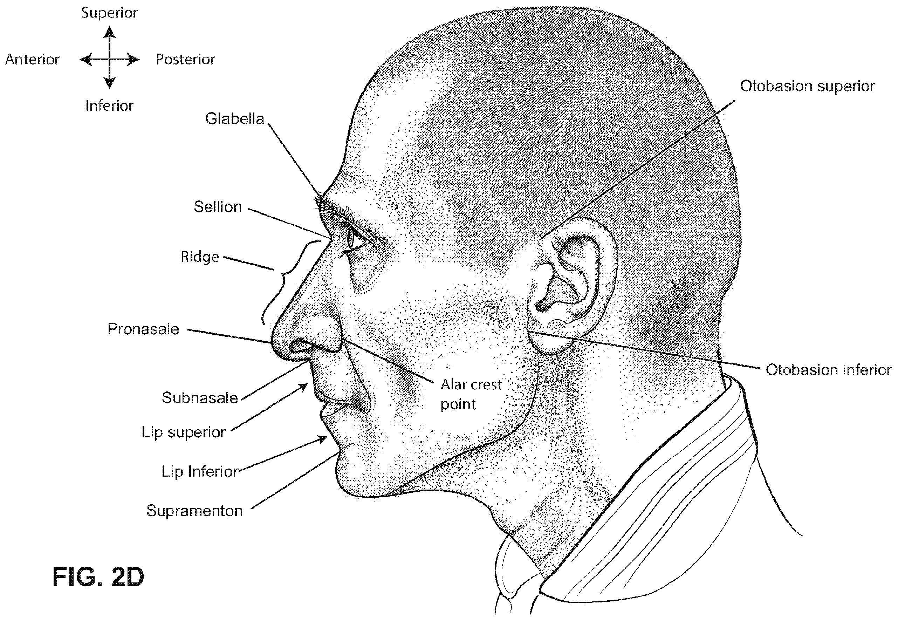

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

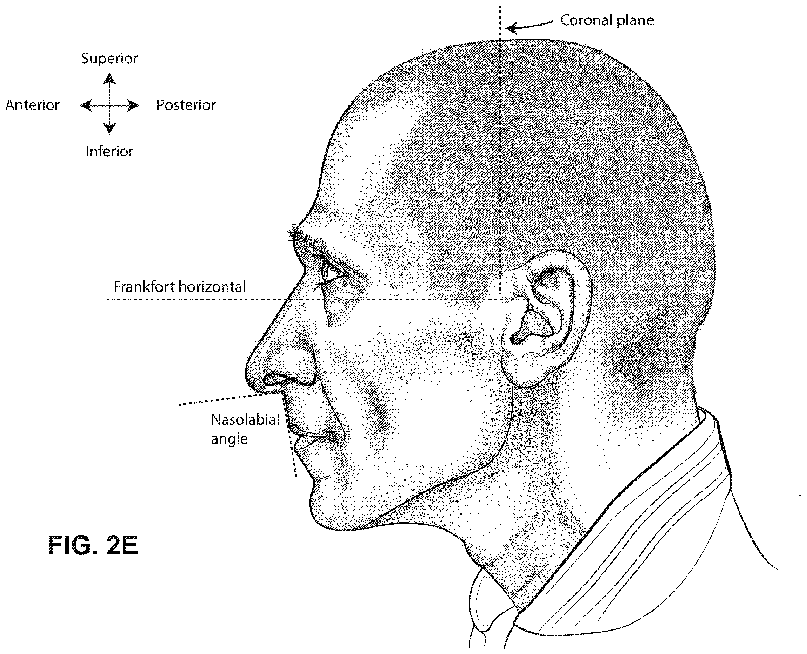

Fig. 2E is a further side view of a head. The approximate locations of the Frankfort horizontal and nasolabial angle are indicated. The coronal plane is also indicated.

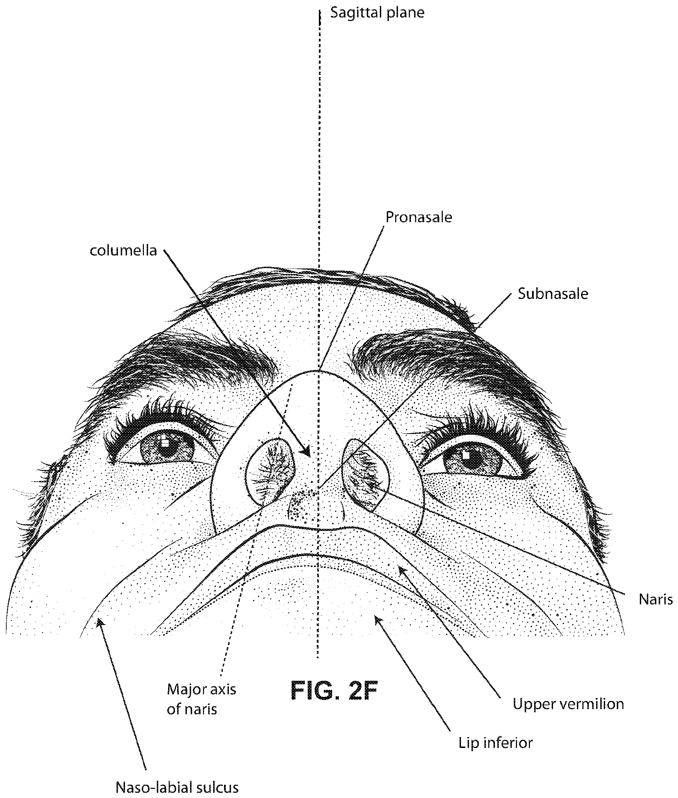

Fig. 2F shows a base view of a nose with several features identified including naso-labial sulcus, lip inferior, upper Vermilion, naris, subnasale, columella, pronasale, the major axis of a naris and the midsagittal plane.

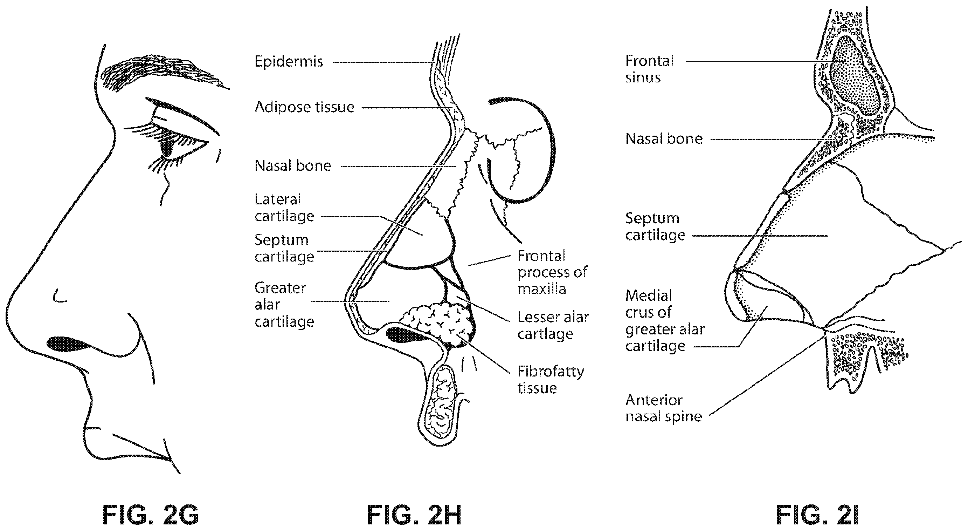

Fig. 2G shows a side view of the superficial features of a nose.

Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.

Fig. 2I shows a medial dissection of a nose, approximately several millimeters from the midsagittal plane, amongst other things showing the septum cartilage and medial crus of greater alar cartilage.

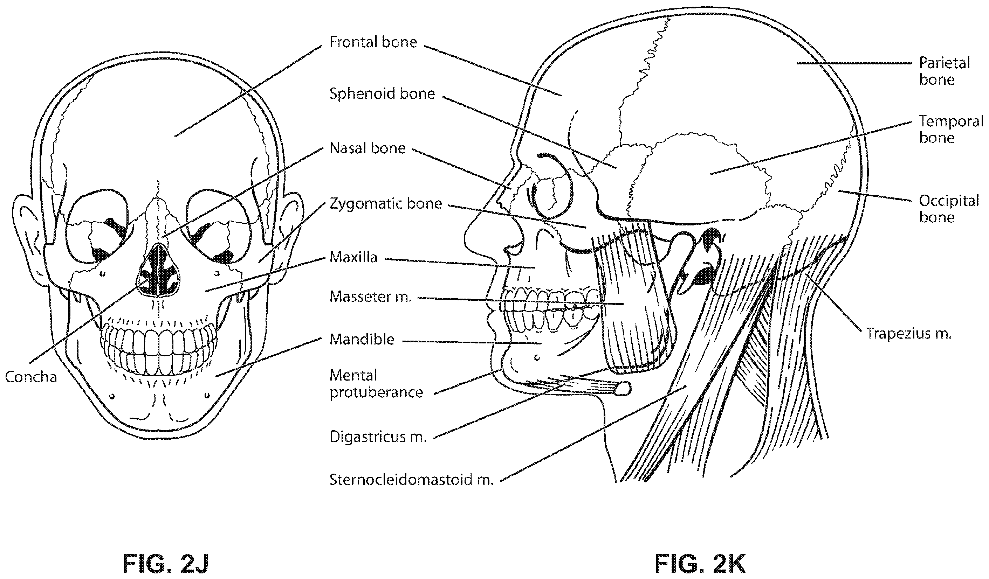

Fig. 2J shows a front view of the bones of a skull including the frontal, nasal and zygomatic bones. Nasal concha are indicated, as are the maxilla, and mandible.

Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

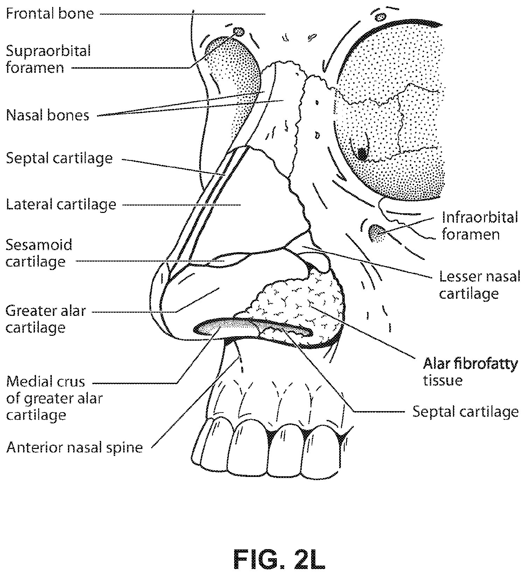

Fig. 2L shows an anterolateral view of a nose.

### 4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 3I, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

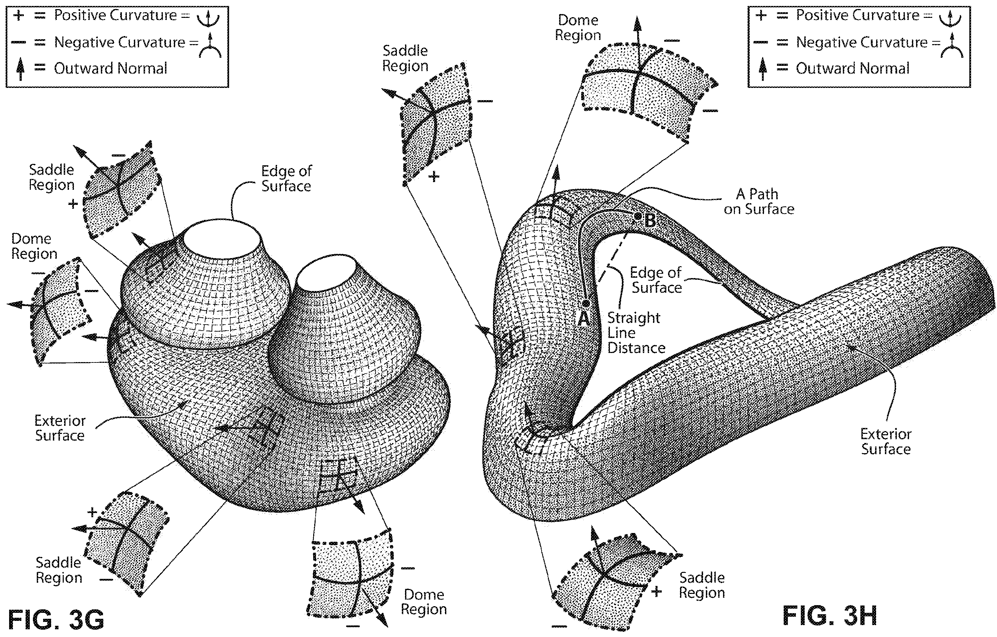

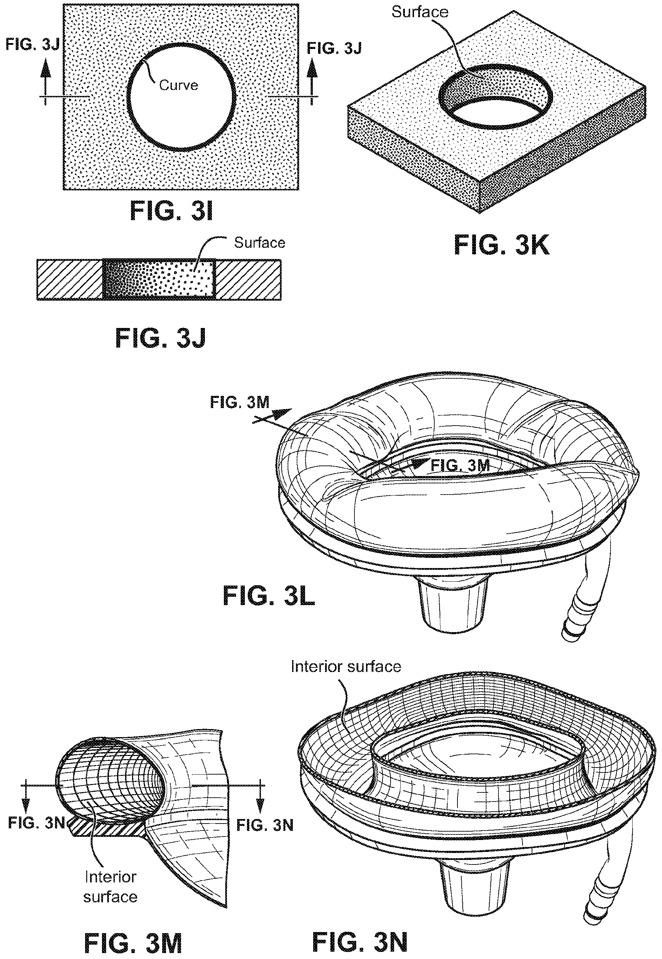

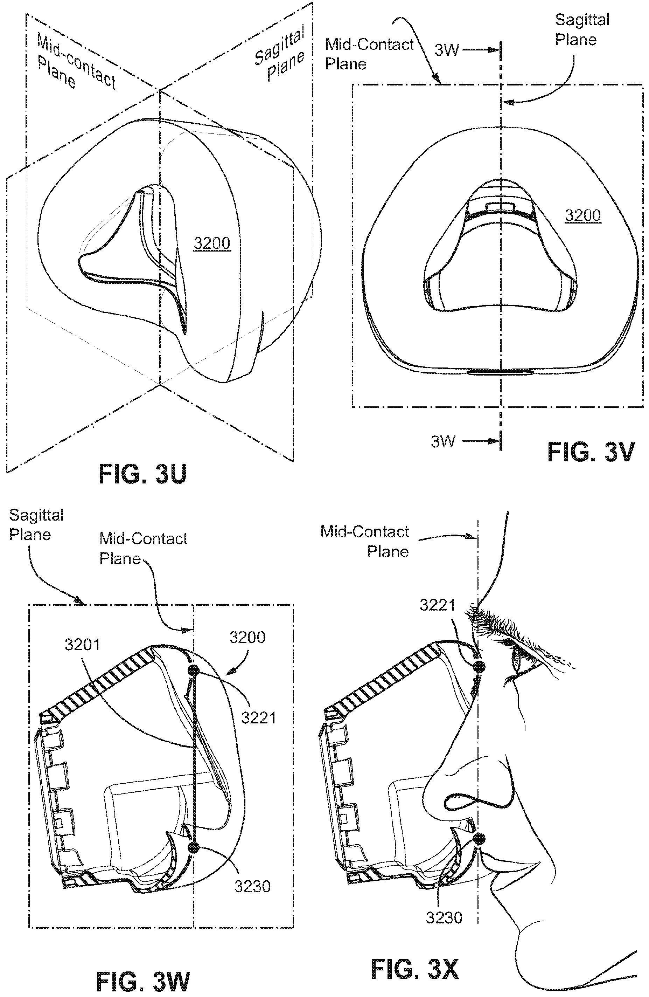

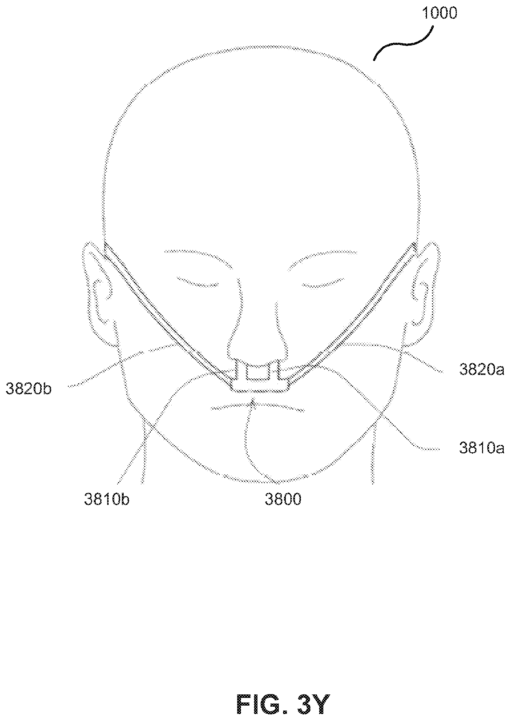

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3201 which lies on the sagittal plane and just touches the cushion of the plenum chamber at two points on the sagittal plane: a superior point 3221 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3221 sits approximately on the sellion, while the inferior point 3230 sits on the lip superior.

Fig. 3Y shows a patient interface in the form of a nasal cannula in accordance with one form of the present technology.

Fig. 3Z shows a patient interface having conduit headgear in accordance with one form of the present technology.

### 4.4 RPT DEVICE

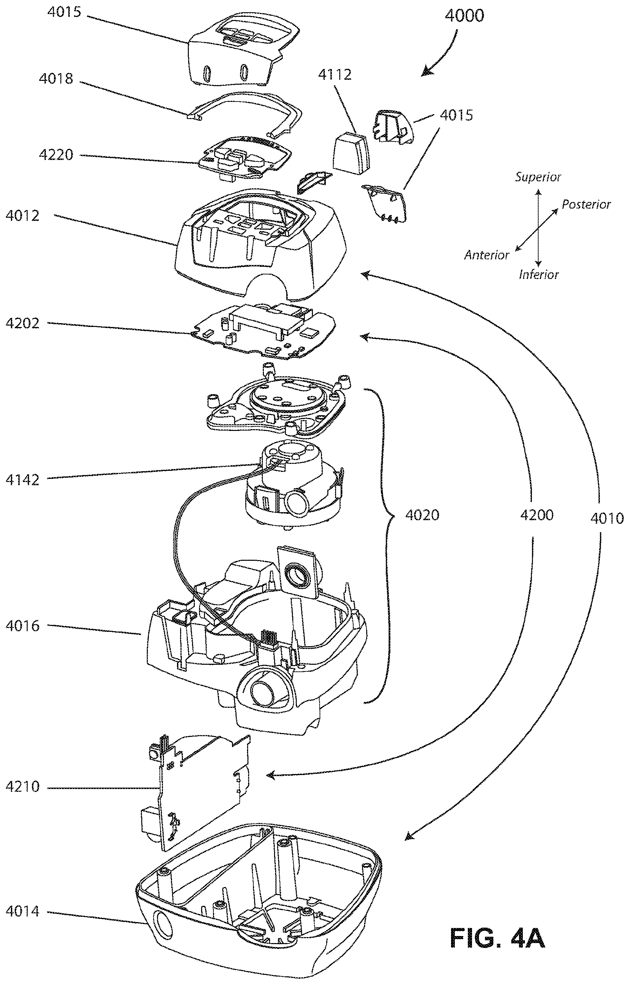

Fig. 4A shows an RPT device in accordance with one form of the present technology.

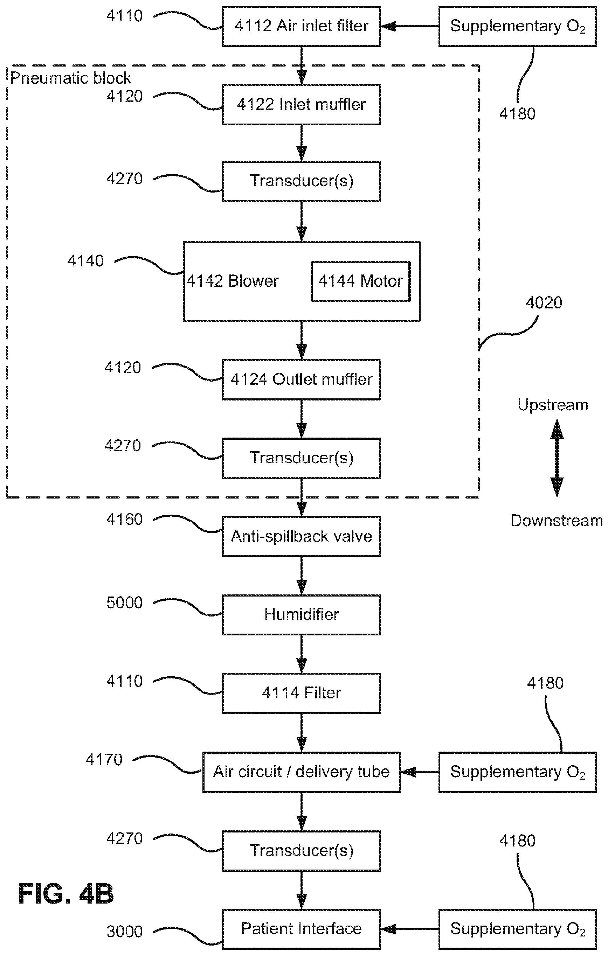

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 HUMIDIFIER

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

### 4.6 BREATHING WAVEFORMS

Fig. 6A shows a model typical breath waveform of a person while sleeping.

### 4.7 FURTHER DRAWINGS OF A PATIENT INTERFACE ACCORDING TO AN EXAMPLE OF THE PRESENT TECHNOLOGY

Fig. 7 is a perspective view illustration of a patient interface according to one example of the present technology.

Fig. 8 is an anterosuperior view illustration of the patient interface shown in Fig. 7.

Fig. 9 is a posterosuperior view illustration of the patient interface shown in Fig. 7.

Fig. 10 is an anterior view illustration of the patient interface shown in Fig. 7.

Fig. 11 is a posterior view illustration of the patient interface shown in Fig. 7.

Fig. 12 shows the patient interface shown in Fig. 7 when worn by a patient prior to pressurisation of a plenum chamber of the patient interface.

Fig. 13 shows the patient interface shown in Fig. 7 when worn by a patient and when the plenum chamber is pressurised.

Fig. 14 shows a superior view of a patient interface according to another example of the present technology.

Fig. 15 shows a posterosuperior view of the patient interface shown in Fig. 14.

Fig. 16 shows an anterosuperior view of the patient interface shown in Fig. 14.

Fig. 17 shows the patient interface shown in Fig. 14 when worn by a patient and when the plenum chamber is pressurised.

Fig. 18 shows a posterior perspective view of a patient interface according to another example of the present technology.

Fig. 19 shows an anterior perspective view of the patient interface shown in Fig. 18.

Fig. 20 shows an anterosuperior perspective view of the patient interface shown in Fig. 18.

Fig. 21 shows an anterosuperior perspective view of a patient interface according to another example of the present technology.

Fig. 22 shows a posterosuperior perspective view of the patient interface shown in Fig. 21.

Fig. 23 shows an exploded view of the patient interface shown in Fig.21.

Fig. 24 shows another exploded view of the patient interface shown in Fig. 21.

Fig. 25 is an anterosuperior perspective view of a patient interface according to another example of the present technology.

Fig. 26 is a posterior perspective view of the patient interface shown in Fig. 25.

Fig. 27 is an anterior perspective view of the patient interface shown in Fig. 25 in use on a patient.

Fig. 28 is an anterosuperior perspective view of the patient interface shown in Fig. 25 in use on a patient.

Fig. 29 is a superior perspective view of the patient interface shown in Fig. 25 in use on a patient.

Fig. 30 is a posteroinferior perspective view of the patient interface shown in Fig. 25.

Fig. 31 is a posterior perspective view of the patient interface shown in Fig. 25 with membrane portion removed.

Fig. 32 is a posterolateral perspective view of the patient interface shown in Fig. 25 with membrane portion removed.

Fig. 33 is an anterosuperior perspective view of a patient interface according to another example of the present technology.

Fig. 34 is a posterior perspective view of the patient interface shown in Fig. 33.

Fig. 35 is an anterior perspective view of the patient interface shown in Fig. 33.

Fig. 36 is an anterosuperior perspective view of the patient interface shown in Fig. 33 in use on a patient.

Fig. 37 is an anterolateral perspective view of the patient interface shown in Fig. 33 in use on a patient.

Fig. 38 is a lateral perspective view of a patient interface according to another example of the present technology in use on a patient.

Fig. 39 is an anterolateral perspective view of a patient interface according to another example of the present technology in use on a patient.

Fig. 40 is an anterolateral perspective view of a patient interface according to another example of the present technology.

Fig. 41 is an anterolateral perspective view of a cushion module of the patient interface shown in Fig. 40.

Fig. 42 is a superior perspective view of the cushion module of the patient interface shown in Fig. 40.

Fig. 43 is a posterosuperior perspective view of the cushion module of the patient interface shown in Fig. 40.

Fig. 44 is an anterolateral perspective view of the cushion module of the patient interface shown in Fig. 40.

Fig. 45 is a superior perspective view of the cushion module of the patient interface shown in Fig. 40.

Fig. 46 is a lateral perspective view of the patient interface shown in Fig. 40 in use on a patient.

Fig. 47 shows a front perspective view of a patient interface in accordance with one form of the present technology.

Fig. 48 shows a front perspective view of a patient interface in accordance with another form of the technology.

Fig. 49 shows a rear view of the patient interface of Fig. 8.

Fig. 50 shows a blank of a frame of a patient interface in accordance with one form of the present technology.

Fig. 51 shows the blank of Fig. 50 folded and joined to form a frame.

Fig 52 shows a foam undercushion of a patient interface in accordance with one form of the present technology prior to mounting to a frame.

Fig. 53 shows a rear perspective view of the patient interface of Fig. 48 with the textile membrane removed to show the undercushion.

Fig. 54 shows one form of textile membrane.

Fig. 55 shows a view from below of the patient interface of Fig. 48.

Fig. 56 shows a perspective view of a patent interface according to one form of the technology.

Fig. 57 is a diagrammatic perspective view of a frame according to one form of the technology.

Fig. 58 is a front view of the patent interface of Fig. 57.

Fig. 59 is a rear view of the patient interface of Fig. 57.

Fig. 60 is a rear view of the patient interface of Fig. 57 flexed into a narrow configuration.

Fig. 61 is a diagrammatic partial cross-section through plane B-B.

Fig. 62 is a diagrammatic perspective view of a frame and an engaging portion of an undercushion according to one form of the technology.

Fig 63 is an enlarged partial view of the frame and engaging portion of the undercushion of Fig. 62.

Fig 64 is an enlarged view of a connection between a frame and an engaging portion of an undercushion according to one form of the technology.

Fig 65 is an enlarged view of a connection between a frame and an engaging portion of an undercushion according to one form of the technology.

Fig. 66 is a diagrammatic cross-section view through plane A-A.

Fig. 67 is a rear perspective view of the patent interface of Fig. 7.

Fig. 68 is a front view of a foam undercushion of one form of the technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

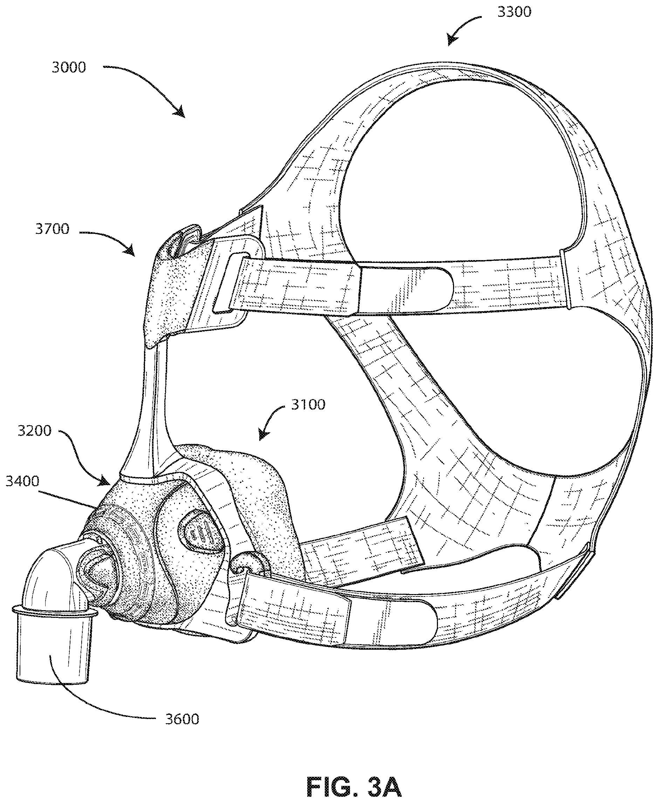

A non-invasive patient interface 3000, such as that shown in Fig. 3A, in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

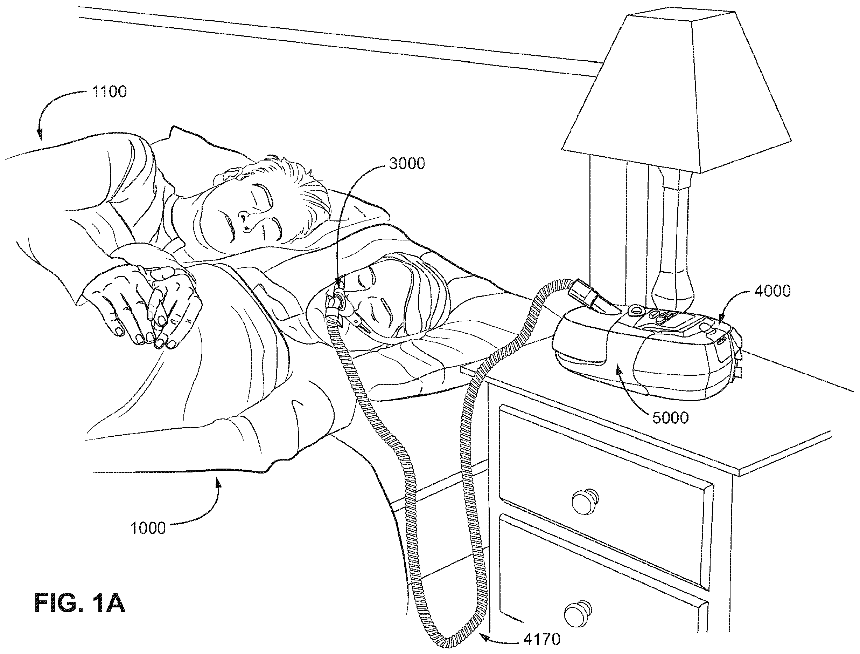

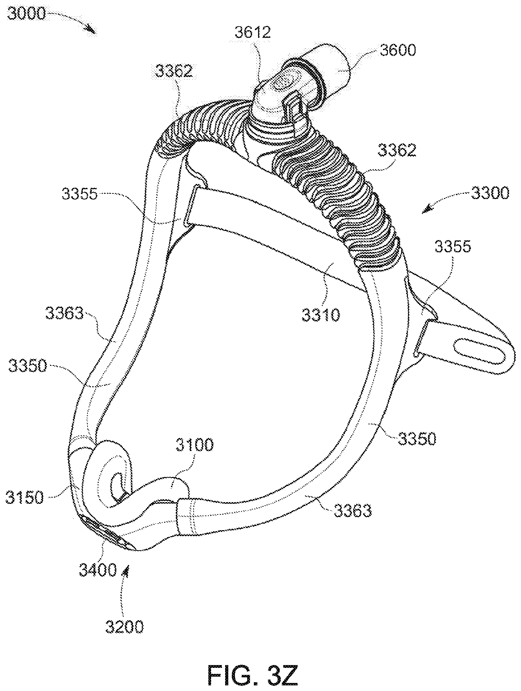

As shown in Fig. 3Z, a non-invasive patient interface 3000 in accordance with another aspect of the present technology comprises the following functional aspects: a seal-forming structure 3100, a plenum chamber 3200, a positioning and stabilising structure 3300, a vent 3400 and one form of connection port 3600 for connection to an air circuit (such as the air circuit 4170 shown in Figs. 1A-1C). The plenum chamber 3200 may be formed of one or more modular components in the sense that it or they can be replaced with different components, for example components of a different size.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 5.3.1 Seal-forming structure

The patient interface 3000 may comprise a seal-forming structure 3100. The seal-forming structure 3100 may be constructed and arranged to form a seal with a region of the patient's face surrounding an entrance to the patient's airways. Furthermore, the seal-forming structure 3100 may have a hole therein such that the flow of air at said therapeutic pressure is delivered to at least an entrance to the patient's nares in use. The seal-forming structure 3100 may be constructed and arranged to maintain the therapeutic pressure in the plenum chamber 3200 throughout the patient's respiratory cycle in use.

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber. In other forms the seal-forming structure 3100 comprises a foam undercushion 3110 and a textile membrane portion 3220, as described further below.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head. However, examples of the technology may be suitable for a large range of heads, and so may be used by patients having a relatively large head and a relatively small head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

In one example of nasal pillows, at least a portion of the frusto-cone of each nasal pillow may be shaped and dimensioned to enter the corresponding naris of the patient. In another example of nasal pillows, the frusto-cone of each nasal pillow may be shaped and dimensioned so as not to enter the corresponding naris of the patient. Each nasal pillow may be configured to seal against portions of the patient's nose defining a respective naris, including the patient's columella and a respective nasal ala.

In some examples of nasal pillows, each nasal pillow may be stalkless. The frusto-cone of each nasal pillow may be attached directly to a portion of the patient-interface 3000 defining a plenum chamber 3200.

### 5.3.1.7 Nasal Mask

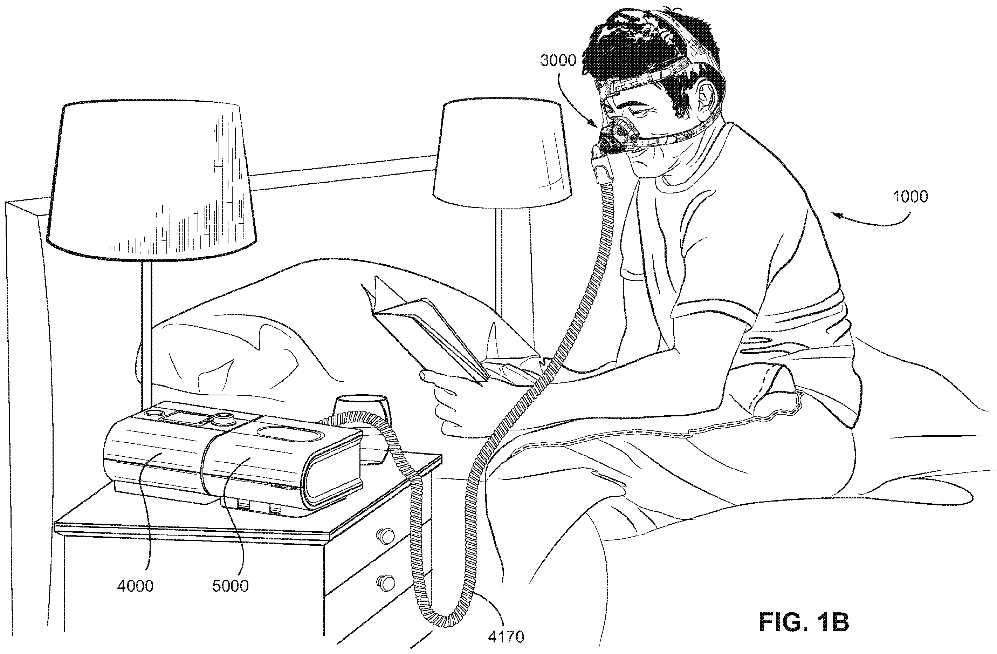

In one form, the non-invasive patient interface 3000 comprises a seal-forming structure 3100 that forms a seal in use to an upper lip region (e.g. the lip superior), to the patient's nose bridge or at least a portion of the nose ridge above the pronasale, and to the patient's face on each lateral side of the patient's nose, for example proximate the patient's nasolabial sulci. The patient interface 3000 shown in Fig. 1B has this type of seal-forming structure 3100. This patient interface 3000 may deliver a supply of air or breathable gas to both nares of patient 1000 through a single orifice. This type of seal-forming structure 3100 may be referred to as a "nasal cushion" and a patient interface 3000 having such a seal-forming structure 3100 may be identified as a "nasal mask".

### 5.3.1.8 Full face Mask

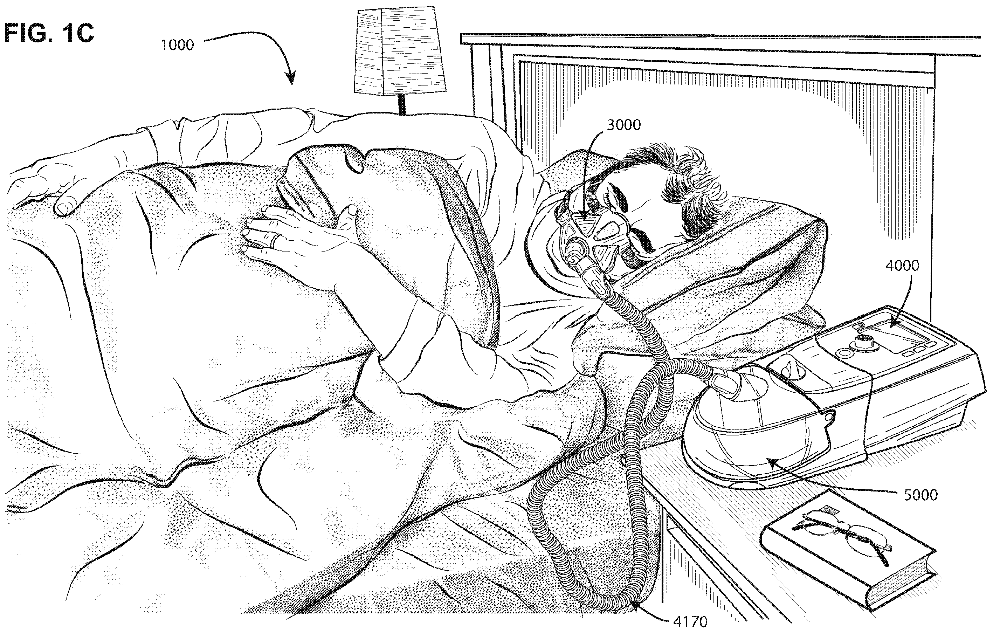

In one form the patient interface 3000 comprises a seal-forming structure 3100 that forms a seal in use on a patient's chin-region (which may include the patient's lip inferior and/or a region directly inferior to the lip inferior), to the patient's nose bridge or at least a portion of the nose ridge superior to the pronasale, and to cheek regions of the patient's face. The patient interface 3000 shown in Fig. 1C is of this type. This patient interface 3000 may deliver a supply of air or breathable gas to both nares and mouth of patient 1000 through a single orifice. This type of seal-forming structure 3100 may be referred to as a "full face cushion" and the patient interface 3000 may be identified as a "full-face mask".

### 5.3.1.9 Ultracompact full-face mask

In one form the patient interface 3000 comprises a seal-forming structure 3100 that forms a seal in use on a patient's chin region (which may include the patient's lip inferior and/or a region directly inferior to the lip inferior), to an inferior and or anterior surface of the patient's pronasale and to the patient's face on each lateral side of the patient's nose, for example proximate the nasolabial sulci. The seal-forming structure 3100 may also form a seal against a patient's lip superior. A patient interface 3000 having this type of seal-forming structure may have a single opening configured to deliver a flow of air or breathable gas to both nares and mouth of a patient, may have an oral hole configured to provide air or breathable gas to the mouth and a nasal hole configured to provide air or breathable gas to the nares, or may have an oral hole for delivering air to the patient's mouth and two nasal holes for delivering air to respective nares. This type of patient interface 3000 may be known as an ultra-compact full face mask and may comprise an ultra-compact full face cushion.

### 5.3.1.10 Nasal cradle mask

In one form, for example as shown in Fig. 3Z, the seal-forming structure 3100 is configured to form a seal in use with inferior surfaces of the nose around the nares. The seal-forming structure 3100 may be configured to seal around the patient's nares at an inferior periphery of the patient's nose including to an inferior and/or anterior surface of the patient's pronasale and to the patient's nasal alae. The seal-forming structure 3100 may seal to the patient's lip superior. This type of seal-forming structure 3100 may be referred to as a "cradle cushion", "nasal cradle cushion" or "under-the-nose cushion", for example.

The shape of the seal-forming structure 3100 may be configured to match or closely follow the underside of the patient's nose and may not contact a nasal bridge region of the patient's nose or any portion of the patient's nose superior to the pronasale. In one form of nasal cradle cushion, the seal-forming structure 3100 comprises a bridge portion dividing the opening into two orifices, each of which, in use, supplies air or breathable gas to a respective one of the patient's nares. The bridge portion may be configured to contact or seal against the patient's columella in use. Alternatively, the seal-forming structure 3100 may comprise a single opening to provide a flow or air or breathable gas to both of the patient's nares.

### 5.3.2 Plenum chamber

The plenum chamber 3200 may be formed by a portion of the patient interface 3000 that has a perimeter that is shaped to be complementary to the surface contour of the face of an average person in the region where a seal will form in use. In use, a marginal edge of the portion of the patient interface 3000 forming the plenum chamber 3200 is positioned in close proximity to an adjacent surface of the face. Actual contact with the face is provided by the seal-forming structure 3100. The seal-forming structure 3100 may extend in use about the entire perimeter of a portion of the patient interface 3000 forming the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is formed by one or more components constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is formed by one or more components constructed from a translucent material. The use of a translucent material can reduce the obtrusiveness of the patient interface, and help improve compliance with therapy.

### 5.3.3 Positioning and stabilising structure

The seal-forming structure 3100 of the patient interface 3000 of the present technology may be held in sealing position in use by the positioning and stabilising structure 3300. The positioning and stabilising structure 3300 may comprise and function as "headgear" since it engages the patient's head in order to hold the patient interface 3000 in a sealing position.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 5.3.3.1 Conduit headgear

### 5.3.3.1.1 Conduit headgear tubes

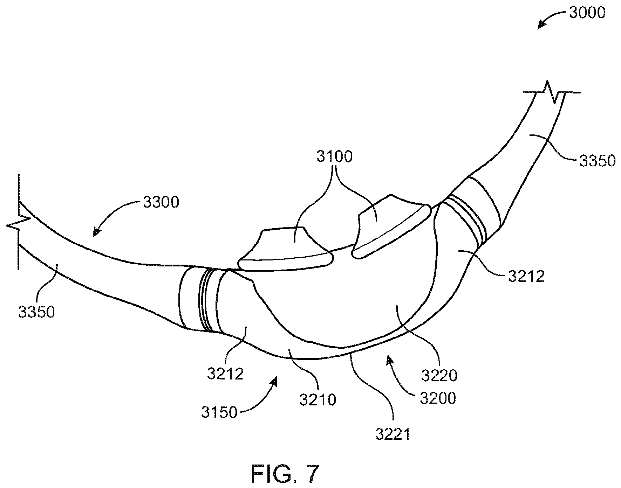

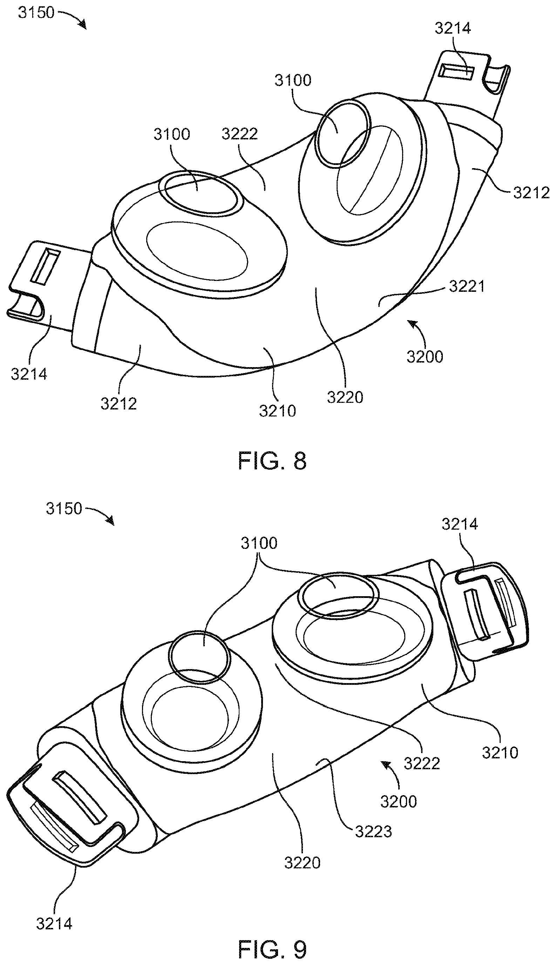

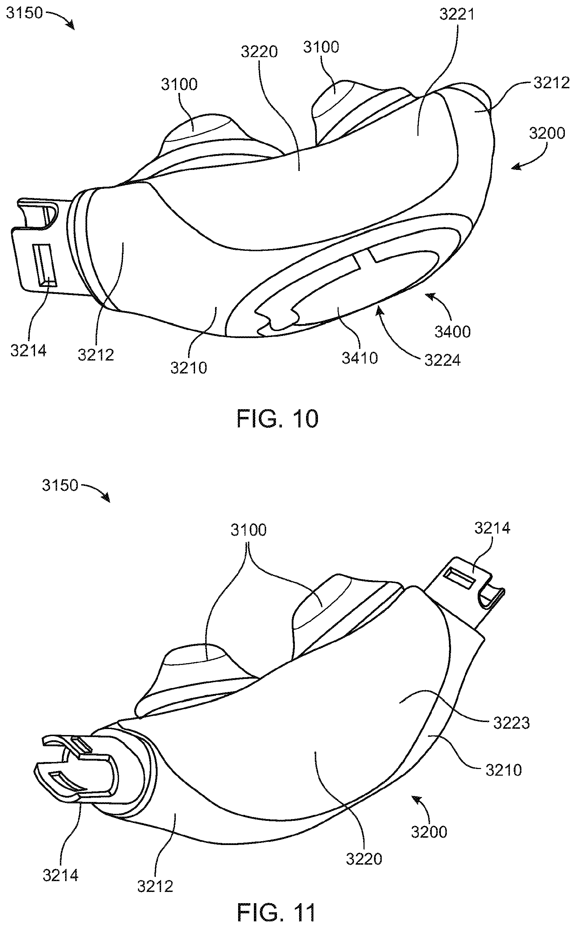

In some forms of the present technology, the positioning and stabilising structure 3300 comprises one or more headgear tubes 3350 that deliver pressurised air received from a conduit forming part of the air circuit 4170 from the RPT device to the patient's airways, for example through the plenum chamber 3200 and seal-forming structure 3100. In the form of the present technology illustrated in Fig. 3Z, the positioning and stabilising structure 3300 comprises two tubes 3350 that deliver air to the plenum chamber 3200 from the air circuit 4170. The tubes 3350 are configured to position and stabilise the seal-forming structure 3100 of the patient interface 3000 at the appropriate part of the patient's face (for example, the nose and/or mouth). This allows the conduit of air circuit 4170 providing the flow of pressurised air to connect to a connection port 3600 of the patient interface in a position other than in front of the patient's face, for example on top of the patient's head. The patient interface 3000 shown in Figs. 7-13 also includes gas delivery tubes 3350 forming conduit headgear.

Since air can be contained and passed through headgear tubing in order to deliver pressurised air from the air circuit 4170 to the patient's airways, the positioning and stabilising structure 3300 may be described as being inflatable. It will be understood that an inflatable positioning and stabilising structure 3300 does not require all components of the positioning and stabilising structure 3300 to be inflatable. For example, in the example shown in Fig. 3Z, the positioning and stabilising structure 3300 comprises the tubes 3350, which are inflatable, and the strap portion 3310, which is not inflatable.

In the form of the present technology illustrated in Fig. 3Z, the positioning and stabilising structure 3300 comprises two tubes 3350, each tube 3350 being positioned in use on a different side of the patient's head and extending across the respective cheek region, above the respective ear (superior to the otobasion superior on the patient's head) to the elbow 3612 on top of the head of the patient 1000. This form of technology may be advantageous because, if a patient sleeps with their head on its side and one of the tubes is compressed to block or partially block the flow of gas along the tube, the other tube remains open to supply pressurised gas to the patient. In other examples of the technology, the patient interface 3000 may comprise a different number of tubes, for example one tube, or three or more tubes. In one example in which the patient interface has one tube 3350, the single tube 3350 is positioned on one side of the patient's head in use (e.g. across one cheek region) and a strap forms part of the positioning and stabilising structure 3300 and is positioned on the other side of the patient's head in use (e.g. across the other region) to assist in securing the patient interface 3000 on the patient's head.

In the form of the technology shown in Fig. 3Z the two tubes 3350 are fluidly connected at superior ends to each other and to the connection port 3600. In some examples, the two tubes 3350 are integrally formed while in other examples the tubes 3350 are formed separately but are connected in use and may be disconnected, for example for cleaning or storage. Where separate tubes are used they may be indirectly connected together, for example each may be connected to a T-shaped connector having two arms/branches each fluidly connectable to a respective one of the tubes 3350 and a third arm or opening providing the connection port 3600 for fluid connection to the air circuit 4170 in use.

The tubes 3350 may be formed from a flexible material, such as an elastomer, e.g. silicone or TPE, or from one or more textile and/or foam materials. The tubes 3350 may have a preformed shape and may be able to be bent or moved into another shape upon application of a force but may return to the original preformed shape in the absence of said force. The tubes 3350 may be generally arcuate or curved in a shape approximating the contours of a patient's head between the top of the head and the nasal or oral region.

As described in US Patent no. 6,044,844, the tubes 3350 may be crush resistant to avoid the flow of breathable gas through the tubes being blocked if either is crushed during use, for example if it is squashed between a patient's head and pillow. Crush resistant tubes may not be necessary in all cases as the pressurised gas in the tubes may act as a splint to prevent or at least restrict crushing of the tubes 3350 during use. A crush resistant tube may be advantageous where only a single tube 3350 is present as if the single tube becomes blocked during use the flow of gas would be restricted and therapy will stop or reduce in efficacy. In some examples, the tubes 3350 may be sized such that each tube 3350 is able to provide sufficient flow of gas to the plenum chamber 3200 on its own should one of the tubes 3350 become blocked.

Each tube 3350 may be configured to receive a flow of air from the connection port 3600 on top of the patient's head and to deliver the flow of air to the seal-forming structure 3100 at the entrance of the patient's airways. In the example shown in Fig. 3Z, each tube 3350 lies in use on a path extending from the plenum chamber 3200 across the patient's cheek region and superior to the patient's ear to the elbow 3612. For example, a portion of each tube 3350 proximate the plenum chamber 3200 may overlie a maxilla region of the patient's head in use. Another portion of each tube 3350 may overlie a region of the patient's head superior to an otobasion superior of the patient's head. Each of the tubes 3350 may also lie over the patient's sphenoid bone and/or temporal bone and either or both of the patient's frontal bone and parietal bone. The elbow 3612 may be located in use over the patient's parietal bone, over the frontal bone and/or over the junction therebetween (e.g. the coronal suture).

In certain forms of the present technology the patient interface 3000 is configured such that the connection port 3600 can be positioned in a range of positions across the top of the patient's head so that the patient interface 3000 can be positioned as appropriate for the comfort or fit of an individual patient. In some examples, the headgear tubes 3350 are configured to allow movement of an upper portion of the patient interface 3000 (e.g. a connection port 3600) with respect to a lower portion of the patient interface 3000 (e.g. a plenum chamber 3200). That is, the connection port 3600 may be at least partially decoupled from the plenum chamber 3200. In this way, the seal-forming structure 3100 may form an effective seal with the patient's face irrespective of the position of the connection port 3600 (at least within a predetermined range of positions) on the patient's head.

As described above, in some examples of the present technology the patient interface 3000 comprises a seal-forming structure 3100 in the form of a cradle cushion which lies generally under the nose and seals to an inferior periphery of the nose (e.g. an under-the-nose cushion). The positioning and stabilising structure 3300, including the tubes 3350 may be structured and arranged to pull the seal-forming structure 3100 into the patient's face under the nose with a sealing force vector in a posterior and superior direction (e.g. a posterosuperior direction). A sealing force vector with a posterosuperior direction may facilitate the seal-forming structure 3100 forming a good seal to both the inferior periphery of the patient's nose and the anterior-facing surfaces of the patient's face on either side of the patient's nose and the patient's lip superior.

### 5.3.3.1.2 Extendable and non-extendable tube portions

In some examples of the present technology, one or both of the tubes 3350 are not extendable in length. However, in some forms, the tubes 3350 may comprise one or more extendable tube sections, for example formed by an extendable concertina structure. In some forms, the patient interface 3000 may comprise a positioning and stabilising structure 3300 including at least one gas delivery tube comprising a tube wall having an extendable concertina structure. The patient interface 3000 shown in Fig. 3Z comprises tubes 3350, the superior portions of which comprise extendable tube sections each in the form of an extendable concertina structure 3362.

The cross-sectional shape of the non-extendable tube sections 3363 of the tubes 3350 may be circular, elliptical, oval, D-shaped or a rounded rectangle, for example as described in US Patent No. 6,044,844. A cross-sectional shape that presents a flattened surface of tube on the side that faces and contacts the patient's face or other part of the head may be more comfortable to wear than, for example a tube with a circular cross-section.

In some examples of the present technology, the non-extendable tube sections 3363 connect to the plenum chamber 3200 from a low angle. The headgear tubes 3350 may extend and inferiorly down the sides of the patient's head and then curve anteriorly and medially to connect to the plenum chamber 3200 in front of the patient's face. The tubes 3350, before connecting to the plenum chamber 3200, may extend to a location at the same vertical position as or, in some examples, inferior to the connection with the plenum chamber 3200. That is, the tubes 3350 may project in an at least partially superior direction before connecting with the plenum chamber 3200. A portion of the tubes 3350 may be located inferior to the cushion module 3150 and/or the seal-forming structure 3100. The low position of the tubes 3350 in front of the patient's face facilitates contact with the patient's face below the patient's cheekbones, which may be more comfortable than contact on the patient's cheekbones and may avoid excessively obscuring the patient's peripheral vision.

### 5.3.3.1.3 Conduit headgear connection port

In certain forms of the present technology, the patient interface 3000 may comprise a connection port 3600 located proximal to a superior, lateral or posterior portion of a patient's head. For example, in the form of the present technology illustrated in Fig 3Z, the connection port 3600 is located on top of the patient's head (e.g. at a superior location with respect to the patient's head). In this example the patient interface 3000 comprises an elbow 3612 forming the connection port 3600. The elbow 3612 may be configured to fluidly connect with a conduit of an air circuit 4170. The elbow 3612 may be configured to swivel with respect to the positioning and stabilising structure 3300 to at least partially decouple the conduit from the positioning and stabilising structure 3300. In some examples the elbow 3612 may be configured to swivel by rotation about a substantially vertical axis and, in some particular examples, by rotation about two or more axes. In some examples the elbow may comprise or be connected to the tubes 3350 by a ball-and-socket joint. The connection port 3600 may be located in the sagittal plane of the patient's head in use.

Patient interfaces having a connection port that is not positioned anterior to the patient's face may be advantageous as some patients may find a conduit that connects to a patient interface anterior to their face to be unsightly and/or obtrusive. For example, a conduit connecting to a patient interface anterior to the patient's face may be prone to interference with bedclothes or bed linen, particularly if the conduit extends inferiorly from the patient interface in use. Forms of the present technology comprising a patient interface having a connection port positioned superiorly to the patient's head in use may make it easier or more comfortable for a patient to lie or sleep in one or more of the following positions: a side-sleeping position, a supine position (e.g. on their back, facing generally upwards) or in a prone position (e.g. on their front, facing generally downwards). Moreover, connecting a conduit to an anterior portion of a patient interface may exacerbate a problem known as tube drag in which the conduit exerts an undesired force upon the patient interface during movement of the patient's head or the conduit, thereby causing dislodgement away from the face. Tube drag may be less of a problem when force is received at a superior location of the patient's head than anterior to the patient's face proximate to the seal-forming structure (where tube drag forces may be more likely to disrupt the seal).

### 5.3.3.1.4 Headgear Tube Fluid Connections

The two tubes 3350 are fluidly connected at their inferior ends to the plenum chamber 3200. In certain forms of the technology, the connection between the tubes 3350 and the plenum chamber 3200 is achieved by connection of two rigid connectors. The tubes 3350 and plenum chamber 3200 may be configured to enable the patient to easily connect the two components together in a reliable manner. The tubes 3350 and plenum chamber 3200 may be configured to provide tactile and/or audible feedback in the form of a 're-assuring click' or like sound which may be easy for a patient to use as the patient may know for sure that each tube 3350 has been correctly connected to the plenum chamber 3200. In one form, the tubes 3350 are formed from a silicone or textile material and the inferior end of each of the silicone tubes 3350 is overmolded to a rigid connector made, for example, from polypropylene, polycarbonate, nylon or the like. The rigid connector on each tube 3350 may comprise a female mating feature configured to connect with a male mating feature on the plenum chamber 3200. Alternatively, the rigid connector on each tube 3350 may comprise a male mating feature configured to connect to a female mating feature on the plenum chamber 3200. In other examples the tubes 3350 may each comprise a male or female connector formed from a flexible material, such as silicone or TPE, for example the same material from which the tubes 3350 are formed.

In other examples a compression seal is used to connect each tube 3350 to the plenum chamber 3200. For example, a resiliently flexible (e.g. silicone) tube 3350 without a rigid connector may be configured to be squeezed to reduce its diameter so that it can be compressed into a port in the plenum chamber 3200 and the inherent resilience of the silicone pushes the tube 3350 outwards to seal the tube 3350 in the port in an air-tight manner. Alternatively, in a hard-to-hard type engagement between the tube 3350 and the plenum chamber 3200, each tube 3350 and/or plenum chamber 3200 may comprise a pressure activated seal, for example a peripheral sealing flange. When pressurised gas is supplied through the tubes 3350 the sealing flange may be urged against the join between the tubes and a circumferential surface around a port or connector of the plenum chamber 3200 to form or enhance a seal between the tube 3350 and plenum chamber 3200.

### 5.3.3.1.5 Conduit headgear straps

In certain forms of the present technology, the positioning and stabilising structure 3300 comprises at least one headgear strap acting in addition to the tubes 3350 to position and stabilise the seal-forming structure 3100 at the entrance to the patient's airways. As shown in Fig. 3Z, the patient interface 3000 comprises a strap portion 3310 forming part of the positioning and stabilising structure 3300. The strap portion 3310 may be known as a back strap or a rear headgear strap, for example. In other examples of the present technology, one or more further straps may be provided. For example, patient interfaces 3000 according to examples of the present technology having a full face cushion may have a second, lower, strap configured to lie against the patient's head proximate the patient's neck and/or against posterior surfaces of the patient's neck.

In the example shown in Fig. 3Z, strap portion 3310 of the positioning and stabilising structure 3300 is connected between the two tubes 3350 positioned on each side of the patient's head and passing around the back of the patient's head, for example overlying or lying inferior to the occipital bone of the patient's head in use. The strap portion 3310 connects to each tube above the patient's ears. With reference to Fig. 3Z, the positioning and stabilising structure 3300 comprises a pair of tabs 3355. In use a strap portion 3310 may be connected between the tabs 3355, The strap portion 3310 may be sufficiently flexible to pass around the back of the patient's head and lie comfortably against the patient's head, even when under tension in use.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 5.3.5 Cushion modules and exemplary patient interfaces

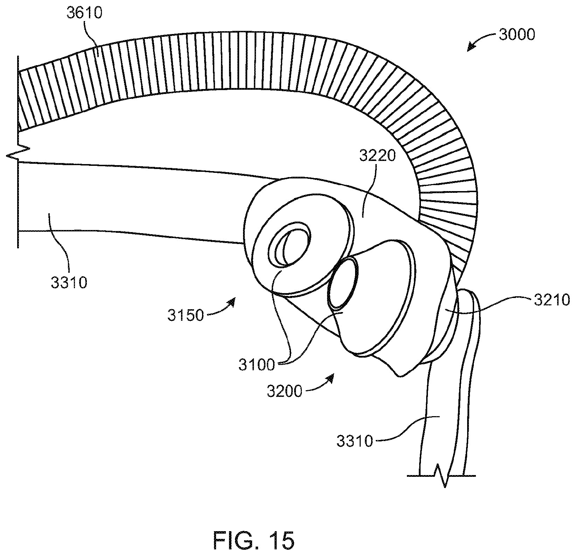

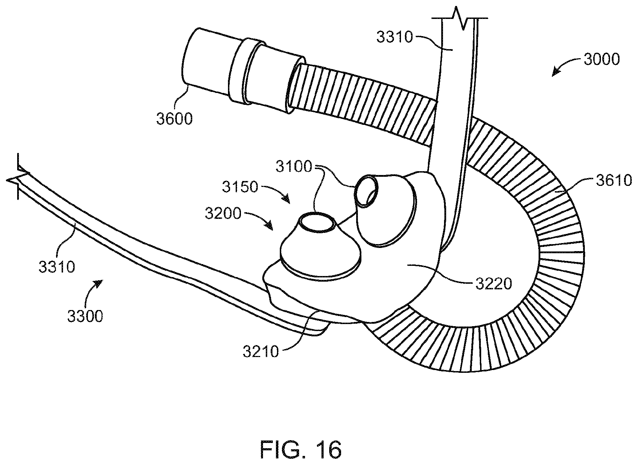

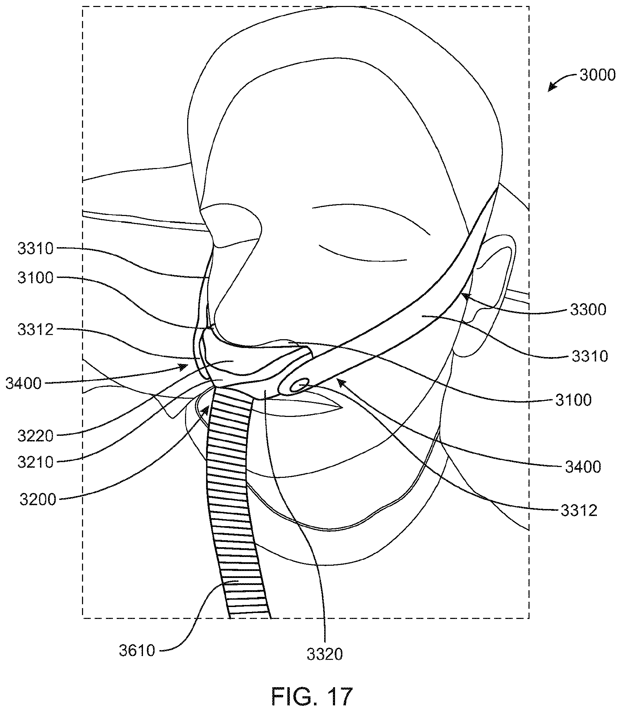

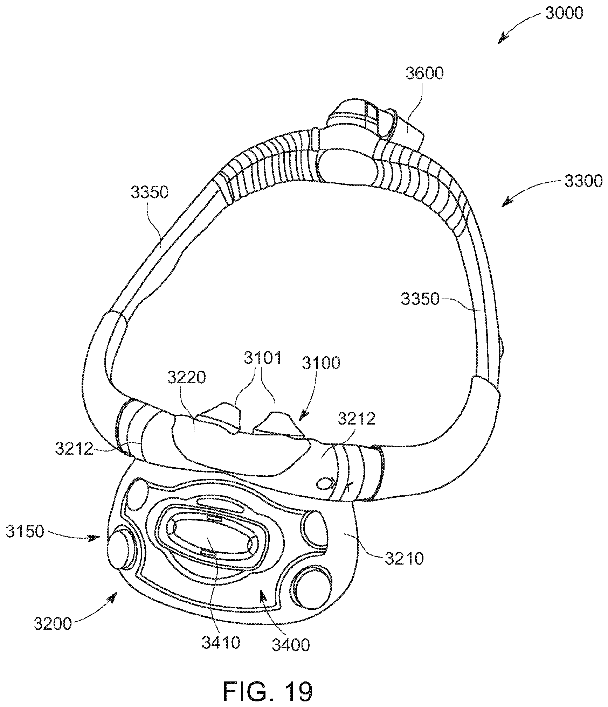

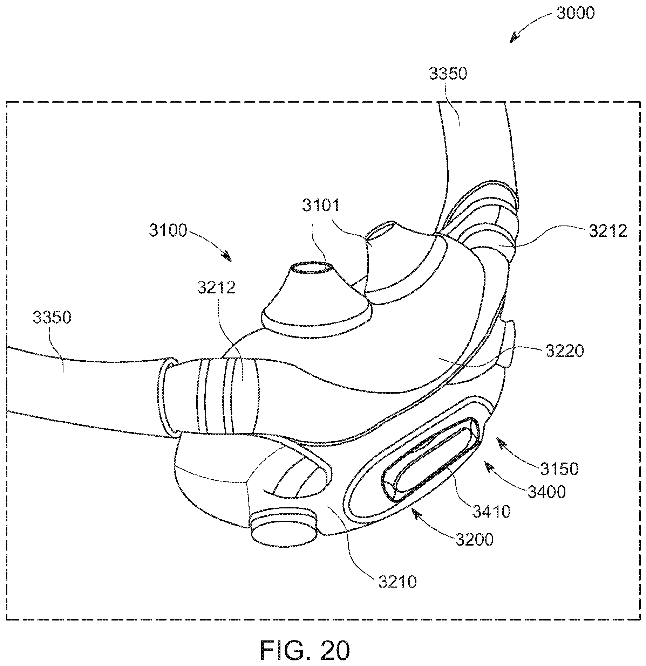

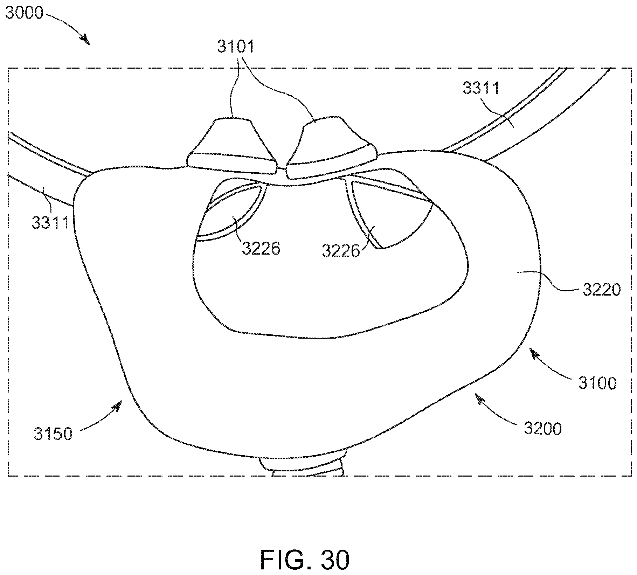

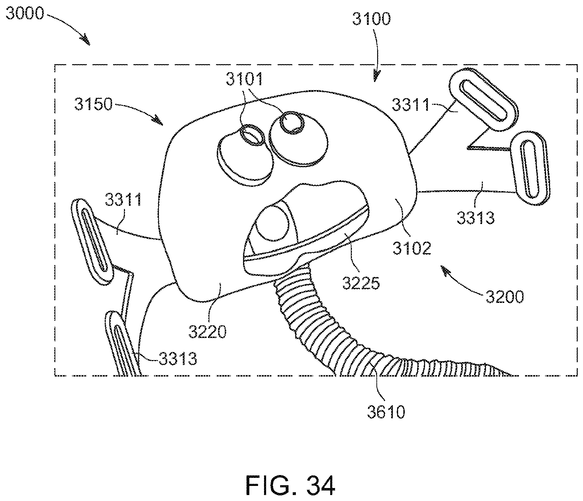

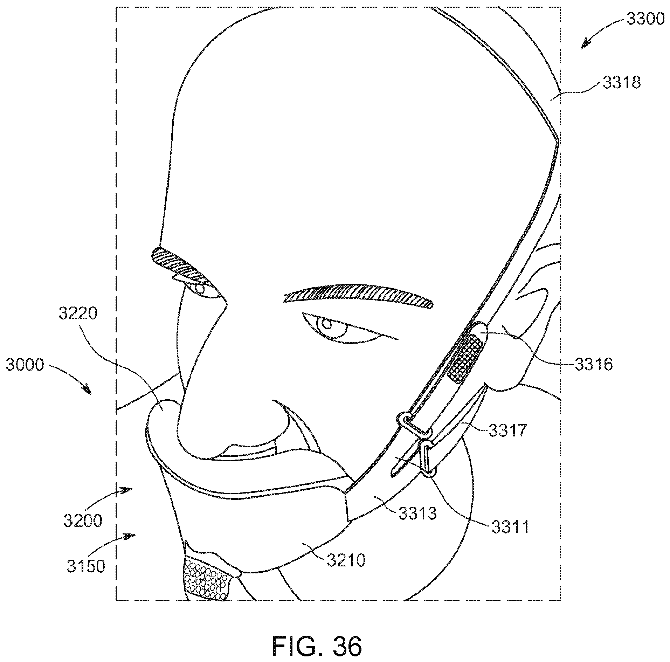

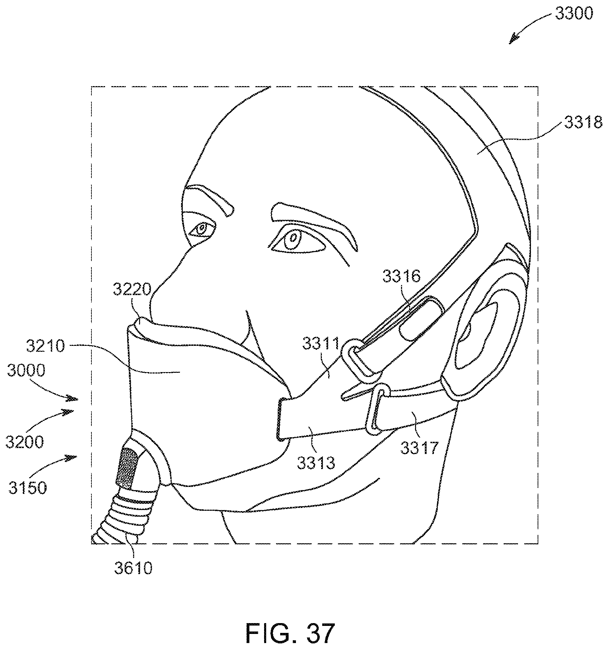

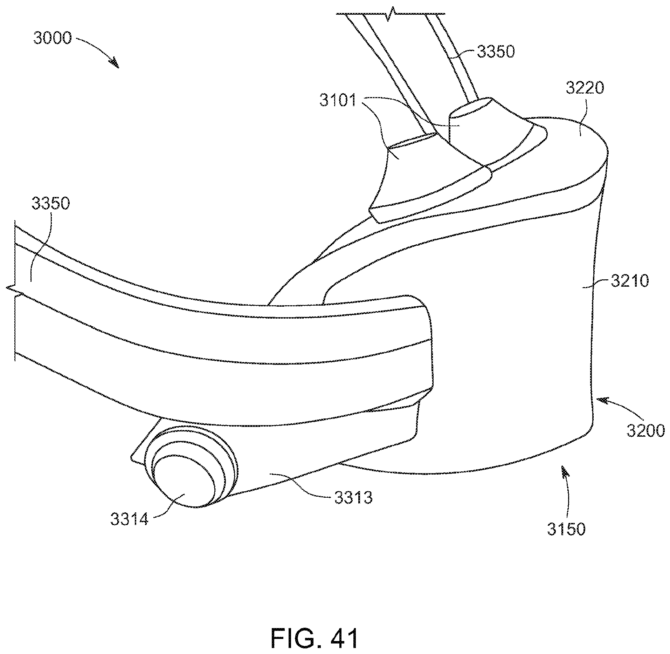

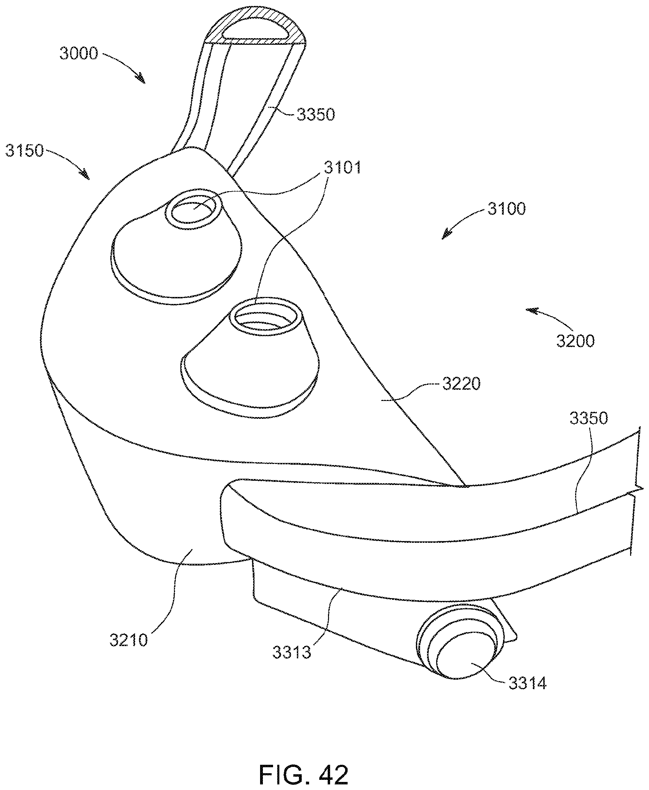

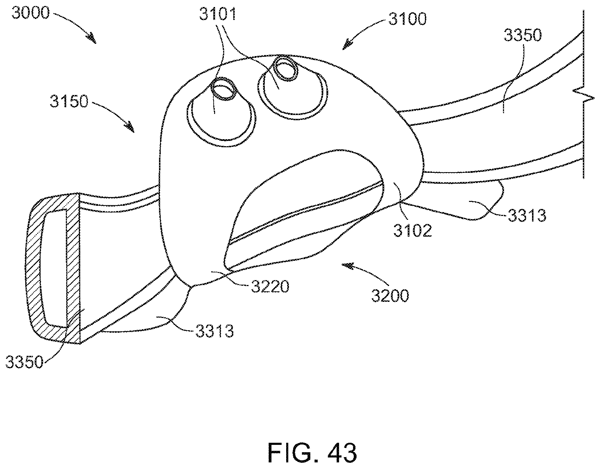

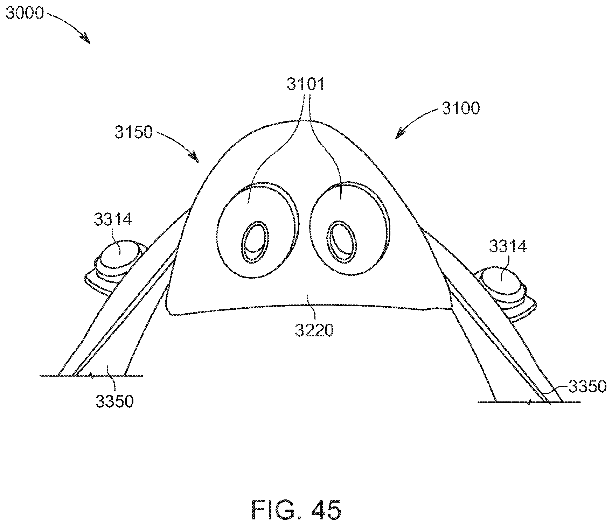

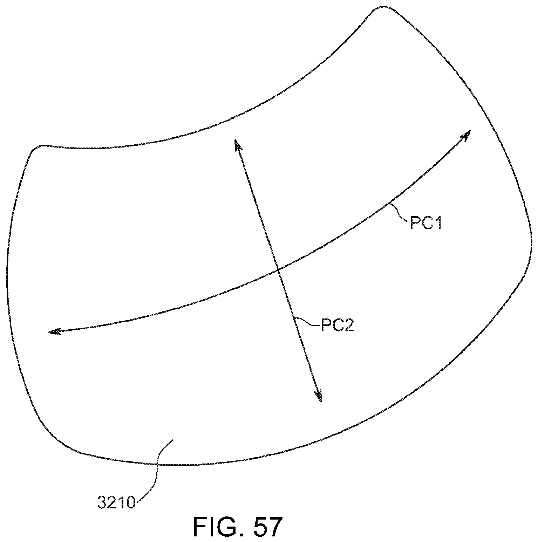

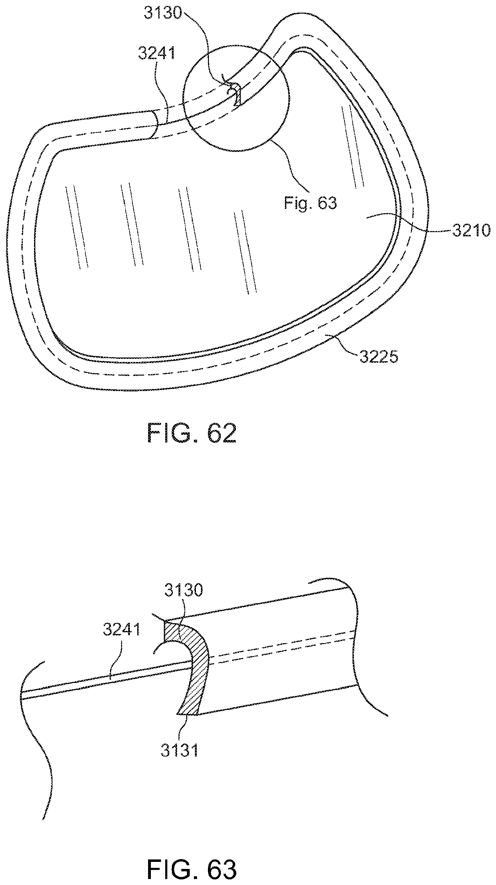

In the examples of the present technology shown in Figs. 7-20 and 25-67, the patient interface 3000 comprises a chassis portion 3210 partially forming the plenum chamber 3200. The chassis portion 3210 and a membrane portion 3220 (to be described below) may together form the plenum chamber 3200 by enclosing a volume of space which can be filled with air at a therapeutic pressure, such as of at least 4 cmH₂O or at least 6 cmH₂O above ambient air pressure. In some examples the seal-forming structure 3100 may also partially form the plenum chamber 3200, depending on the type and/or shape of the seal-forming structure 3100.

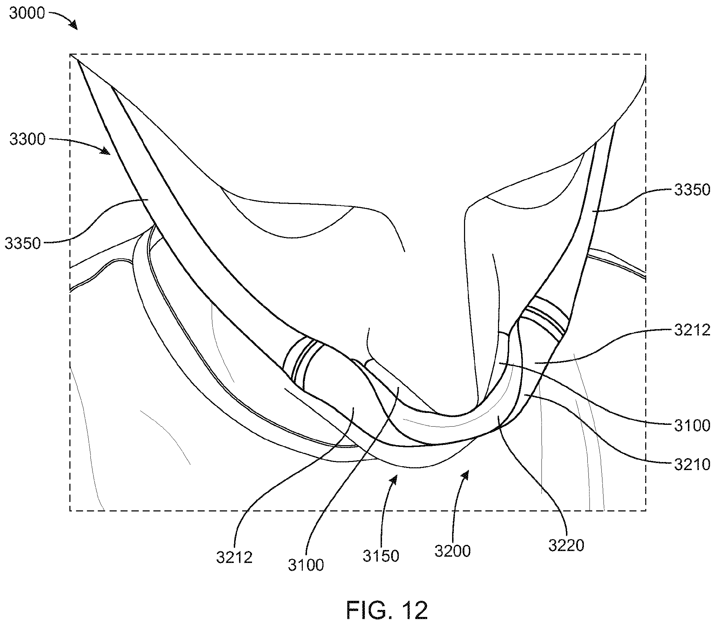

The components forming the seal-forming structure 3100 and the plenum chamber 3200 form a cushion module 3150 of the patient interface 3000, in the examples shown in Figs. 7-20 and 25-67. In the example shown in Figs. 7-13, Figs. 7-11 show the cushion module 3150 in isolation and Figs. 12-13 show it connected to headgear tubes 3350. The cushion module 3150 in these examples is separable from other components of the patient interface 3000, such as the positioning and stabilising structure 3300. The cushion module 3150 may be separable from headgear tubes 3350 of the positioning and stabilising structure 3300. Accordingly, in some examples of the present technology the patient interface 3000 comprises a removable cushion module 3150.

In some examples, the cushion module 3150 may be replaced in the patient interface 3000 by another cushion module 3150, for example one of a different size (or at least having a seal-forming structure 3100 having a different size or shape).

In other examples the cushion module 3150 may not be separable from other components or portions of the patient interface 3000, such as the positioning and stabilising structure 3300. In some examples the cushion module 3150 may comprise a chassis portion 3210 at least part of which is integrally formed with either or both of the headgear tubes 3350 or integrally formed with portions of the positioning and stabilising structure. Features of the patient interface 3000 disclosed herein are to be understood as being relevant whether or not the chassis portion 3210 is part of a removable cushion module 3150 or not, unless the context clearly requires otherwise.

The plenum chamber 3200 may include one or more plenum chamber inlet ports sized and structure to receive a flow of air at the therapeutic pressure for breathing by the patient.

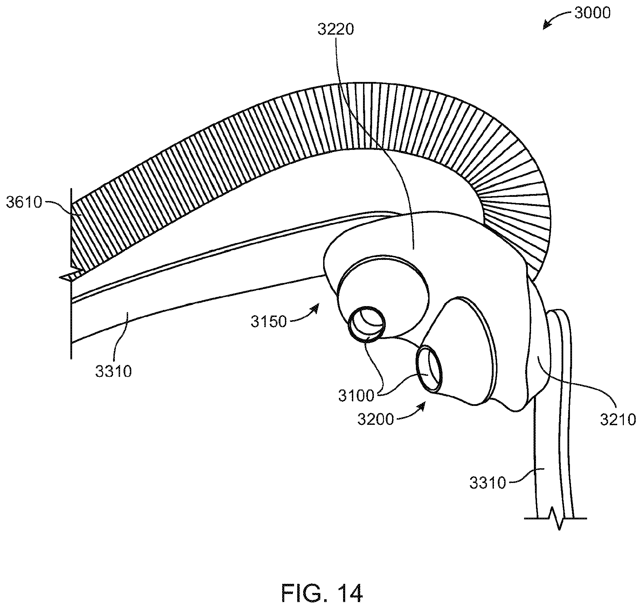

In the example shown in Figs. 7-13, the plenum chamber 3200 of the patient interface 3000 has two plenum chamber inlet ports. In particular the chassis portion 3210 defines two openings, one on each side of the chassis portion 3210. In the example shown in Figs. 14-17, the plenum chamber 3200 comprises one plenum chamber inlet port. In particular, the chassis portion 3210 defines an opening at a connection to a short tube 3610, forming the plenum chamber inlet port.

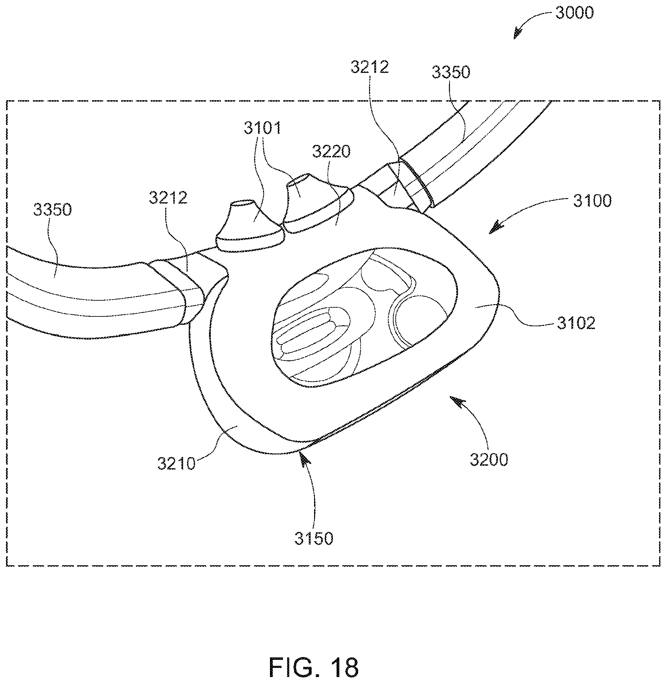

In the example of the present technology shown in Figs. 18-20, the patient interface 3000 comprises a chassis portion 3210 and membrane portion 3220 forming a plenum chamber 3200. In this example the patient interface 3000 is configured to supply a pressurised flow of air to the patient's mouth as well as to the patient's nares. The plenum chamber 3200 is configured to be positioned in front of the patient's mouth in use, in addition to under the patient's nose. The plenum chamber 3200 is therefore larger than the plenum chamber 3200 of the patient interface 3000 shown in Figs. 7-13. The plenum chamber 3200 in the example shown in Fig. 18-20 may therefore be described as having a nasal portion configured to be positioned proximate the user's nose in use and an oral portion configured to be positioned proximate the user's mouth in use. In this example, the chassis portion 3210 forms a majority of an anterior-facing side of the oral portion of the plenum chamber 3200. In some examples, the chassis portion 3210 forms substantially all of the anterior-facing side of the oral portion of the plenum chamber 3200. In some examples, the chassis portion 3210 and oral portion of the seal-forming structure 3100 form substantially all of the posterior-facing side of the oral portion of the plenum chamber 3200. The components forming the seal-forming structure 3100 and the plenum chamber 3200 form a cushion module 3150 of the patient interface 3000, in this example. The cushion module 3150 in this example is separable from other components of the patient interface, such as the positioning and stabilising structure 3300.

### 5.3.5.1 Nasal cushion module and oral cushion module

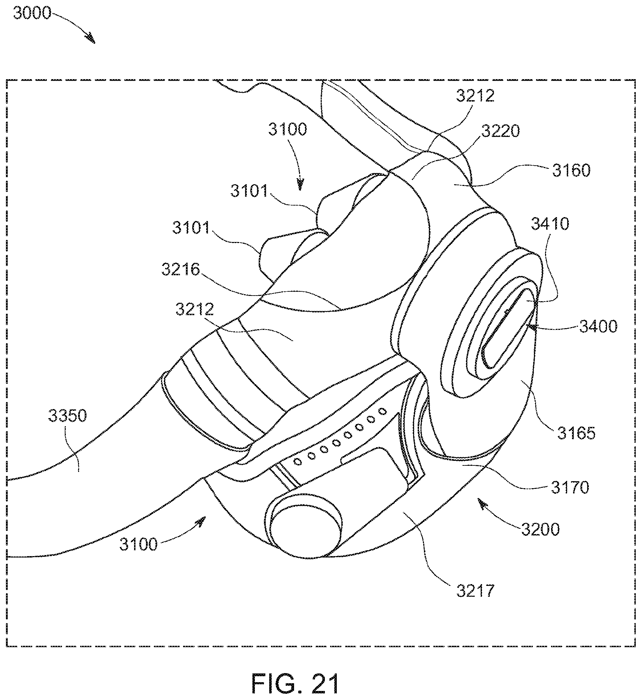

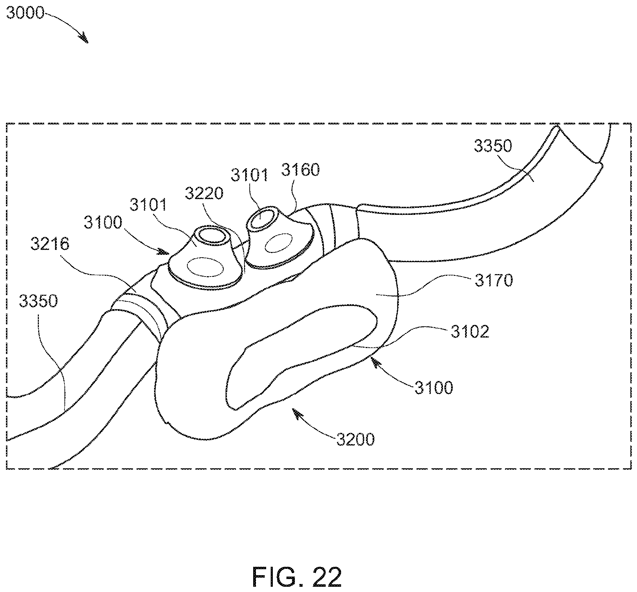

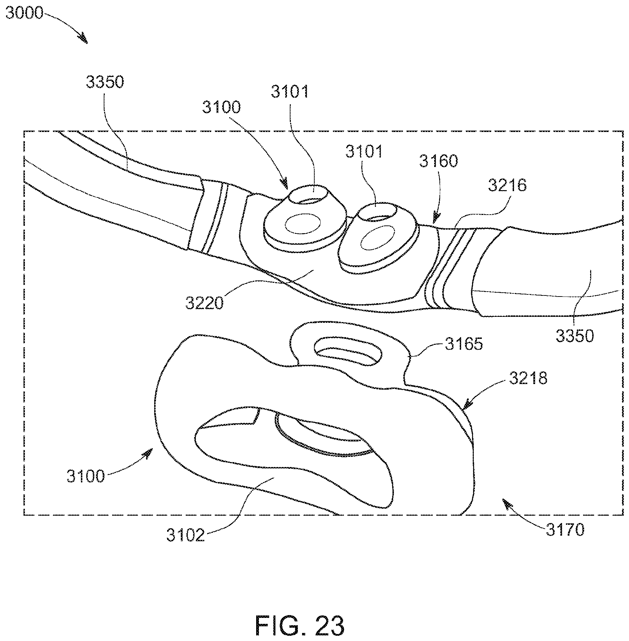

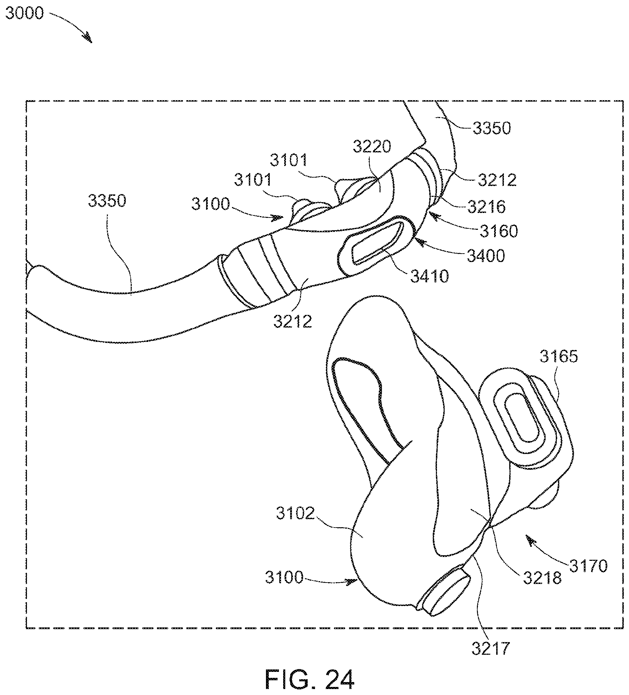

In the example of the present technology shown in Figs 21-24, the patient interface 3000 comprises a nasal cushion module 3160 and an oral cushion module 3170, each forming part of a plenum chamber 3200 of the patient interface 3000. In this example the patient interface 3000 is configured to supply a pressurised flow of air to the patient's mouth as well as to the patient's nares. The nasal cushion module 3160 and oral cushion module 3170 may be fluidly connected by an oronasal connector 3165 which may also form part of the plenum chamber 3200. The nasal cushion module 3160 may be described as forming a nasal portion of the plenum chamber 3200 and the oral cushion module 3170 may be described as forming an oral portion of the plenum chamber 3200. The nasal cushion module 3160 may be configured to be positioned under the patient's nose in use and the oral cushion module 3170 may be configured to be positioned in front of the user's mouth in use.

In the example of Figs. 21-24, the nasal cushion module 3160 may be formed by a nasal chassis portion 3216, a membrane portion 3220 and a nasal portion 3101 of the seal-forming structure 3100. The nasal portion 3101 of the seal-forming structure 3100 in this example comprises nasal pillows but in other examples may comprise another type of seal, such as a cradle cushion, for example. A seal-forming structure 3100 in the form of a cradle cushion may be formed from silicone or TPE, for example. The nasal chassis portion 3216 and membrane portion 3220 may form the nasal portion of the plenum chamber 3200. The nasal portion 3101 of the seal-forming structure 3100 may be formed from an elastomeric material.

The oral cushion module 3170 may be formed by an oral chassis portion 3217 and an oral portion 3102 of the seal-forming structure 3100, which may together form an oral portion of the plenum chamber 3200. The oral portion 3102 of the seal-forming structure 3100 is configured to form a seal around the patient's mouth and may comprise a sealing flange configured to seal to the patient's lip superior, lip inferior and cheeks. The sealing flange is formed from silicone in the example shown in Figs. 21-24 although in other examples it may be formed from another elastomeric material such as TPE, may be formed from foam, or may be formed from a textile having an airtight membrane such as a thin layer of silicone, or another suitable material. The sealing flange forming the oral portion 3102 of the seal-forming structure 3100 may be integrally formed with the oral chassis portion 3217. The sealing flange may have a thickness within the range of 0.1mm-1mm or within the range of 0.2-0.8mm or 0.25-0.5mm, for example. The sealing flange may have a thickness of 0.25mm in the lip superior region which may provide for a comfortable and effective seal at the patient's lip superior and adaptability to different patient geometry. In some particular examples the sealing flange may have a thickness of 0.2mm.

The oral cushion module 3170 may comprise a cavity 3218 (e.g. a recess portion) within which the nasal cushion module 3160 is able to be received. The cavity 3218 may open towards a superior direction such that the nasal cushion module 3160 is able to be positioned superiorly to the oral cushion module 3170 in use. The cavity 3218 may comprise a complementary shape to a portion of the nasal cushion module 3160 to enable the nasal cushion module 3160 to fit within the cavity 3218. The cavity 3218 may be defined by the oral chassis portion 3217 and/or the oral portion 3102 of the seal-forming structure 3100. The cavity may be shaped such that when the nasal cushion module 3160 is received in the cavity 3218, the nasal cushion module 3160 and oral cushion module 3170 are in correct orientations relative to each other for use of the patient interface 3000.

The nasal cushion module 3160 of the patient interface 3000 shown in Figs. 21-24 shares various features with the cushion modules 3150 of the patient interfaces shown in Figs. 7-13, 14-17 and 18-20, including a chassis portion 3210, laterally projecting connection portions 3212, membrane portion 3220 and a seal-forming structure 3100 or portion thereof. Unless the context requires otherwise, features of the cushion module 3150 described with reference to the patient interfaces 3000 shown in Figs. 7-13, 14-17 and 18-20 are to be understood to be applicable to a nasal cushion module 3160 of the type shown in Figs. 21-24. In some examples the nasal cushion module 3160 of a modular patient interface 3000 such as that shown in Figs. 21-24 is substantially identical to the cushion module 3150 shown in Figs. 7-13. In some examples, the nasal cushion module 3160 of a modular patient interface 3000 may be separable from and usable independently of the oral cushion module 3170 to form a nasal-only patient interface 3000 leaving the patient's mouth uncovered. A vent module 3410 may be provided in place of the oronasal connector 3165 (described below).

Likewise, features of the chassis portion 3210 described with reference to the patient interfaces 3000 shown in Figs. 7-13, 14-17 and 18-20 are to be understood to be applicable to the nasal chassis portion 3216 of the type shown in Figs. 21-24. Features of a membrane portion 3220 described herein are to be understood to be features applicable to a membrane portion of a cushion module 3150 of the type shown in Figs. 7-13, 14-17 and 18-20 and of a nasal cushion module 3160 of the type shown in Figs. 21-24, unless context clearly requires otherwise.

### 5.3.5.1.1 Oronasal connector

The oronasal connector 3165 of the patient interface 3000 shown in Figs. 21-24 is in the form of a tubular portion fluidly connecting an interior of the nasal cushion module 3160 to an interior of the oral cushion module 3170. A tubular portion forming a majority of the oronasal connector 3165 may be formed from silicone or TPE and one or both ends may be formed from a harder plastic material (e.g. polycarbonate or nylon). The tubular portion forming most of the oronasal connector 3165 in some examples may be the same material as the oral chassis portion 3217 and/or the nasal chassis portion 3216. In the example shown in Figs. 21-24 the oronasal connector 3165 comprises a cross section having a rounded shape. The cross section may be elliptical. In some examples it may be rectangular and may have rounded corners. In some examples the entirety of the oronasal connector 3165 may be formed from an elastomer, for example silicone or TPE.

The oronasal connector 3165 may be removably attached to one or both of the nasal cushion module 3160 and oral cushion module 3170 although in some examples it may be permanently connected to one or both of the nasal cushion module 3160 and oral cushion module 3170. Each of the nasal cushion module 3160 and oral cushion module 3170 may comprise an opening for fluid connection to the oronasal connector 3165. The opening in the nasal cushion module 3160 may align with the oronasal connector 3165 when the nasal cushion module 3160 is received in the cavity 3218 in the oral cushion module 3170.

The oronasal connector 3165 may be configured to transfer sealing force vectors from the nasal cushion module 3160 to the oral cushion module 3170 to assist in holding the oral cushion module 3170 in correct orientation for use of the patient interface 3000 (and vice versa). The oronasal connector 3165 may be configured to orient the nasal cushion module 3160 and oral cushion module 3170 correctly with respect to each other.

### 5.3.5.2 Connection to positioning and stabilising structure

The patient interfaces 3000 illustrated in Figs. 12-13 (the cushion module 3150 of which is illustrated in Figs. 7-11), Figs. 18-20 and Figs. 21-24 each comprise a positioning and stabilising structure 3300 comprising one or more gas delivery tubes 3350 configured to provide a flow of air at the therapeutic pressure to the plenum chamber 3200. In particular, the positioning and stabilising structure 3300 comprises a pair of gas delivery tubes 3350. The gas delivery tubes 3350 function as conduit headgear in these examples. More description of gas delivery tubes 3350, conduit headgear, the positioning and stabilising structure 3300 and features thereof is included elsewhere herein.

The chassis portion 3210 (or nasal chassis portion 3216 as the case may be) may comprise one or more laterally projecting connection portions 3212 configured to connect to gas delivery tubes 3350 and which may be sized and structured to receive a flow of air at the therapeutic pressure for breathing by a patient. The laterally projecting connection portions 3212 may also partially form the plenum chamber 3200 along with other portions of the chassis portion 3210 or nasal chassis portion 3216 (as the case may be) and membrane portion 3220. Each of the laterally projecting connection portions 3212 may be configured to connect to and receive a flow of gas from a respective gas delivery tube 3350 and may comprise an inlet to an interior of the chassis portion 3210. Each laterally projecting connection portion 3212 may define a plenum chamber inlet port. The chassis portion 3210 may comprise a pair of connectors 3214 configured to connect with gas delivery tubes 3350 of a positioning and stabilising structure 3300.

As shown in Figs. 7-11, the connectors 3214 are provided to either lateral side of the chassis portion 3210 and, in particular, each connector 3214 in this example is provided to a respective one of the laterally projecting connection portions 3212 of the chassis portion 3210. The connectors 3214, in this example, are provided at the inlets of the laterally projecting connection portions 3212. Figs. 12-13 show the gas delivery tubes 3350 connected to the laterally projecting connection portions 3212. The patient interface 3000 shown in Figs. 18-20 also has a chassis portion 3210 comprising laterally projecting connection portions 3212 and which may connect to gas delivery tubes 3350 via connectors 3214 (hidden in Figs. 18-20 within the tubes 3350). In the example shown in Figs. 21-24 the nasal cushion module 3160 comprises a nasal chassis portion 3216 comprising laterally projecting connection portions 3212 and which may connect to gas delivery tubes 3350 via connectors 3214 (also hidden within the gas delivery tubes 3350 in Figs. 21-24).

The angles at which the laterally projecting connection portions 3212 project advantageously orient the seal-forming structure 3100 (e.g. the nasal portion 3101 and oral portion 3102 thereof in the case of the patient interface 3000 shown in Figs. 21-24) at an angle which allows the seal-forming structure 3100 to form a stable seal with the patient's face without occluding the patient's nose.

In other examples, the chassis portion 3210 may not comprise laterally projecting connection portions 3212. In some examples, such as the example shown in Figs. 14-17, to be described in more detail below, the chassis portion 3210 comprises a single opening, for example on an anterior side thereof, at which the chassis portion 3210 may fluidly connect with an air circuit 4170, for example via a short tube, to receive a flow of air at the therapeutic pressure into the plenum chamber 3200. In such examples, the chassis portion 3210 may be configured to connect to headgear straps (for example via rigidisers, which may be in the form of substantially rigid arms sheathed in a textile material or at least having a textile material on the patient facing side thereof for patient comfort) of a positioning and stabilising structure 3300 of the patient interface 3000.

In the example shown in Figs. 18-20, the patient interface comprises a cushion module 3150 that supplies air to both the patient's nose and mouth and, in the example shown in Figs. 21-24, the patient interface 3000 comprises a nasal cushion module 3160 and an oral cushion module 3170. As described above, the positioning and stabilising structure 3300 in these examples comprise headgear tubes 3350 forming conduit headgear. Additionally, in these examples the positioning and stabilising structure 3300 comprises a pair of lower straps (not shown in the drawings, but the lower straps may be as disclosed in International (PCT) Application Publication No. WO 2019/183680.

Each lower strap may be configured to be positioned on a respective side of the patient's head below a respective otobasion inferior of the patient's head. The lower straps may be configured to releasably attach to the chassis portion 3210 or oral chassis portion 3217, as the case may be. In some examples the connection between the lower straps and chassis portion may be a magnetic connection and/or a mechanical connection. The lower straps may be elastically extendable, which may advantageously allow the positioning and stabilising structure 3300 to tolerate changes in spacing between the patient's chin and neck when the patient moves their head.

### 5.3.5.3 Vents

### 5.3.5.3.1 Vent in chassis portion

In some forms, the patient interface 3000 may comprise a vent 3400 to allow a continuous flow of gases exhaled by the patient from an interior of the plenum chamber 3200 to ambient, for example throughout the patient's entire respiratory cycle, said vent 3400 being sized and shaped to maintain the therapeutic pressure in the plenum chamber in use. The patient interface 3000 in the example illustrated in Figs. 7-13 comprises a vent module 3410 comprising the vent 3400. The vent module 3410 may be a component or assembly formed from a substantially rigid material and comprising a plurality of holes forming the vent 3400. The vent module 3410 may be provided to an anterior side of the chassis portion 3210. In the example shown in Figs. 7-13, the vent module 3410 is provided to an anteroinferior side of the chassis portion 3210. The chassis portion 3210 comprises a hole to receive the vent module 3410, which is an anteroinferior hole in the illustrated example, and may be an anterior- or inferior-facing hole in other examples. In some examples, the vent module 3410 is configured to support a diffuser through which the continuous flow of gases exhaled by the patient is able to pass when flowing to ambient. In some examples, the vent module 3410 is configured to allow the continuous flow of gases exhaled by the patient from the interior of the plenum chamber 3200 to ambient to pass by the diffuser without flowing through the diffuser.

In the example shown in Figs. 18-20, the patient interface 3000 comprises a vent 3400 and vent module 3410 which may be substantially as described above in relation to Figs. 7-13. However, in this example the vent 3400 is provided to the chassis portion 3210 proximate (e.g. anterior to) the oral portion 3102 of the seal-forming structure 3100. The vent module 3410 may be provided to an anterior side of the chassis portion 3210. The chassis portion 3210 may comprise an anterior-facing hole to receive the vent module 3410.

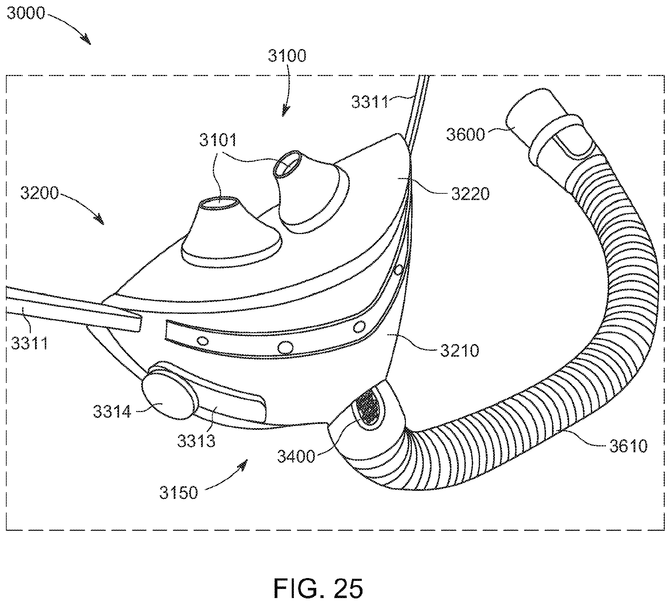

Patient interfaces according to other examples of the present technology, such as those described with reference to Figs. 25-67, are to be understood to have a vent 3400 for gas washout (e.g. a vent 3400 to allow a continuous flow of gases exhaled by the patient from an interior of the plenum chamber 3200 to ambient, said vent 3400 being sized and shaped to maintain the therapeutic pressure in the plenum chamber in use), even if no vent 3400 is depicted or identified in the drawings. The vent 3400 may be provided within a vent module 3410.

### 5.3.5.3.2 Vent in oronasal connector

In the example shown in Fig. 21-24, the oronasal connector comprises the vent 3400 of the patient interface 3000. In this example, a vent module 3410 comprising the vent 3400 is provided to the oronasal connector 3165. The vent module 3410 is received in a hole on the oronasal connector 3165. The vent module 3410 may be provided to an anterior portion of the oronasal connector 3165 and patient interface 3000 to direct vented gas flow away from the patient. The vent 3400 and vent module 3410 may be as described in relation to the vent 3400 and vent module 3410 in the example shown in Figs. 7-13.

### 5.3.5.4 Anti-asphyxia valve (AAV)

The patient interfaces 3000 shown in or described with reference to Figs. 18-20 and 21-67 each further comprise an anti-asphyxia valve (AAV), not visible in the drawings. The AAV may be provided to the oral portion of the patient interface 3000. In some examples the AAV is integrated into the vent module 3410. The vent module 3410 may comprise a gas washout vent 3400 and an AAV. In some examples the AAV is provided in the chassis portion 3210 (or oral chassis portion 3217) of the patient interface 3000. In further examples the AAV is provided at a connection port 3600 of the patient interface 3000 or a connection between the plenum chamber and a short tube 3610, such as an inlet port connector 3605.

### 5.3.5.5 Seal-forming structure

As described above, the patient interface 3000 comprises a seal-forming structure 3100. The seal-forming structure 3100 may form part of the cushion module 3150. In examples in which the patient interface 3000 comprises a nasal cushion module 3160 and an oral cushion module 3170, the seal-forming structure 3100 may form part of the nasal cushion module 3160 and oral cushion module 3170.

In the examples shown in Figs. 7-13, 14-17, 18-20, 21-24, and 25-46, the seal-forming structure 3100 comprises a pair of nasal pillows. Each nasal pillow may be constructed and arranged to form a seal with a respective naris of the nose of a patient. Each nasal pillow may have a hole therein such that a flow of air at a therapeutic pressure can be delivered to at least the patient's nares.

In the examples shown in Figs. 18-20, 21-24 and 25-46, the seal-forming structure 3100 comprises a pair of nasal pillows forming a nasal portion 3101 of the seal-forming structure 3100 and further comprises an oral portion 3102 of the seal-forming structure. The oral portion 3102 of the seal-forming structures 3100 shown in Figs. 18-20 and 21-24 may be configured to form a seal around the patient's mouth such that the flow of air (e.g. at least a portion thereof) at the therapeutic pressure is delivered to the patient's mouth.

The nasal pillows or, more generally, any seal-forming structure 3100 of a patient interface 3000 according to one of the examples of the present technology, may be constructed and arranged to maintain said therapeutic pressure in the plenum chamber throughout the patient's respiratory cycle in use.

In each of the examples shown in Figs. 7-46, the nasal pillows forming the seal-forming structure 3100 are stalkless. Each of the nasal pillows may comprise a conical portion (e.g. a frusto-cone/frustoconical portion) having a tip and a base wider than the tip. The base may be attached directly to the membrane portion 3220 (which will be described in detail below). That is, there may be no stalk or narrower connecting portion between the frustoconical portion of each nasal pillow and the membrane portion 3220. The lack of a stalk may advantageously help keep the force exerted by each pillow on the patient's nose low, which may provide for a comfortable patient interface 3000. Also, having no stalk makes the nasal pillows more stable, and therefore only low force is required to hold them in position in the nares which provide for patient comfort. In other examples the nasal pillows each comprise a stalk connecting a frustoconical portion of the nasal pillow to the membrane portion 3220. However, the highly flexible membrane portion 3220 may allow for sufficient movement of the nasal pillows that a stalk for purpose of decoupling may not be required.

The frustoconical portion of each nasal pillow may seal against a respective naris of the patient's nose. For example, the frustoconical portion of each nasal pillow may be structured to seal against inferior surfaces of the patient's nose defining a respective one of the patient's nares. For example, the frustoconical portion of each nasal pillow may seal against the patient's nose at a respective nasal opening.

The nasal pillows forming part of the seal-forming structure 3100 may be formed separately from the membrane portion 3220 and may be attached to the membrane portion 3220 by any method which creates a seal between the nasal pillows and the membrane portion 3220 and which secures the nasal pillows to the membrane portion 3220 throughout the intended life of the cushion module 3150. In some examples the nasal pillows may be adhered to the membrane portion 3220. In other examples the nasal pillows may be moulded to the membrane portion 3220, for example by supporting the membrane portion 3220 or cushion module in a mould and moulding the nasal pillows in place on the membrane portion 3220.

In some examples, the nasal pillows are formed from a different material to the membrane portion 3220 (e.g. the nasal pillows may be formed from a first material and the membrane portion 3220 may be formed from a second material different to the first material). Each of the nasal pillows may be formed from an elastomeric material. The nasal pillows in the examples shown in Figs. 7-46 are formed from silicone. In other examples the nasal pillows may be formed from a thermoplastic elastomer (TPE), foam, textile or combinations thereof, for example. The nasal pillows may be formed from a single wall or a double wall structure. In some examples each nasal pillow is formed from a single wall. In other examples the nasal pillows may be dual-wall nasal pillows (e.g. formed from two walls).

In other examples the seal-forming structure 3100 may take the form of a nasal cradle cushion (e.g. a seal-forming structure 3100 configured to seal to the pronasale, nasal alae and lip superior of the patient). A nasal cradle seal-forming structure 3100 may be formed from an elastomeric material such as silicone or TPE, or may be formed from at least partially from a textile material, optionally with an undercushion, which may for example be formed from foam.

### 5.3.5.6 Chassis portion and membrane portion

The patient interface 3000 may comprise a membrane portion 3220 connected to the chassis portion 3210 (or nasal chassis portion 3216, as the case may be). The chassis portion 3210 and membrane portion 3220 may together form the plenum chamber 3200, for example together enclosing a volume which can be filled with air at the therapeutic pressure.

In some examples of the present technology, the membrane portion 3220 supports the seal-forming structure 3100 or at least a portion thereof (e.g. a nasal portion 3101 of the seal-forming structure 3100. For example, the seal-forming structure 3100 may be supported on the membrane portion 3220. In the examples shown in Figs. 7-13 and 14-17, the nasal pillows forming the seal-forming structure 3100 are supported on a membrane portion 3220. In the examples shown in Figs. 18-20 and 21-24, the nasal pillows supported on the membrane portion 3220 form a nasal portion 3101 of the seal-forming structure 3100.

The chassis portion 3210 (or the nasal chassis portion 3216 or oral chassis portion 3217 as the case may be) may be configured to support the membrane portion 3220, for example by being stiffer than the membrane portion 3220. The chassis portion 3210 may be formed from a material that is stiffer than a material from which the membrane portion 3220 is formed. Additionally, or alternatively, the chassis portion 3210 may be thicker than the membrane portion, such as at least 5 times thicker, at least 7 times thicker, at least 10 times thicker, or more.

The chassis portion 3210 may be a portion of the cushion module 3150 (or nasal cushion module 3160 or oral cushion module 3170, as the case may be) having sufficient stiffness (e.g. as a result of material and/or shape/structure) to maintain the shape of the of the cushion module 3150 and support the membrane portion 3220. The chassis portion 3210 may hold the membrane portion 3220 substantially taut when the plenum chamber 3200 is unpressurised. For example, the chassis portion 3210 may hold the membrane portion 3220 taut such that the nasal pillows are held substantially in an in-use position prior to the plenum chamber 3200 being pressurised. The membrane portion 3220 may be sufficiently taut to align the nasal pillows with the patient's nares when the patient dons the patient interface 3000 prior to the plenum chamber 3200 being pressurised. In particular, the chassis portion 3210 may hold the membrane portion 3220 taut but unstretched at rest. The nasal pillows, or more generally the seal-forming structure 3100, may also provide some support to the at-rest shape of the membrane portion 3220. The membrane portion 3220 at rest may be taut such that it is held substantially in a predetermined shape. In some examples, the membrane portion 3220 at rest may be taut but there may be no stress within the material forming the membrane portion 3220. The membrane portion 3220 at rest may be taut such that it is generally held in a predetermined shape and holds the seal-forming structure 3100 (e.g. nasal pillows) generally in a predetermined position with respect to the chassis portion 3210, but may be easily deformable by forces acting on the membrane portion 3220, such as finger pressure for example.

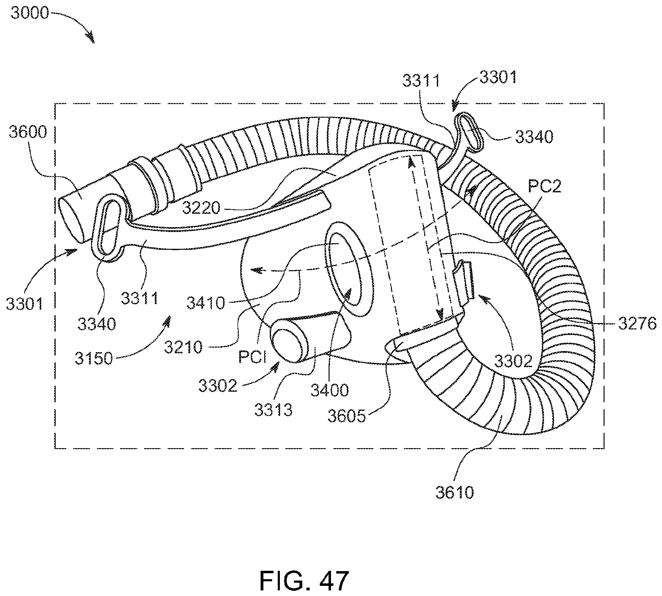

The chassis portion 3210 may be formed from an elastomeric material, in some examples. In the example shown in Figs. 7-13, the chassis portion 3210 is formed from silicone. In other examples, the chassis portion 3210 may be formed from a thermoplastic elastomer (TPE). In further examples, the chassis portion 3210 may be formed from a substantially rigid material, such as a thermoplastic material, for example polycarbonate, Nylon or the like. In the examples described with reference in Figs. 47-67, the chassis portion 3210 may be formed from a textile material. In other examples the chassis portion 3210 may be formed from foam or a combination of materials, such as a combination of the materials disclosed herein.

The membrane portion 3220 may be constructed and arranged to be flexible to allow the seal-forming structure 3100 to move with respect to one or more other portions of the patient interface 3000 in use. In particular, the seal-forming structure 3100 may move with respect to the chassis portion 3210. For example, in the patient interface 3000 shown in Figs. 7-24, the membrane portion 3220 is constructed and arranged to be flexible to allow for relative movement between the nasal pillows and the chassis portion 3210 (e.g. nasal chassis portion 3216 in the case of the example shown in Figs. 21-24). The membrane portion 3220 may allow for relative movement between the nasal pillows and a portion of the cushion module 3150 (or 3160), such as the chassis portion 3210, in use. The membrane portion 3220 may provide a trampoline function in which the seal-forming structure 3100, such as the nasal pillows, is held in place yet the chassis portion 3210 is able to move with respect to the nasal pillows via deformation of the membrane portion 3220. Relative movement between the seal-forming structure 3100 and chassis portion 3210 may comprise the seal-forming structure 3100 being stationary as it may be held fixed against the patient's face, or in the patient's nares, and the chassis portion may move with respect to the seal-forming structure 3100 and the user's face. Advantageously, this at least partially decouples the seal-forming structure 3100 from the chassis portion 3210, which may improve stability and seal, especially during head movement.

The highly flexible nature of the membrane portion 3220 supporting the nasal pillows, produced for example by the material and the low thickness of the membrane portion 3220, may result in a cushion that is highly resistant to transfer of disruptive force from structural components/portions (such as the chassis portion 3210 and positioning and stabilising structure 3300) through to the nasal pillows. Most or all of the disruptive forces received by the chassis portion 3210 expected during use of the patient interface 3000 may be absorbed by the membrane portion 3220 and prevented from travelling through to the nasal pillows.

In some examples the membrane portion 3220 may be constructed and arranged to at least partially decouple movement of the nasal pillows from each other. In particular, the membrane portion 3220 may be constructed and arranged to allow each of the nasal pillows to move to align to a respective one of the patient's nares in use. The membrane portion 3220 may be constructed and arranged to stretch and/or flex to allow each of the nasal pillows to move to align to the respective one of the patient's nares prior to pressure being applied. This may allow the nasal pillows to readily fit to a wider range of patient's noses and/or tolerate an uneven mask setup. Furthermore, disruptive forces, especially during movement or side sleeping may result in asymmetrical loading on the nasal pillows. The membrane portion 3220 may be configured to tolerate uneven loading by decoupling the nasal pillows from each other to keep each nasal pillow in sealing position.

The membrane portion 3220 may be constructed and arranged to be flexible to provide the effects described herein by, for example, being thin and not being held so tightly that it is unable to move or deform. For example, the membrane portion 3220 may be formed from a thin layer or layers of material(s) and held such that it is able to deform to allow for relative movement between the seal-forming structure 3100 and the chassis portion 3210. The chassis portion 3210 being formed from a flexible material, e.g. an elastomer, such as silicone or TPE, may also advantageously help the membrane portion 3220 to deform such that chassis portion 3210 is able to move with respect to the nasal pillows. The chassis portion 3210 in some examples is able to flex, which may allow further shape change in the membrane portion 3220 over and above the ability of the membrane portion 3220 alone to flex, stretch and inflate, since the boundary of the membrane portion 3220 is able to move when the chassis portion 3210 flexes.

In some examples, the membrane portion 3220 may be constructed and arranged to be inflated upon pressurisation of the plenum chamber 3200 to the therapeutic pressure in use. The membrane portion 3220 may be stretchable and, in some examples, may be constructed and arranged to stretch during inflation upon pressurisation of the plenum chamber 3200 to the therapeutic pressure in use. In some examples, the membrane portion 3220 may be formed from a stretchable material (e.g. a material able to stretch during inflation upon pressurisation of the plenum chamber 3200 to the therapeutic pressure in use).

In some examples the membrane portion 3220 may be structured and arranged to be taut in the absence of therapeutic pressure in the plenum chamber 3200. In such examples, the membrane portion 3220 may stretch (e.g. balloon in shape) during inflation to have a larger surface area than at rest in the absence of pressure in the plenum chamber 3200. In other examples the membrane portion 3220 may not be taut (e.g. it may be loose/floppy) and during inflation may assume a taut configuration and may also then stretch with further inflation.

As described in more detail below, the membrane portion 3220 may not be moulded into a three-dimensional shape (for example, it may be formed as a sheet). The lack of a predetermined three-dimensional shape may result in the membrane portion 3220 allowing a large amount of movement of the nasal pillows or other seal-forming structure 3100 for setup and decoupling purposes, since the membrane portion 3220 may have little or no inherent bias back towards a predetermined shape.

In examples of the present technology in which the membrane portion 3220 partially forms both a nasal portion of a plenum chamber 3200 and an oral portion of the plenum chamber 3200 (such as the example shown in Fig. 18-20), at least the portion of the membrane portion 3220 forming the nasal portion of the plenum chamber 3200 (which may be identified as a nasal portion of the membrane portion 3220) may inflate in the manner described above. Alternatively, or additionally, in examples in which the seal-forming structure 3100 comprises a nasal portion 3101 and an oral portion 3102, the portion of the membrane portion 3220 supporting the nasal portion 3101 of the seal-forming structure 3100 may inflate in the manner described above.

### 5.3.5.6.1 Pressurisation of plenum chamber

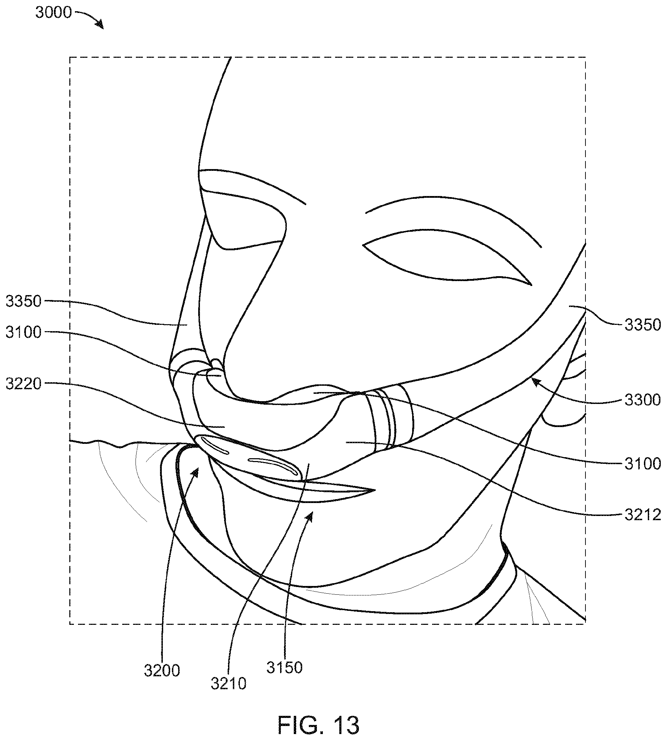

Figs. 7-11 show a patient interface 3000 prior to being donned by a patient. As illustrated, the membrane portion 3220 is taut to the extent that there are substantially no creases, folds or the like. Figs. 12 and 13 show the patient interface 3000 of Figs. 7-11 when donned by a patient. In Fig. 12 the plenum chamber 3200 is unpressurised and in Fig. 13 the plenum chamber 3200 is pressurised. Some aspects and behaviour of the patient interface 3000 shown in Figs. 7-11 will be described below with reference to Figs. 12-13 and, unless the context requires otherwise, the aspects and behaviour described are to be understood to be applicable to the patient interfaces 3000 shown in Figs. 14-17, 18-20 and 21-24 and 25-46.

Fig. 12 shows the patient interface 3000 having been donned by a patient but prior to pressurisation of the plenum chamber 3200. Prior to pressurisation, the membrane portion 3220 may form to the underside of the patient's nose and/or upper lip, for example to the underside of the patient's pronasale as shown in Fig. 12. As the plenum chamber 3200 is not pressurised, the patient's nose pressing against the membrane portion 3220 forms a crease in the membrane portion 3220 on an anterosuperior-facing side of the plenum chamber 3200. At this point the nasal pillows are held in the nose by the elastic forces from the membrane portion 3220 (e.g. a spring return force).

As shown in Fig. 12, the membrane portion 3220 has an anterior edge that is spaced from the patient's nose (e.g. the anterior-most boundary of the membrane portion 3220, at which the membrane portion 3220 is attached to the chassis portion 3210). This spacing from the patient's nose or from the posterosuperior side of the plenum chamber 3200 may be considered a "depth" of an anterior portion of the membrane portion 3220. The depth provides space for the patient's nose to press into the membrane portion 3220 and cause the anterior portion of the membrane portion 3220 to compress, during setup. The depth of the membrane portion 3220 in this region may be selected such that the patient is able to tighten the headgear such that their nose presses into this region but not to the extent that their nose touches the chassis portion 3210.

Fig. 13 shows the patient interface 3000 donned by a patient and with pressurisation of the plenum chamber 3200. As shown, there is no longer a crease in the membrane portion 3220 just under/in front of the patient's nose, as was visible prior to pressurisation in Fig. 12, because the membrane portion 3220 has inflated and stretched (e.g. ballooned). This inters/buries the frustoconical sealing portions of the nasal pillows (portions of the seal-forming structure) into the patient's nares. The pressure in the plenum chamber 3200 maintains a push on the pillows during use, which provides the mask with a robust seal. The inflatable nature of the membrane portion 3220 may bias the nasal pillows towards respective nares in use for a better and more stable seal, such that the patient interface 3000 is effectively "self-adjusting".

Inflation of the membrane portion 3220 may advantageously help urge the seal-forming structure 3100 towards the patient's face. With respect to the examples shown in Figs. 7-24, the membrane portion 3220 may be constructed and arranged to inflate to urge each of the nasal pillows towards a respective one of the patient's nares in use. This may advantageously facilitate a good seal between each nasal pillow and the respective naris.

In some examples, the membrane portion 3220 may be constructed and arranged to inflate to conform to one or more portions of the patient's nose in use. This may effectively provide a customised fit which cradles the patient's nose (e.g. the underside of the nose) and distributes sealing forces resulting in patient comfort and a good seal. As described above, the membrane portion 3220 may conform to the patient's pronasale. The membrane portion 3220 may conform to an inferior-facing surface of the patient's pronasale. In some examples the membrane portion 3220 may be configured to leave the anterior-most portion of the patient's pronasale uncovered. This may advantageously provide for an unobtrusive patient interface 3000. In some examples the membrane portion 3220 may be configured to conform to inferior-facing surfaces of the patient's nasal ala. The ballooning effect (together with the ability of the thin membrane portion 3220 to conform to the patient's facial form) of the membrane portion 3220 to seal against inferior-facing surfaces of the patient's nose and against the patient lip superior (as described below) may advantageously distribute forces on the patient's nose and face over a large area, which may provide for comfort in use, as the risk of high contact pressure regions occurring may be low.

The inflation of the membrane portion 3220 may also help maintain a good seal in use, for example during movement of the patient's head or when the patient interface 3000 receives disruptive forces, such as tube drag or force from contact between the patient interface 3000 and the patient's pillow or bedsheets. In particular, with reference to the examples shown in Figs. 7-24, the membrane portion 3220 is constructed and arranged to resist separation of the nasal pillows from the patient's nares upon movement of the chassis portion 3210 in use. In some examples, the geometrical orientation of the conical seal relative to the pressurised membrane ensures the conical seal is locked in the nares.

The membrane portion 3220 may be configured to engage the patient's lip superior in use. In some examples, this may provide further support, stability and/or sealing to supplement the seal-forming structure 3100. In some examples this may provide for a stable and comfortable fit (e.g. by distributing the load over a large area). The chassis portion 3210 may be configured to not engage the patient's lip inferior in use, which may be uncomfortable. The chassis portion 3210 may be sufficiently small and/or low profile that the membrane portion 3220 contacts the lip superior in use but the chassis portion 3210 does not contact the lip inferior. A small and/or low-profile chassis portion 3210 may provide for an unobtrusive patient interface 3000. Furthermore, the chassis portion 3210 may be configured to not engage the patient's lip superior in use. That is, in some examples the membrane portion 3220 may contact the patient's lip superior but the chassis portion 3210 may not.

In some examples, the membrane portion 3220 does not contact the patient's face other than the patient's nose and upper lip in use. The membrane portion 3220 may not contact the patient's cheeks in use. In some examples the membrane portion 3220 may not contact the patient's nasolabial sulci in use. In some examples, the membrane portion 3220 may not contact lateral facing sides of the patient's nose in use. The membrane portion 3220 may not contact the patient's nasal ridge in use.

In some examples, the chassis portion 3210 does not contact the patient's face in use. In some examples, the chassis portion does not contact the patient's cheeks in use. In some examples, the chassis portion does not contact the patient's nasolabial sulci in use.

### 5.3.5.6.2 Textile membrane portion

In some examples of the present technology, the membrane portion 3220 is formed at least partially from a textile material, e.g. a fabric material. The textile material may advantageously provide the membrane portion 3220 with a surface that is comfortable when contacting the patient's face. In the examples described with reference to Figs. 7-67 the membrane portion 3220 comprises a textile layer and an air impermeable layer. The textile layer may comprise a woven material and the air impermeable layer may comprise a polymer film, for example. In some examples, the air impermeable layer may be formed from silicone. In some examples the membrane portion 3220 may be formed from an air impermeable film flocked with a textile material. Advantageously, the use of a textile material in forming the membrane portion 3220 may allow the membrane portion 3220 to be sufficiently stretchable yet sufficiently resistant to tearing that it is able to stretch during inflation upon pressurisation of the plenum chamber 3200 and do so without tearing (at least during its service life). As illustrated in Figs. 7-13, the textile layer provides an external surface of the membrane portion 3220. That is, the textile layer may be exterior to the plenum chamber 3200 and, in the Figs. 7-13 example, be on an exterior of the cushion module 3150. In some examples, if the textile material of the membrane portion 3220 is sufficiently air-impermeable, an additional sealing layer may not be included.

In some examples, the knitting stitch of the textile material of the membrane portion 3220 may be a Single Jersey Weft Knit. In other examples the textile material may comprise an alternative knitting stitch (in some examples a Tricot knit).

The textile material may comprise one or more synthetic fibres. In some examples the textile material may comprise 80% Polyamide and 20% Elastane. The Elastane may advantageously provide for high stretchiness/elasticity. The Elastane content may be within the range of 5-20%, within the range of 10-20% or within the range of 5-15%, in examples. In some examples the Elastane content of the textile material is 15% or 10%. In some examples the textile material comprises Polyester instead of Polyamide. Other suitable materials to provide the effects of the membrane portions 3220 described herein may also be used.

In some examples the textile material is 0.27mm in thickness. The membrane portion 3220 may comprise an airtight silicone backing having a thickness of around 0.03mm, providing for an overall thickness of 0.3mm. In some examples the weight of the textile material may be 105gsm.

In some examples the textile material is provided to the patient interface 3000 with wales running in a generally superior-inferior direction in use and courses running laterally in use. The textile material may have greater stretch in the direction of the courses and the membrane portion 3220 may be required to stretch laterally across the patient's face more so than in the superior-inferior directions.

More generally, an aspect of the present technology, as illustrated in Figs. 7-13 among others, is a patient interface 3000 having a chassis portion 3210 comprising a membrane portion 3220 at least partially formed from a textile material, the seal-forming structure 3100 of the patient interface 3000 being formed from an elastomeric material and being supported on the membrane portion 3220. In the example shown in Figs. 7-13 the chassis portion 3210 comprises a textile membrane portion 3220 which supports an elastomeric seal-forming structure 3100 in the form of nasal pillows. The textile surface of the membrane portion 3220 may provide for comfortable contact against the patient's face around their airways and the elastomeric material forming the seal-forming structure 3100 may provide for a good seal. The textile membrane portion 3220 may also have further advantages in relation to its ability to inflate and stretch without tearing, as discussed above.

Another advantage which may be provided by a configuration in which the membrane portion 3220 is formed at least partially from a textile material and the seal-forming structure 3100 comprises nasal pillows formed from an elastomeric material and supported on the membrane portion 3220 is that the nasal pillows can be stiffer than the textile membrane portion 3220. The nasal pillows may both help hold the membrane portion 3220 in a predetermined shape prior to inflation upon pressurisation of the plenum chamber 3200. The nasal pillows may also transfer forces to the more flexible membrane portion 3220 during use. The flexibility of the membrane portion 3220 may also allow for distribution of load over a large area on the patient's face (in particular the underside of the nose and lip superior) which may result in a comfortable patient interface 3000.

A textile membrane portion 3220 may be applied to the chassis portion 3210 by moulding the chassis portion 3210 to a textile membrane portion 3220 while the textile membrane portion 3220 is supported in a mould. Alternatively the chassis portion 3210 may be moulded separately and then the textile membrane portion 3220 may be attached to it (e.g. glued, welded, taped or otherwise attached). Similarly the nasal pillows (or other seal-forming structure) may be moulded onto the textile membrane portion 3220 or moulded separately and then attached (e.g. glued, welded or the like).

### 5.3.5.6.3 Elastomeric membrane portion

In other examples of the present technology, the membrane portion 3220 may be formed from an elastomer, such as silicone or TPE. The elastomer forming the membrane portion 3220 may be sufficiently thin and have material properties that enable the membrane portion 3220 to function in the manner of the textile membrane portion 3220 described herein. Accordingly, features of a membrane portion 3220 described with reference to provided by a textile membrane portion 3220 described herein are to be understood to be applicable to an elastomeric/silicone membrane portion 3220, unless context requires otherwise, and vice versa. In particular, a silicone membrane portion 3220 may be sufficiently thin and formed from an appropriate silicone material that it is able to stretch during inflation upon pressurisation of the plenum chamber 3200 in use, biasing nasal pillows into engagement with the patient's nares and/or providing for relative movement between the nasal pillows and the chassis portion 3210 or between each of the nasal pillows. The silicone membrane portion 3220 may be less than 0.25mm in thickness, for example 0.2mm in thickness, or may be less than 0.2mm in thickness, less than 0.15mm in thickness and, in some examples, may be within the range of 0.05mm-0.2mm, or 0.1mm-0.15mm, in thickness. The silicone membrane portion 3220 may be formed from a silicone having a Durometer hardness within the range of D20-D40. Other durometer values are also contemplated, such as softer durometer values in some examples.

In examples in which the membrane portion 3220 is formed from silicone, the membrane portion 3220 may be formed separately to the chassis portion 3210. For example, the membrane portion 3220 may be formed first and -placed in a mould, after which the chassis portion 3210 may be injection moulded and joined (e.g. bonded) to the membrane portion 3220. Alternatively, the chassis portion 3210 may be moulded in isolation and then the membrane portion 3220 may be attached to the chassis portion 3210 (e.g. by gluing, welding or the like). In other examples, the membrane portion 3220 may be integrally formed with the chassis portion 3210, for example by moulding the membrane portion 3220 and chassis portion 3210 together in the same moulding step, provided the membrane portion 3220 has mouldable thickness. In some examples, the membrane portion 3220 is formed from silicone and comprises a thickness of 0.2mm. Advantageously, a silicone membrane portion 3220 having a thickness of 0.2mm may have high stretchability, may be able to be moulded with the chassis portion 3210 and may be sufficiently resistant to tearing.

As described elsewhere herein, in some examples the membrane portion 3220 is formed from an elastomer but is not formed into a predetermined three-dimensional shape. The membrane portion 3220 may have no bias to a predetermined shape. This may provide for a highly flexible membrane portion 3220 which may advantagesouly aid in setup (allowing nasal pillows or other seal-forming structure 3100 to move to create a good seal) and decoupling (allowing some disruptive movement of the chassis portion 3210 with respect to nasal pillows or other seal-forming structure 3100 instead of transferring such movement to the nasal pillows).

### 5.3.5.6.4 Plenum chamber geometry and other aspects

As described above, the chassis portion 3210 and membrane portion 3220 may form the plenum chamber 3200. The plenum chamber 3200 may comprise a posterosuperior-facing side configured to face posteriorly and superiorly in use. In examples of the present technology in which the patient interface 3000 comprises an oral portion, the posterosuperior-facing side described herein may be part of the nasal portion of the plenum chamber 3200. A seal-forming structure 3100 (or nasal portion of a seal-forming structure 3100, as the case may be), such as the nasal pillows of the patient interface 3000 shown in Figs. 7-24, may be provided to the posterosuperior-facing side. The seal-forming structure 3100 may be provided to the posterosuperior-facing side to enable the seal-forming structure 3100 to face posteriorly and superiorly in use to engage the anterior- and inferior-facing surfaces of the patent's nose in use. The membrane portion 3220 and seal-forming structure 3100 may form a majority of the posterosuperior-facing side of the plenum chamber 3200. As illustrated in Figs. 7-24, the membrane portion 3220 and seal-forming structure 3100 form substantially all of the posterosuperior-facing side.

The plenum chamber 3200 may comprise an anterosuperior-facing side configured to face anteriorly and superiorly in use. In examples of the present technology in which the patient interface 3000 comprises an oral portion, the posterosuperior-facing side described herein may be part of the nasal portion of the plenum chamber 3200. As illustrated in Figs. 7-24, the membrane portion 3220 may form a majority of the anterosuperior-facing side of the plenum chamber 3200 or nasal portion thereof, as the case may be. The membrane portion 3220 may extend from the posterosuperior-facing side into the anterosuperior-facing side. The plenum chamber 3200 may comprise a curved boundary at which the membrane portion 3220 meets the chassis portion 3210 on the anterosuperior-facing side.

The plenum chamber 3200 may further comprise a posteroinferior-facing side configured to face posteriorly and inferiorly in use. As illustrated in Figs. 7-17 and 21-24, the membrane portion 3220 may form a majority of the posteroinferior-facing side of the plenum chamber 3200 (or nasal portion thereof, as the case may be). The membrane portion 3220 may extend from the posterosuperior-facing side into the posteroinferior-facing side. The plenum chamber 3200 may comprise a curved boundary at which the membrane portion 3220 meets the chassis portion 3210 on the posteroinferior-facing side.

In the examples of the present technology shown in Figs. 18-67, the plenum chamber 3200 may not comprise a posteroinferior-facing side in the nasal portion as the posterosuperior-facing side of the plenum chamber 3200, which supports the nasal pillows, may be adjacent and superior to an oral portion of the seal-forming structure 3100 also formed by the membrane portion 3220. The portion of the membrane portion 3220 that forms the oral portion of the seal-forming structure 3100 may be identified as an oral portion of the membrane portion 3220. As the oral portion of the seal-forming structure 3100 is configured to seal to the patient's face around the patient's mouth, the oral portion of the membrane portion 3220 may be configured to face substantially posteriorly in use, the oral portion being an extension of the membrane portion 3220 down the posterior side of the cushion module 3150 in this example.

In some examples of the present technology, such as the examples illustrated in Figs. 7-13, the chassis portion 3210 forms a majority of an anteroinferior-facing side of the plenum chamber 3200 configured to face anteriorly and inferiorly in use. The chassis portion 3210 in this example comprises a hole configured to receive the vent module 3410 comprising the vent 3400. The chassis portion 3210may stretch-fit around the vent module 3410 to enable the vent module 3410 to be inserted into the hole in the chassis portion 3210.

In some examples, the chassis portion 3210 and membrane portion 3220 may each provide about half of the surface area of the cushion module 3150.

In some examples, the anterosuperior-facing side of the plenum chamber 3200 or at least a portion thereof is substantially planar in an at rest position. Similarly the posteroinferior-facing side of the plenum chamber 3200 or at least a portion thereof may be substantially planar in an at rest position. The anterosuperior-facing side of the plenum chamber 3200 or at least a portion thereof may be substantially parallel to the posteroinferior-facing side of the plenum chamber 3200 or at least a portion thereof.

In some examples, the posterior-most portions of the nasal pillows are spaced posteriorly in use from a posterior-most portion of the membrane portion 3220. Substantially all of the membrane portion 3220 may be located anteroinferiorly to the nasal pillows. The membrane portion 3220 may not extend posteriorly past the tips of the nasal pillows and in some examples may not extend posteriorly past the base of the nasal pillows.

In some examples, the cushion module 3150 (or nasal cushion module 3160, as the case may be) of the patient interface 3000 does not comprise portions on lateral sides of the nasal pillows that contact the face. The cushion module 3150 may contact the nose and the lip superior but may not contact the patient's cheeks or nasolabial sulci, for example. The patient interface 3000 may be structured and/or arranged such that the nasal pillows are positioned in the patient's nares without support portions of the cushion module 3150 contacting the patient's cheeks. In the examples illustrated herein, the membrane portion 3220 flexes and/or stretches when the nasal pillows enter the nares and then provides reaction forces which resist separation of the nasal pillows from the patient's nares. The pressure within the plenum chamber 3200 also acts on the membrane portion 3220 to push the nasal pillows into the nares. Furthermore, in examples in which the patient interface 3000 comprises conduit headgear, the gas delivery tubes 3350 may bend the flexible cushion module 3150 to wrap it around and towards the nose which may help prevent separation of the nasal pillows from the nares.

The membrane portion 3220 in some forms of the present technology may not have a predetermined three-dimensional shape in the absence of positive pressure with respect to atmospheric pressure in the plenum chamber 3220. In some examples of the present technology, the nasal pillows are supported on a membrane portion 3220 which is not moulded into a predetermined shape. In some examples, the membrane portion 3220 may not be formed by injection moulding into a predetermined shape. In some examples, the membrane portion 3220 may be formed by a process other than moulding (e.g. calendering). The membrane portion 3220 may not comprise a three-dimensional predetermined shape. That is, the membrane portion 3220 may be formed such that it is not biased into a predetermined shape. For example, the membrane portion 3220 may be manufactured in sheet form and therefore may be in the form of sheet. In particular, the membrane portion 3220 may be in the form of a sheet connected to the chassis portion 3210 at edges of the membrane portion 3220 (e.g. after the membrane portion 3220 has been cut to a shape and size to fit to the edges of the chassis portion. The lack of a predetermined three-dimensional shape may provide a particularly low amount of restriction on movement of the nasal pillows. As discussed elsewhere herein, the membrane portion 3220 may also be sheetlike, slack and/or may be structured so as to be unable to support itself in a predetermined three-dimensional shape.

A membrane portion 3220 that is a thin sheet with no other preformed geometry (e.g. a thin silicone membrane or textile membrane) may allow the nasal pillows to freely conform to the patient's nasal geometry. In contrast, a membrane portion 3220 that is moulded into a shape may have resistance to deformation from that shape which may limit the ability of the pillows to move to conform to the patient's nose. The nasal pillows may be able to move in a less impeded manner when the membrane portion 3220 is not moulded in a predetermined shape than if the membrane portion 3220 is moulded to shape. In particular, the nasal pillows may be better able to wobble around their bases and may be highly moveable up and down along their axes. Furthermore, the lack of a moulded shape in the membrane portion 3220 means the nasal pillows are not moulded in predetermined orientations with respect to the cushion module 3150. Furthermore, a membrane portion 3220 that is not moulded into a predetermined shape may allow the nasal pillows to have a large range of motion. This may help the nasal pillows tolerate disruptive forces in use as they are highly free to move with respect to the chassis portion 3210.

In some examples the nasal pillows are not moulded together. Each nasal pillow may be moulded separately. Even if the nasal pillows are moulded together, they may be separated from each other (e.g. by cutting) when attached to the membrane portion 3220. This may advantageously help decouple the nasal pillows from each other. In some examples the nasal pillows are attached to the membrane portion 3220 such that they are not angled towards each other in an at rest state prior to pressurisation of the plenum chamber 3200.

In some examples, the membrane portion 3220 inflates towards a convex shape (e.g. towards a shape in which the external surface is convex) when the plenum chamber 3200 is pressurised. This may assist in urging the nasal pillows towards the patient's nares and maintaining a continuously adjusting seal. The presence of the patient's nose and face acting against this inflation may cause some or all of the membrane portion 3220 to become concave due to the restrained nasal pillows and/or conforming to the patient's nose and upper lip.

In some examples, the cushion module 3150 (or nasal cushion module 3160, as the case may be) may not have a rigid frame. With the lack of a rigid frame the sides of the cushion module 3150 may be at least partially decoupled from each other, which may allow the cushion module 3150 to conform as required to a particular patient to provide a good seal and which may allow the cushion module 3150 to tolerate disruptive forces.

### 5.3.5.6.5 Membrane portion forming seal-forming structure

In some examples, the membrane portion 3220 may form one or more portions of the seal-forming structure 3100. For example, the membrane portion 3220 may form an oral portion of a seal-forming structure 3100.

In the example shown in Figs. 18-20, the seal-forming structure 3100 comprises a nasal portion 3101 and an oral portion 3102. The nasal portion 3101 of the seal-forming structure 3100 comprises nasal pillows supported on the membrane portion 3220 and the oral portion 3102 of the seal-forming structure 3100 is formed by the membrane portion 3220. The patient interfaces 3000 shown in Figs. 25-46 also have this configuration. The patient interfaces 3000 shown in Figs. 47-67 also have nasal portions and oral portions, although in these examples the patient interfaces 3000 comprise no nasal pillows and instead the membrane portion 3220 provides both a nasal portion 3101 of the seal-forming structure 3100 and the oral portion 3102 of the seal-forming structure 3100.

With reference to Figs. 18-20, the membrane portion 3220 may comprise a nasal portion forming the portion of the membrane portion 3220 supporting the nasal pillows (or other portion of the seal-forming structure 3100 that forms a seal to provide the flow or air to the patient's nares, e.g. a cradle in some examples), and the membrane portion 3220 may further comprise an oral portion forming the oral portion of the seal-forming structure 3100.

The membrane portion 3220 (e.g. either or both of the nasal portion and oral portion of the membrane portion 3220), may be sheetlike. It may be sheetlike by being in the form of a sheet of material. A sheetlike membrane portion 3220 may be substantially flat, in some examples. In some examples, a membrane portion 3220 may not necessarily be perfectly planar but be sheetlike in the sense that it is formed resembling a sheet, e.g. generally flat and having a width and length that are much greater than its thickness. The membrane portion 3220 may have uniform thickness. A sheetlike membrane portion 3220 may be formed by a sheet-production process, such as calendaring, or may be formed by moulded.

In some examples, the membrane portion 3220 is slack in the absence of positive pressure in the plenum chamber 3200. The membrane portion 3220 in some examples may be structured so as to be unable to support itself in a predetermined three-dimensional shape. The membrane portion 3220 may be floppy although may be held in place by its connection to the chassis portion 3200. In some examples, the membrane portion 3220 does not have a predetermined three-dimensional shape. The membrane portion 3220 may be structured so as to not be biased into a predetermined three-dimensional shape. A membrane portion 3220 so flexible that it has no predetermined shape and/or is unable to support itself in a predetermined shape may advantageously be particularly effective in decoupling the nasal portion 3101 of the seal-forming structure 3100 from the chassis portion 3210 and/or allowing the nasal portion 3101 of the seal-forming structure 3100 to fit or conform to the patient's face.

The membrane portion 3220 may comprise a sealing flange forming an oral portion 3102 of the seal-forming structure 3100. A superior portion of the sealing flange is integrally formed with the nasal portion of the membrane portion 3220 in the examples shown in Figs. 18-20 and 25-46. More generally, the portion of the membrane portion 3220 supporting the nasal pillows is integrally formed with the portion of the membrane portion 3220 forming the oral portion 3102 of the seal-forming structure 3100. The nasal portion of the membrane portion 3220 is integrally formed with the oral portion of the membrane portion 3220. The nasal portion of the membrane portion 3220 may also seal to the patient's nose and/or face proximate the nose to form a seal, in addition to nasal pillows.

The oral portion 3102 of the seal-forming structure 3100 is configured to form a seal around the patient's mouth and may comprise a sealing flange configured to seal to the patient's lip superior, lip inferior and cheeks and define an oral opening into the plenum chamber 3200. The sealing flange is formed by the membrane portion 3220 in the examples shown in Figs. 18-20 and 25-46. The membrane portion 3220 is connected to and supported by the chassis portion 3210. A periphery of the sealing flange formed by the membrane portion 3220 is supported by the chassis portion 3210 at lateral and inferior sides of the sealing flange. The inside periphery of the sealing flange formed by the membrane portion 3220 is unrestrained to allow it to conform well to the shape of the patient's face around the patient's mouth.

The sealing flange may comprise a curved cross section at one or more locations around the oral opening, for example at the upper lip and/or the chin, such that a convex surface contacts the patient's face prior to conforming to the surface of the patient's face. In some examples the cross section of the sealing flange may be convex at the patient's cheeks. In other examples it may be substantially flat. The curved cross section may provide for a pressure-assisted seal against the patient's face whereby the therapeutic pressure in the plenum chamber 3200 helps hold the sealing flange against the surface of the patient's face. The sealing flange formed by the membrane portion 3220 may be joined to the chassis portion 3210 such that in use the chassis portion 3210 does not contact the patient's face. For example, the join between the chassis portion 3210 and the membrane portion 3220 in the oral portion of the plenum chamber 3200 may be spaced from the patient's face in use.

The membrane portion 3220 in the example shown in Figs. 18-20 may be formed from a textile material (e.g. with air-tight silicone layer) as described elsewhere herein, or may be formed from thin silicone (e.g. around 0.2mm in some examples) or another elastomer, such as TPE.

Figs. 25-46 show further examples of patient interfaces 3000 comprising a cushion module 3150 having a membrane portion 3220 forming an oral portion 3102 of the seal-forming structure 3100. These examples will be described in more detail below.

In some examples, including those shown in Figs 25-46, the chassis portion 3210 may be flexible to at least partially decouple the membrane portion 3220 from disruptive forces applied to the chassis portion 3210 in use. Disruptive forces may be forces that may adversely affect the seal formed between the seal-forming structure 3100 and the patient's face, such as tube drag or forces exerted on the patient interface 3000 by the patient's pillow/bed when side sleeping. Despite some flexibility to provide this decoupling effect, the chassis portion 3210 has sufficient stiffness as a structure to support the membrane portion 3220.

In some examples, the chassis portion 3210 may be formed at least partially from a textile material, which may be a textile material able to support its own shape, such as a thick and somewhat stiff textile material such as breathoprene. For example, the chassis portion 3210 may be formed from 2.5mm thick breathoprene. In other examples, the chassis portion 3210 may be formed from a polymer material which is flexible due to thinness and geometry. Such a polymer material may be substantially rigid at larger thicknesses but the chassis portion 3210 may have a sufficiently low thickness that the material is able to deform to at least partially absorb disruptive forces. The chassis portion 3210 may be flexible to absorb disruptive forces, but have a sufficient stiffness to support its own shape and support the membrane portion 3220 in use.

In the examples illustrated in Figs 25-46 the chassis portion 3210 is formed from Mylar. The Mylar material may have a thickness in the range of 0.2mm-0.5mm, for example 0.25mm. In other examples, the chassis portion 3210 may be formed from polycarbonate, having a thickness within the range of 0.4-1mm, such as within the range of 0.5-0.75mm for example. The chassis portion 3210 may comprise a moulded shell or a sheet that has been vacuum formed (and trimmed to size if necessary). The chassis portion 3210 may be formed from an elastomeric material such as silicone or TPE in other examples.

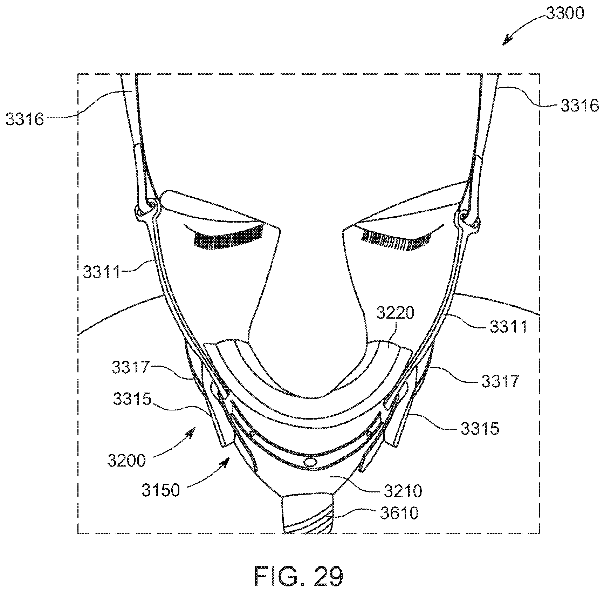

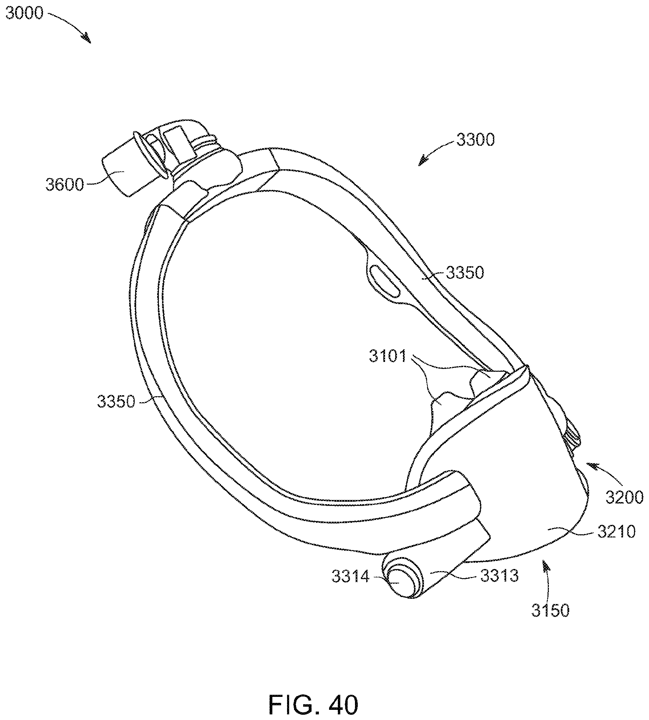

In the examples shown in Figs. 25-46, the chassis portion 3210 is shaped to curve from one lateral side of the patient's face in use to the other lateral side of the patient's face. The chassis portion 3210 may be substantially flush with the patient's cheeks at each lateral side. As shown in Fig. 29 for example, at either lateral side of the patient's face the chassis portion 3210 is substantially flush with the patient's cheeks, e.g. a line following the curvature of the chassis portion 3210 substantially aligns with the surface of the patient's cheeks. This flush relationship may help keep the patient interface 3000 low profile and/or provide for better side sleeping than if the sides of the chassis portion 3210 were proud of the patient's cheeks. The patient interface 3000 shown in Figs. 40-46 (described in more detail below) also has this structure and curvature of the cushion module 3150 which helps avoid disruptive forces, especially during side sleeping.

The chassis portion 3210 may be shaped substantially as a hyperbolic paraboloid. The membrane portion 3220 may also be shaped substantially as a hyperbolic paraboloid. In the examples shown in Figs. 25-46 the chassis portion 3210 and membrane portion 3220 are each in the shape of a hyperbolic paraboloid. It is to be understood that the chassis portion 3210 and membrane portion 3220 may each be shaped substantially as a hyperbolic paraboloid despite having one or more portions or features not forming part of the hyperbolic paraboloid shape. The chassis portion 3210 and membrane portion 3220 may be shaped substantially as hyperbolic paraboloids rotated 90 degrees with respect to each other, and may be joined to each other around the edges. In some examples either or both of the chassis portion 3210 and membrane portion 3220 may be shaped substantially as parabolic cylinders. That is, in some examples the chassis portion 3210 and/or the membrane portion 3220 may be as described elsewhere herein, but may have a parabolic cylinder shape, in an undeformed state prior to being donned by a patient. In further examples the chassis portion 3210 and/or the membrane portion 3220 may have a shape other than a hyperbolic paraboloid or parabolic cylinder.

For example, the membrane portion 3220 may have a curvature in the sagittal plane that, in an in-use orientation and prior to engagement with the patient's face, curves from a chin region of the patient's face towards the patient's nose superiorly and then anteriorly. After the patient interface 3000 has been donned and the membrane portion 3220 engages the patient's face, its shape will have more complex curvature to matching contours on the surface of the patient's face.

In a plane perpendicular to the sagittal plane (e.g. a horizontal plane or a posterosuperior-anteroinferior oriented plane), in an in-use orientation the membrane portion 3220 may have no curvature prior to engaging the patient's face due to being held sufficiently taut by the chassis portion 3210. After the patient interface 3000 is donned by the patient and the membrane portion 3220 engages the patient's face, the membrane portion 3220 may conform to the geometry of the patient's face, curving from a lateral side of the patient's face alongside one of the patient's cheeks anteriorly and then medially towards the sagittal plane of the user's head and then curving laterally and then posteriorly to the other lateral side of the patient's face alongside the other of the patient's cheeks. In the same plane, the chassis portion 3210 may also curve from a lateral side of the patient's face alongside one of the user's cheeks anteriorly and then medially towards the sagittal plane and then curve laterally and posteriorly to the other lateral side alongside the other of the user's cheeks.

### 5.3.5.6.6 Cushion module connections to positioning and stabilising structure

Figs. 25-46 show patient interfaces 3000 according to further examples of the present technology. The patient interfaces 3000 in these examples have various forms of positioning and stabilising structures 3300. All of the patient interfaces 3000 shown in Figs. 25-46 comprise a cushion module 3150 having a membrane portion 3220 which forms an oral portion 3102 of the seal-forming structure 3100 as well as supporting nasal pillows forming a nasal portion 3101 of the seal-forming structure 3100. The membrane portion 3220 may be as described above with reference to Figs. 18-20. It is to be understood that the positioning and stabilising structures 3300 described with reference to Figs. 25-46 and the way the cushion modules 3150 connect to the positioning and stabilising structure 3300 are applicable to other examples of the present technology, such as patient interfaces 3000 having separate nasal cushion modules 3160 and oral cushion modules 3170, unless context requires otherwise. Likewise, any features of the cushion modules 3150 described with reference to Figs. 25-46 are to be understood to be applicable to patient interfaces 3000 having an alternative positioning and stabilising structure 3300.

The patient interfaces 3000 shown in Figs. 47-67 have some similarities and differences in the positioning and stabilising structure 3300, in comparison to the positioning and stabilising structures 3300 shown in Figs. 25-46. It is to be understood that aspects of the positioning and stabilising structures 3300 described with reference to Figs. 25-46 may be applied to those of Figs. 47-67, and vice versa, unless context clearly requires otherwise.

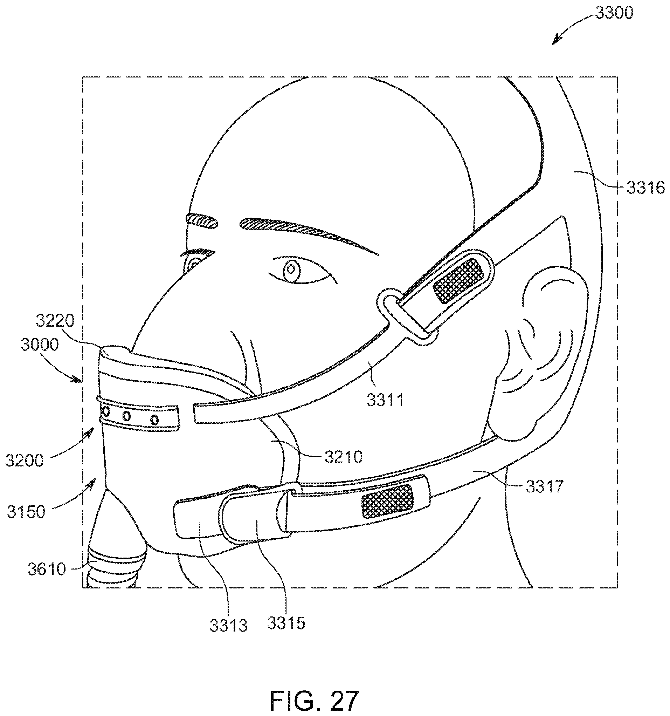

Figs. 25-30 show a patient interface 3000 according to one example of the present technology. Figs. 27-29 show the patient interface 3000, together with the positioning and stabilising structure 3300, in use on a patient.

In this example, the positioning and stabilising structure 3300 comprises a pair of upper straps 3316. Each upper strap 3316 is configured to be positioned on a respective side of the patient's head superior to a respective otobasion superior of the patient's head. In this example, the patient interface 3000 comprises a pair of upper arms 3311 connected to the chassis portion 3210. Each upper arm 3311 is configured to attach to a respective one of the upper straps 3316, as shown in Figs. 27-29. Each upper arm 3311 in this example extends superiorly and posteriorly with respect to the chassis portion 3210. The upper arms 3311 may also extend laterally with respect to the chassis portion 3210. The upper arms 3311 may be flexible in one or more directions but rigidised to bending in one or more other directions. For example, the upper arms 3311 may be able to bend towards the medial and lateral directions to accommodate variation in patient head sizes. However, the upper arms 3311 may be structured to resist bending in the superior-inferior directions, which may help translate force vectors from the upper straps 3316 to the seal-forming structure 3100. In some examples the upper arms 3311 are overmoulded to the chassis portion 3210. In other examples the upper arms 3311 may be formed separately and then mechanically connected to the chassis portion 3210 (e.g. with a snap fit connection).

The upper arms 3311 each provide an upper headgear connection point at which a strap portion of the positioning and stabilising structure 3300 is able to connect to the respective upper arm 3311. The upper arms 3311 may each comprise an upper headgear connection point in the form of an opening (e.g. an eyelet or slot) at an end thereof. In the example shown in Figs. 25-30, each upper strap 3316 of the positioning and stabilising structure 3300 passes through an opening in a respective upper arm 3311 and is looped back and secured to itself, for example by a hook-and-loop connection.

In the example shown in Figs. 25-30, the chassis portion 3210 is flexible (although stiff enough to support the membrane portion 3220 and to transfer sealing force vectors effectively). The chassis portion 3210 may be formed from mylar in this example. More particularly, the chassis portion 3210 may be flexible to bending in a horizontal plane (e.g. the chassis portion 3210 may be able to deform such that the lateral sides thereof are able to move closer together or further apart), but due to its geometry may not bend in the vertical planes.

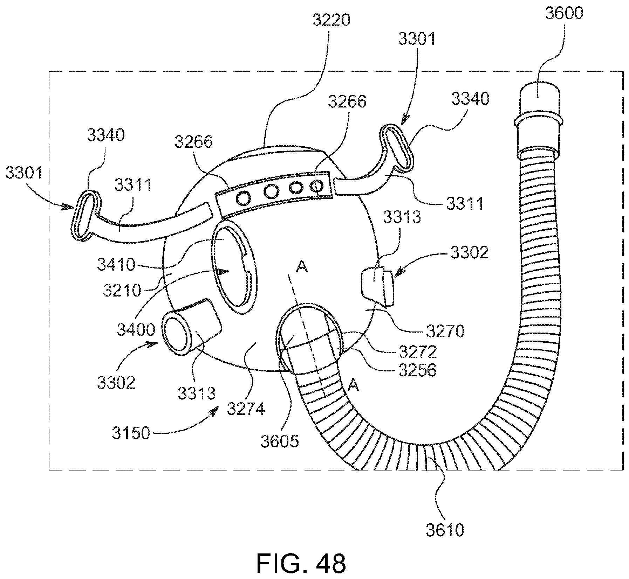

The chassis portion 3210 may comprise one or more wires extending from one lateral side of the chassis portion 3210 to the other lateral side which can be adjusted in shape to selectively adjust the shape (such as the curvature or width) of the chassis portion 3210. As shown in Fig. 25 in particular, the chassis portion 3210 comprises a pair of wires extending from one lateral side of the chassis portion 3210 to the other lateral side. The wires in this example connect between the locations at which the upper arms 3311 connect to the chassis portion 3210. In other examples the wires may be located such that their ends do not coincide with ends of the upper arms 3311. The wires may more generally be plastically deformable elements 3266 as described with reference to Fig. 48 for example.

In further examples there may be no wires. The chassis portion 3210 may be sufficiently flexible to adjust in shape in use without the user selectively adjusting its shape using wires. In some examples the upper arms 3311 and wires may be constructed together as an assembly and then attached to the chassis portion 3210. The patient or their clinician may plastically deform the wires to selectively adjust the shape of the chassis portion 3210 to achieve a good fit of the patient interface 3000 to the patient's particular anatomy. The flexibility of the chassis portion 3210 may also allow it to at least partially decouple lateral forces from the seal-forming structure 3100, such as disruptive forces that may occur during side sleeping if the chassis portion 3210 pushed against a pillow.

The upper arms 3311 of the patient interface 3000 shown in Figs. 25-30 are low profile with respect to the chassis portion 3210, e.g. they are almost flush with the external surface of the chassis portion 3210. As described above, the chassis portion 3210 in this example is structured to be flush with the surface of the user's cheeks in use, to keep a low profile. The upper arms 3311 are also close to flush with the external surface of the chassis portion 3210 such that the cushion module 3150 as a whole remains low profile with respect to the patient's face. The upper arms 3311 and the chassis portion 3210 are designed such that when worn, the patient interface 3000 sits flush with the cheeks and then follows the triangular shape of the nose. The final shape creates a smooth rounded compact mask volume under the nose and in-front of the mouth. This shape may advantageously be conducive to side sleeping during use. Advantageously, the cushion module 3150 contains no shoulder features adjacent the sides of the nose which could otherwise shunt the cushion module 3150 sideways during side sleeping.

The positioning and stabilising structure 3300 of the patient interface 3000 shown in Figs. 25-30 also comprises a pair of lower straps 3317, each lower strap 3317 configured to be positioned on a respective side of the patient's head inferior to a respective otobasion inferior of the patient's head. The patient interface 3000 also comprises a pair of lower arms 3313 extending from the chassis portion 3210, each lower arm 3313 configured to attach to a respective one of the lower straps 3317. Each lower strap 3317 may be attached to a headgear clip 3315 configured to releasably attach to the respective lower arm 3313, for example via a magnetic connection. As shown in Fig. 25, each lower arm 3313 comprises a lower headgear connection point 3314, which in this example comprises a magnet. Each lower arm 3313 may comprise a magnet attached to an end portion of the lower arm 3313, for example by encased and attached to the lower arm 3313 by overmoulding. The headgear clip 3315 may also comprise a magnet or may comprise a magnetic material. In other examples each lower headgear connection point 3314 may comprise a magnetic material and the headgear clips 3315 may comprise magnets. In some examples the lower arms 3313 are overmoulded to the chassis portion 3210. In other examples the lower arms 3313 may be formed separately and then mechanically connected to the chassis portion 3210 (e.g. with a snap fit connection).

In the example shown in Figs. 25-30, the patient interface 3000 comprises a short tube 3610 fluidly connected to the chassis portion 3210 at a proximal end. The short tube 3610 comprises a connection port 3600 at a distal end to receive a flow of air or other breathable gas from a conduit of an air circuit 4170 connected to an RPT device 4000 in use. The patient interface 3000 comprises a vent 3400, which in this example is provided at the connection of the short tube 3610 to the chassis portion 3210. The connection between the short tube 3610 and chassis portion 3210 may be a swivel connection, allowing the short tube 3610 to rotate with respect to the chassis portion 3210 to help avoid tube drag, and may be in the form of an elbow. The short tube 3610 may extend anteriorly and/or inferiorly from the cushion module 3150. The patient interface 3000 comprises an AAV (not shown) which may also be in the swivel connection.

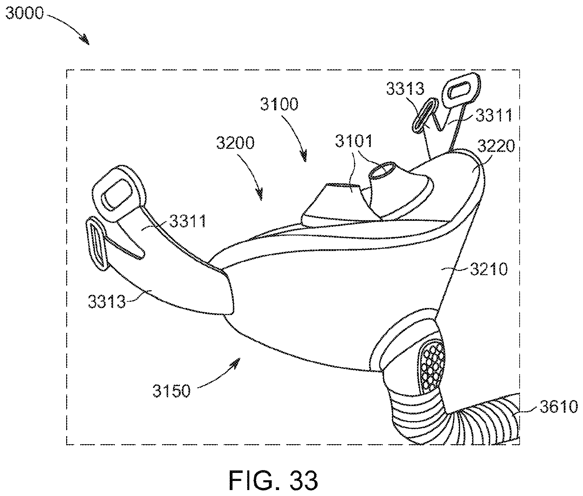

Figs 33-37 show a patient interface 3000 according to another example of the present technology. In this example the patient interface 3000 comprises a seal-forming structure 3100 of the type described with reference to Figs. 18-20 and 25-30, e.g. a nasal portion 3101 of the seal-forming structure 3100 in the form of nasal pillows and a membrane portion 3220 forming an oral portion 3102 of the seal-forming structure 3100. The chassis portion 3210, membrane portion 3220 and seal-forming structure 3100 may be as described with reference to the patient interface 3000 shown in Figs. 18-20. The patient interface 3000 comprises a positioning and stabilising structure 3300 comprising a pair of upper straps 3316 and pair of lower straps 3317. In this example the patient interface 3000 comprises a pair of upper arms 3311, each upper arm 3311 configured to attach to a respective one of the upper straps 3316. Each upper arm 3311 may extend superiorly and posteriorly with respect to the chassis portion 3210.

In the example shown in Figs. 33-37, the patient interface 3000 also comprises a pair of lower arms 3313 extending from the chassis portion 3210. Each lower arm 3313 is configured to attach to a respective one of the lower straps 3317 of the positioning and stabilising structure 3300. In this particular example, each upper arm 3311 extends from a respective one of the lower arms 3313. The lower arms 3313 extend posteriorly from the chassis portion 3210 and the upper arms 3311 extend partially superiorly from the lower arms 3313. Accordingly, the upper arms 3311 are not attached directly to the chassis portion 3210. This provides for a single connection point on each lateral side of the patient interface 3000 between the positioning and stabilising structure 3300 and the cushion module 3150. In other examples, each lower arm 3313 may extend from a respective one of the upper arms 3311, and there may be a single point of attachment between the arms 3311 and 3313 and the chassis portion 3210 on each lateral side of the patient interface 3000, made by connection of the lower arms 3313 to the chassis portion 3210.

In the example shown in Figs. 33-37, each of the upper arms 3311 and lower arms 3313 comprises an eyelet at the end thereof, through which a respective one of the upper straps 3316 or lower straps 3317 is looped through and secured back to itself, for example with a hook-and-loop connection. This is the same manner of connection between the headgear straps and arms as described above with reference to Figs. 25-30. Each of the upper straps 3316 and lower straps 3317 are connected to and extend from a crown strap 3318 which overlies a superior surface of the patient's head in use and extends inferiorly on either lateral side of the patient's head over an at least partially posteriorly facing surface of the patient's head to connect to the upper straps 3316 and lower straps 3317. The crown strap 3318 engages superior and posterior surfaces of the patient's head to provide an anchor for the upper straps 3316 and lower straps 3317, allowing the upper straps 3316 and lower straps 3317 to be tensioned between the cushion module 3150 and the crown strap 3318.

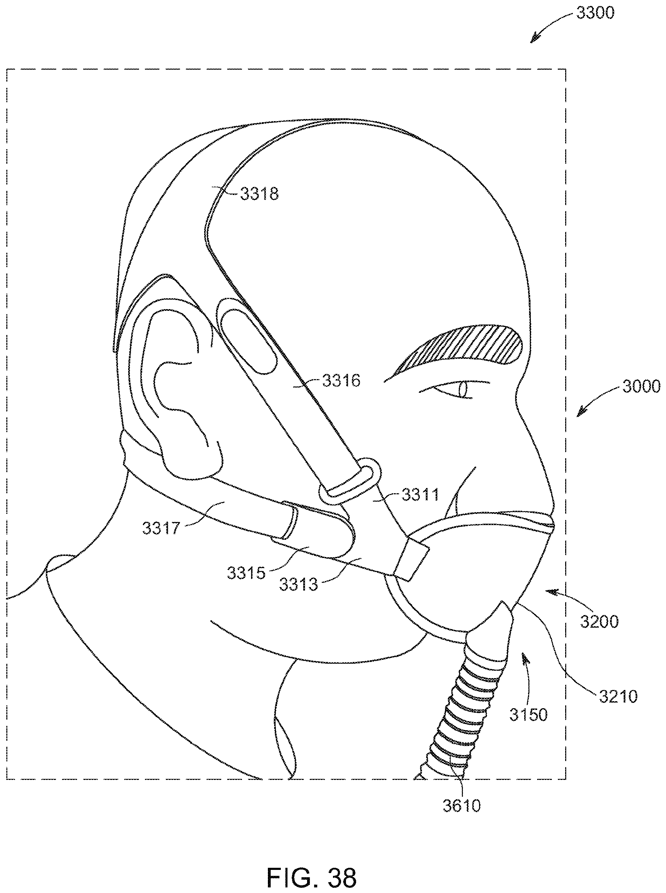

Fig. 38 shows a patient interface 3000 similar to that shown in Figs. 33-37 but having a positioning and stabilising structure 3300 providing an alternative type of connection between the lower strap 3317 and lower arm 3313. In this example the lower arm 3313 extends from the chassis portion 3210 and the upper arm 3311 extends from the lower arm 3313, in the same manner as shown in Figs. 33-37. However, in Fig. 38 the lower strap 3317 comprises a headgear clip 3315 configured to releasably attach to a headgear connection point on the lower arm 3313. The headgear clip 3315 may magnetically attach to the lower arm 3313 (e.g. in the same manner as the lower headgear connection point 3314 shown in and described above with reference to Fig. 25). This may allow for a quick-connect and quick-disconnect attachment between the lower straps 3317 and lower arms 3313, which may allow the patient to quickly disconnect the lower straps 3317 from the lower arms 3313 during doffing of the patient interface 3000 and to quickly connect them during donning. The patient interface 3000 in this example also comprises a crown strap 3318 from which the upper straps 3316 and lower straps 3317 extend.

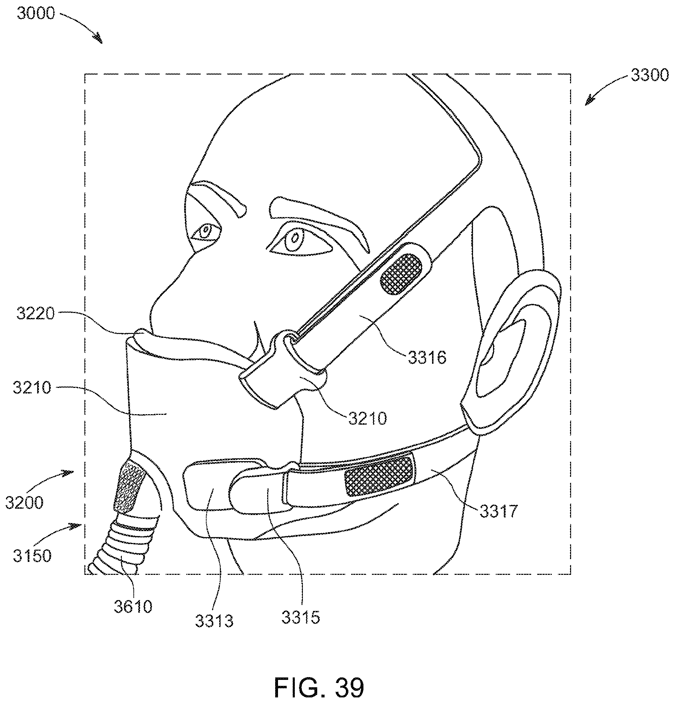

Fig. 39 shows a patient interface 3000 according to another example of the present technology, in which the cushion module 3150 comprises two headgear connections on each lateral side thereof. In this example the patient interface 3000 does not comprise upper arms 3311. In this example the cushion module 3150 comprises an eyelet on each lateral side thereof. The eyelets may be formed by portions of the chassis portion 3210 or may be formed by components attached to the chassis portion 3210, in examples. Each of the upper straps 3316 is able to be looped through a respective one of the eyelets provided to the cushion module 3150 and secured back to itself, for example with a hook-and-loop connection. The cushion module 3150 also comprises lower arms 3313 to which lower straps 3317 of the positioning and stabilising structure 3300 are able to connect. The lower straps 3317 in this example comprise headgear clips 3315 configured to releasably attach to lower headgear connection points (which may be in the same form as the lower headgear connection points 3314 shown in and described with reference to Fig 25) on the lower arms 3313. The patient interface 3000 in this example also comprises a crown strap 3318 from which the upper straps 3316 and lower straps 3317 extend.

Figs. 40-46 show a patient interface 3000 according to another example of the present technology. In this example the patient interface 3000 comprises a similar cushion module 3150 to that described with reference to Figs. 33-39. Many features and properties of the membrane portion 3220, chassis portion 3210 and undercushion 3225 (described below) are the same as described with reference to Figs. 33-39. However, in this example the patient interface 3000 comprises a positioning and stabilising structure 3300 including conduit headgear. As illustrated, the positioning and stabilising structure 3300 comprises a pair of gas delivery tubes 3350. The chassis portion 3210 comprises a pair of openings at which the gas delivery tubes 3350 are configured to connect to the chassis portion, the openings being sized and structured to receive a flow of air from the gas delivery tubes 3350 at the therapeutic pressure for breathing by a patient. The gas delivery tubes 3350 are configured to convey a supply of air from a connection port 3600 on top of the patient's head to a plenum chamber 3200 within the cushion module 3150 defined by the chassis portion 3210 and membrane portion 3220. The gas delivery tubes 3350 may be configured to be disconnected from the chassis portion 3210, for example for cleaning. Features of positioning and stabilising structures 3300 in the form of conduit headgear are described elsewhere herein, for example with reference to Fig. 7-13 and 18-24.

In this particular example, the gas delivery tubes 3350 comprise a textile outer layer. The textile material may be highly comfortable to the user and may give the patient interface 3000 an appearance more like sleepwear than a medical device. The gas delivery tubes 3350 may be thermoformed to shape, may comprise a foam inner layer and may comprise a sealing layer in the form of a film applied to an inside surface of each gas delivery tube 3350. The patient-facing exterior surface of each gas delivery tube 3350 may be substantially flat and may be wide, which may distribute force on the patient's head and face over a large area, providing for a comfortable fit.

In the example shown in Figs. 40-46, each of the gas delivery tubes 3350 connects to a respective lateral side of the cushion module 3150 and, in particular, connects to the chassis portion 3210. The chassis portion 3210 defines a pair of plenum chamber inlet ports through which the plenum chamber 3200 is able to receive the supply of air at the therapeutic pressure for breathing by the patient. The attachment of the gas delivery tubes 3350 to the chassis portion 3210 helps with stability of the patient interface 3000. The gas delivery tubes 3350 function as both conduits and part of the positioning and stabilising structure 3300 to supply the air and help hold the cushion module 3150 in place with a good seal and with good stability. The cushion module 3150 in this example also comprises a tight curvature in the chassis portion 3210, which may make the chassis portion 3210 highly flexible to bending about the vertical centreline and readily able to absorb potentially disruptive forces that it may experience in use, for example during side sleeping.

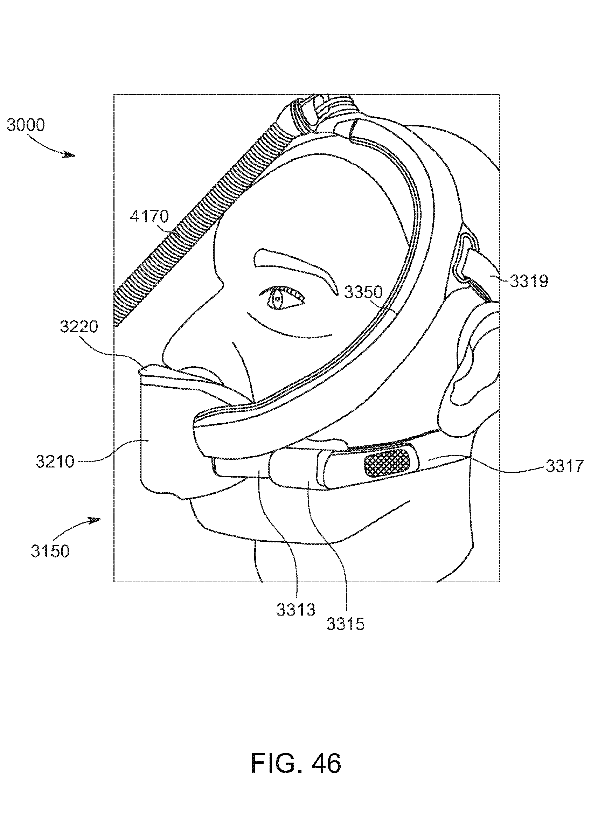

As shown in Fig. 46, the positioning and stabilising structure 3300 comprises a pair of lower straps 3317 configured to be positioned on a respective side of the patient's head inferior to a respective otobasion inferior of the patient's head. The lower straps 3317 in this example each comprise a headgear clip 3315 configured to releasably attach to the cushion module 3150. The headgear clips 3315 may attach to the chassis portion 3210 with a magnetic connection. As shown in Figs. 40, 41, 44 and 45, the cushion module comprises lower arms 3313 having lower headgear connection points 3314 to which the headgear clips 3315 on the lower straps 3317 are able to releasably attach. The lower headgear connection points 3314 may be the same as shown in and described with reference to Fig. 25. The lower straps 3317 in this example are elastically extendable to allow the positioning and stabilising structure 3300 to tolerate changes in the patient's head orientation and in particular changes in the distance between the patient's jaw and the posterior surface of the patient's neck proximate the base of the user's head.

The patient interface 3000 in this example comprises a pair of lower arms 3313 extending from the chassis portion 3210, each lower arm 3313 configured to attach to a respective one of the lower straps 3317 of the positioning and stabilising structure 3300. Each lower arm 3313 may comprise a lower headgear connection point at which a respective one of the lower straps 3317 is able to connect, for example a magnetic connection point (e.g. a magnet or magnetic material). In this example of the present technology, on each lateral side of the chassis portion 3210, the respective lower arm 3313 is connected to the chassis portion 3210 immediately adjacent to the respective gas delivery tube 3350. This arrangement has the appearance of a single headgear connection on each lateral side of the cushion module 3150, which may make the patient interface 3000 look smaller and easier to fit.

The positioning and stabilising structure 3300 further comprises a backstrap 3319 configured to connect between the gas delivery tubes 3350 and configured to overlie or lie inferior to the occipital bone of the patient's head in use. The backstrap 3319 may be connected to the lower straps 3317 behind the patient's head and/or neck. As illustrated, the backstrap 3319 is located on each side of the patient's head with at least a portion of the backstrap 3319 being positioned superior to the otobasion superior. The backstrap 3319 may connect to the gas delivery tubes 3350 superior to the otobasion superior.

### 5.3.5.6.7 Undercushion

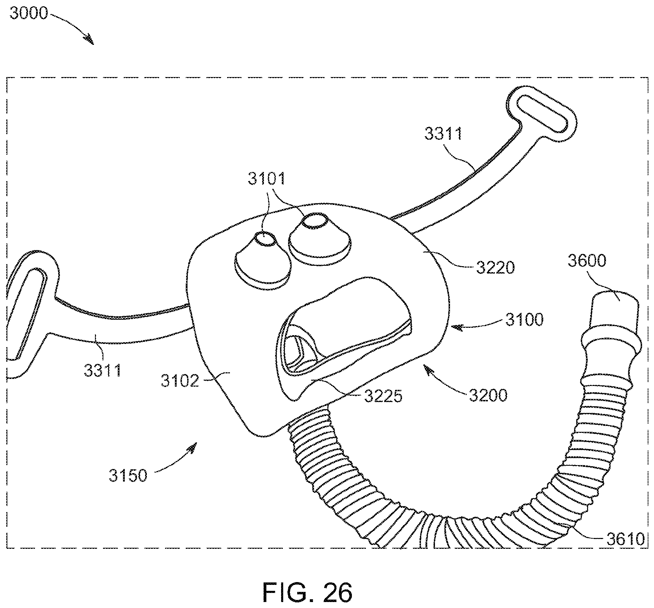

In some examples of the present technology, the patient interface 3000 comprises an undercushion. The patient interface 3000 shown in Figs. 25-30 includes an undercushion 3225 behind the membrane portion 3220, visible in Fig. 26. The undercushion 3225 may be attached to the chassis portion 3210 and may be configured to engage an internal surface of the membrane portion 3220 to support the membrane portion 3220 in use. The undercushion 3225 may be configured to engage any one or more of a chin region of the membrane portion 3220 configured to contact a chin region of the patient's face in use, cheek regions of the membrane portion 3220 configured to contact the patient's cheeks in use, and a nose region of the membrane portion 3220 configured to contact the patient's nose in use. In some examples the undercushion 3225 may be located behind cheek regions of the membrane portion 3220 and behind a chin region of the membrane portion 3220. In some examples the undercushion may be located behind only the chin region of the membrane portion 3220. It is to be understood that in some examples of the present technology, the patient interface 3000 has no undercushion.

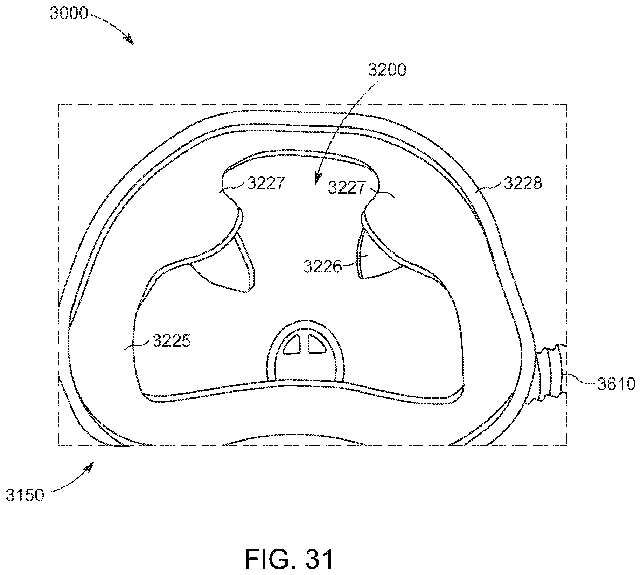

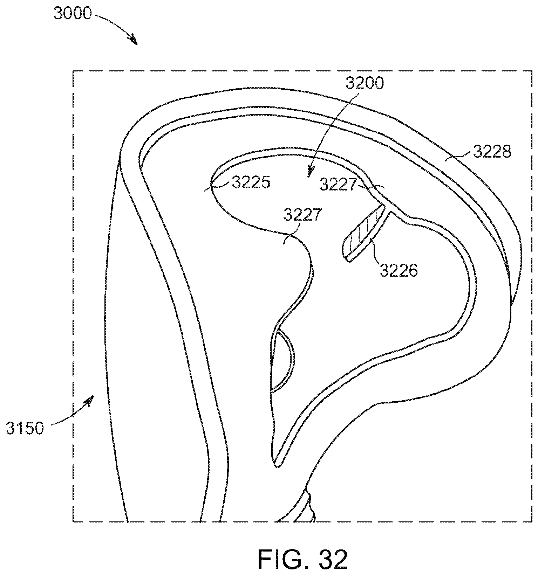

Figs. 31 and 32 show views of the patient interface 3000 shown in Figs. 25-30 with portions removed, including the membrane portion 3220, to reveal the undercushion 3225. In this example the undercushion 3225 engages a chin region of the membrane portion 3220, cheek portions of the membrane portion and a nose region of the membrane portion 3220. The undercushion 3225 is configured to engage the membrane portion 3220 around an entire periphery of the membrane portion 3220 in this example.

The undercushion 3225 may be formed from a flexible and resilient material, such as an elastomer (e.g. silicone or TPE). The undercushion 3225 may be moulded onto the chassis portion 3210, for example if the undercushion 3225 is formed from an elastomer and the chassis portion 3210 is formed from Mylar, polycarbonate or the like, or a stiff textile material. The undercushion 3225 may be in the form of a flange and may extend inwardly from a periphery of the chassis portion 3210. The undercushion 3225 may be structured to behave as a cantilever support to hold the membrane portion 3220 against the patient's face in use. In some examples the undercushion 3225 is formed from the same material as the chassis portion 3210 and may be integrally formed with the chassis portion 3210. In further examples, the undercushion 3225 may be adhered to the chassis portion 3210.

The undercushion 3225 may deform together with the membrane portion 3220 when engaging with the patient's face, to conform to the shape of the patient's face. A superior portion of the undercushion 3225 shown in Figs. 31-32 is configured to flex to conform to the patient's nose and cheeks when the patient interface 3000 is donned by the patient. The superior portion of the undercushion 3225 may have a thickness within the range of 0.5mm-1.5mm, within the range of 0.75-1.25mm, or may have a thickness of 1mm, for example. The inferior portion of the undercushion 3225 is configured to flex to conform to a chin region (e.g. proximate the patient's supramenton and lip inferior, superior to the mental protruberance). The inferior portion of the undercushion 3225 may have a varying thickness. For example, a medial portion of the inferior portion of the undercushion 3225 may be thinner than lateral portions of the inferior portion of the undercushion 3225. This may provide for patient comfort in use.

In the example shown in Figs. 31-32, the undercushion 3225 comprises a pair of wing portions 3227. The wing portions 3227 extend inwardly with respect to an outer periphery of the undercushion 3225. Each wing portion 3227 is configured to urge the membrane portion 3220 to seal against the patient's face in a region proximate a respective one of the patient's nasal alae, such as a region between the patient's nasolabial sulcus and lip superior. The wing portions 3227 may urge the membrane portion 3220 against regions of the patient's face inferior and/or lateral to the patient's nasal alae (e.g. the corners of the nose). In examples in which the undercushion 3225 is in the form of a flange, the wing portions 3227 may be extended portions of the flange (e.g. flange portions extending inwardly to a greater extent than other flange portions). In some examples the wing portions 3227 are integrally formed with other portions of the undercushion 3225. In other examples the wing portions 3227 may be formed separately to other portions of the undercushion 3225. The undercushion 3225 may be formed from multiple separate pieces in some examples. In the example shown in Figs. 31-32 the undercushion 3225 is of unitary construction. The wing portions 3227 may each extend medially in an in-use orientation of the patient interface 3000. As shown in Figs. 31-32, the undercushion 3225 may define an opening through which air can flow from the plenum chamber 3200 to the patient's airways. The wing portions 3227 may split this opening into a nasal portion of the opening and an oral portion of the opening.

The patient interface 3000 in the example shown in Figs. 25-32 also comprises a pair of ribs 3226 each extending between the chassis portion 3210 and a respective one of the wing portions 3227 to provide support for the respective wing portion 3227. The ribs 3226 may provide support for the wing portions 3227 by resisting compression and/or buckling. The ribs 3226 may functions as struts, resisting movement of the wing portions 3227 towards the internal surface of chassis portion 3210. The ribs 3226 may be formed from the same material as the undercushion 3225, such as silicone by way of example, and may be integrally formed with the undercushion 3225. In other examples, the ribs 3226 may be formed from the same material as the chassis portion 3210 and may be integrally formed with the chassis portion 3210. The ribs 3226 may be 1mm thick, in an example. In some examples the patient interface 3000 does not comprise ribs 3226 and the undercushion 3225 alone supports the membrane portion 3220.

The undercushion 3225 of the patient interface 3000 shown in Figs. 25-32 also comprises a rim 3228. The rim 3228 may space a portion of the membrane portion 3220 from the undercushion 3225. The membrane portion 3220 in this example is attached to the rim 3228 and the rim 3228 is structured to space the membrane portion 3220 from the undercushion 3225 at least at the periphery of the undercushion 3225. The rim 3228 of the undercushion 3225 may provide a connection surface at which the membrane portion 3220 is connected to the undercushion 3225, the connection surface being proud of the remainder of the undercushion 3225 (the remainder of the undercushion 3225 being a flange in the example shown in Figs. 31-32). The connection surface may be 2mm proud of the remainder of the undercushion 3225. The connection surface may be 2mm wide, for example. The rim 3228 may have a square or rectangular cross section. In some examples the connection surface may be more (or less) than 2mm wide and may be more (or less) than 2mm proud of the remainder of the undercushion. The membrane portion 3220 may be adhered to the connection surface.

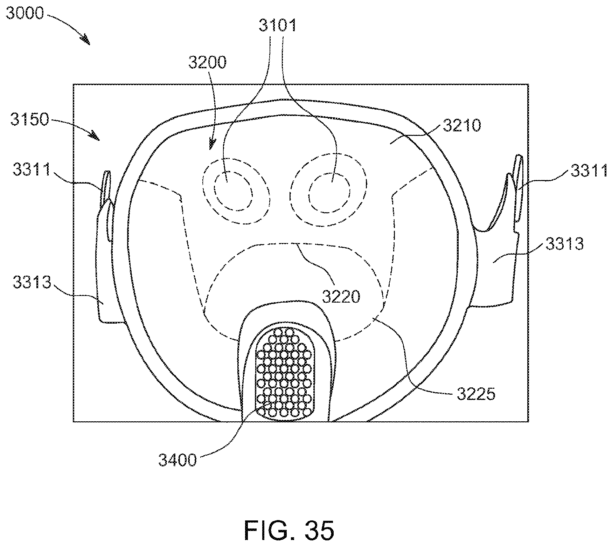

The patient interface 3000 shown in Figs. 33-37 also comprises an undercushion 3225. Fig. 35 shows the undercushion 3225 by way of hidden detail behind the chassis portion 3210. The nasal pillows forming the nasal portion 3101 of the seal-forming structure 3100 are also visible as hidden detail in this view, along with a peripheral edge of the oral opening in the membrane portion 3220. In this example of the present technology, the undercushion 3225 does not extend around the entire periphery of the chassis portion 3210. The undercushion 3225 extends from a location proximate one of the patient's cheeks in use inferiorly and medially to intersect with the sagittal plane and then extends laterally and superiorly to a location proximate the other one of the patient's cheeks. The undercushion 3225 may be configured to engage a chin region of the membrane portion 3220 configured to contact the patient's chin region, and may be configured to engage cheek regions of the membrane portion 3220 configured to contact the patient's cheeks in use. However, the undercushion 3225 does not extend into a nasal portion of the cushion module 3150. That is, in this example there is no undercushion 3225 behind a nasal portion of the membrane portion 3220 configured to contact the patient's nose. The undercushion 3225 may comprise ends located behind the membrane portion 3220 at the user's cheeks on either side of the patient's lip superior and/or nose. The lack of the undercushion 3225 behind the membrane portion 3220 at the patient's nose may provide for a cushion module 3150 that is able to be more easily or cost effectively manufactured, while still having an undercushion that supports the membrane portion 3220 against at least the patient's cheeks and chin. The lack of an undercushion 3225 in the nasal region of the cushion module 3150 may also provide for better comfort as the patient's nose does not press into an undercushion 3225 and may also provide for a better seal as the thin membrane portion 3220 may be freely able to deform to allow the nasal pillows to wrap around the nose and move into the correct orientation for sealing to the extent necessary for the patient's particular facial geometry. Furthermore, tension in the membrane portion 3220 alone may be sufficient to hold the nasal pillows in place for set-up and use.

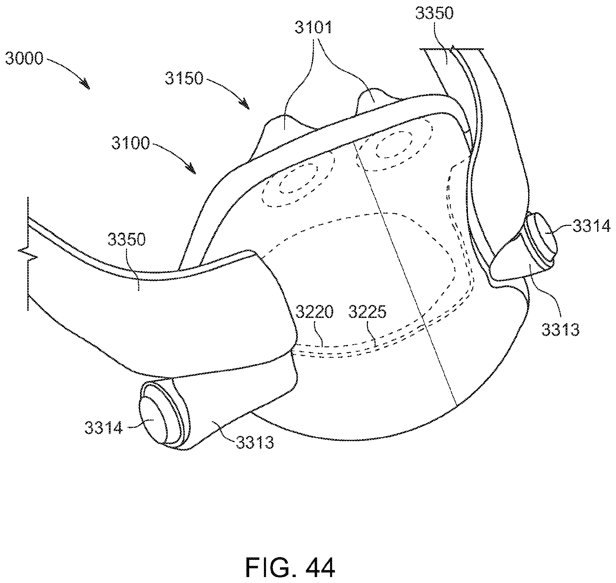

The patient interface shown in Figs. 40-46 also comprises an undercushion 3225. The undercushion 3225 is shown by way of hidden detail in Fig. 44. As illustrated, it is substantially the same as the undercushion 3225 of the patient interface 3000 shown in Figs. 33-37.

### 5.3.5.7 Plenum chamber connected to frame

Figs. 14-17 show an example of a patent interface 3000 in which the seal-forming structure 3100 and membrane portion 3220 are substantially the same as in the example shown in Figs. 7-13, although there are other differences. All disclosure above regarding the plenum chamber 3200, chassis portion 3210, membrane portion 3220 and seal-forming structure 3100 is to be understood to be applicable to the example shown in Figs. 14-17, unless stated otherwise or the context clearly requires otherwise. In this example, the patient interface 3000 does not have conduit headgear. Instead, the patient interface 3000 comprises a positioning and stabilising structure 3300 comprising a pair of headgear strap portions 3310 (which may be rigidised in some examples). The positioning and stabilising structure 3300 holds the plenum chamber 3200 and seal-forming structure 3100 in position in use. The patient interface 3000 also comprises a frame 3320 and short tube 3610. The plenum chamber 3200 and seal-forming structure 3100 in this example form a cushion module 3150.

### 5.3.5.7.1 Strap portions

Each of the strap portions 3310 is located on a respective side of the patient's head in use. In some examples the strap portions 3310 are connected to each other posterior to the patient's head, for example by a buckle. In other examples the strap portions 3310 are integrally formed with each other. That is, they may be portions of a single length of headgear strap. Each strap portion 3310 may lie superior to a respective otobasion superior of the patient's head. The strap portions 3310 may be configured to overlie the parietal bones of the patient's head in use. In some examples the strap portions 3310 may be connected to or formed with a bifurcated strap portion between the two strap portions 3310 configured to lie against posterior and/or posterosuperior surfaces of the patient's head.

The overall length of the strap portions 3310 may be selectively adjustable by the user, for example with a buckle between the two strap portions 3310. In some examples the strap portions 3310 may be elastically extendable such that when donned by a patient tension is created in the strap portions 3310 to pull the seal-forming structure 3100 towards the patient's airways and resist forces tending to separate the seal-forming structure 3100 from the patient's face.

### 5.3.5.7.2 Frame

The patient interface 3000 in the example shown in Figs. 14-17 also comprises a frame 3320. The plenum chamber 3200 of the patient interface 3000 in this example is connected to the frame 3320. The chassis portion 3210 may be configured to connect to the frame 3320. Each of the strap portions 3310 are also connected to the frame 3320. The frame 3320 may be substantially rigid (e.g. difficult to deform by finger pressure alone) and may be formed from polycarbonate as one example. In other examples the frame 3320 may be semi-rigid (e.g. able to flex to at least some extent by finger pressure and/or when there is tension in the headgear strap portions 3310). The frame 3320 may be curved in a left-to-right direction and may comprise a curvature corresponding to a curvature of an anterior side of the chassis portion 3210. In some examples the curvature of the frame 3320 may follow a curvature of an anterior boundary of the membrane portion 3220.

The plenum chamber 3200 may be larger than the frame 3320 such that the chassis portion 3210 extends about 2-3mm past the frame 3320 on each side of the frame.

### 5.3.5.7.3 Short tube

While the patient interface 3000 shown in Figs. 7-13 includes conduit headgear and the plenum chamber 3200 receives a flow of air at the therapeutic pressure via gas delivery tubes 3350, the patient interface 3000 shown in Figs. 14-17 comprises a short tube 3610 from which the plenum chamber 3200 receives a flow of air. The plenum chamber 3200 comprises a central opening through which the flow of air is received from the short tube 3610. The central opening of the plenum chamber 3200 may form a plenum chamber inlet port.

The short tube 3610 may be a short length of tubing having a connection port 3600 at an end thereof distal from the patient. A longer conduit forming part of the air circuit 4170 between RPT device 4000 and patient interface 3000 may fluidly connect to the short tube 3610 at the connection port 3600. The short tube 3610 may comprise a swivel connector forming the connection port 3600. The short tube 3610 may advantageously function to at least partially decouple a conduit connected to the connection port 3600 from the seal-forming structure 3100. The short tube 3610 may be considered to form part of the patient interface 3000.

The frame 3320 may also comprise a central opening through which the flow of air passes to enter the plenum chamber 3200. The central opening in the frame 3320 may be aligned with the central opening in the plenum chamber 3200 and may be aligned with the short tube 3610. In this particular example the plenum chamber 3200 connects to the frame 3320 around a periphery of the central opening in the frame 3320. The frame 3320 in this example comprises a flange about the periphery of the central opening to which the plenum chamber 3200 is able to connect.

The plenum chamber 3200 also comprises a chassis portion 3210 and a membrane portion 3220 like the example shown in Figs. 7-13. The central opening in the plenum chamber 3200 may be formed in the chassis portion 3210. The chassis portion 3210 may be formed from silicone and may connect to the frame 3320 about the central opening of the frame 3320 with a stretch fit connection.

### 5.3.5.7.4 Connection between frame and strap portions

In the example shown in Figs. 14-17, the plenum chamber 3200 comprises two lateral openings through which air is able to be vented from the plenum chamber 3200, for example for gas washout. Each lateral opening in the plenum chamber 3200 may be on a respective lateral side of the central opening and may be formed by a hole in the chassis portion 3210. Each lateral opening may be aligned in use with a corresponding vent 3400 in the frame 3320. Air from the plenum chamber 3200 may pass through the lateral openings in the chassis portion 3210 and then through the corresponding vents 3400 in the frame 3320. Each vent 3400 may be formed by one or more openings in the frame 3320 through which air can pass.

The frame 3320 may comprise lateral openings corresponding to the lateral openings in the chassis portion 3210. The frame 3320 may comprise flanges around the lateral openings in the frame 3320 and the lateral openings in the chassis portion 3210 may stretch fit over the flanges around the lateral openings in the frame 3320. The chassis portion 3210 may be connected to the frame 3320 at the central opening and each lateral opening of the frame 3320. In the example shown in Figs. 14-17, the lateral openings in the frame 3320 each comprise a plurality of holes forming a vent 3400.

The headgear strap portions 3310 in this example are configured to connect to the frame 3320 on an opposite side of the frame 3320 to the plenum chamber 3200. The headgear strap portions 3310 each comprise a connector 3312 configured to attach to a corresponding connection point on the frame 3320. Each connector 3312 may attach to a respective connection point at a location corresponding to a respective vent 3400 in the frame. For example, the vent 3400 may be provided around or formed together with the headgear strap connection point. Accordingly, in this example, the patient interface 3000 comprises a split vent configuration.

### 5.3.5.8 Textile chassis portion

Referring to Figs. 47-55, in some examples the patient interface 3000 may comprise a chassis portion 3210 constructed at least partially from a textile material. In some examples the textile is coated with an air impermeable coating, for example a silicone coating. In some examples the chassis portion 3210 is formed from Breathoprene. In some examples the chassis portion 3210 may be formed from foam. The chassis portion 3210 may form part of a cushion module 3150 of the patient interface 3000.

The chassis portion 3210 may be constructed to be sufficiently stiff that it maintains a predetermined three-dimensional shape throughout the patient's respiratory cycle. The stiffness may be as a result of the geometry of the chassis portion 3210 and/or the chassis portion 3210 may be thermoformed such that material forming the chassis portion 3210 (e.g. a textile material) is inherently stiffened. In some examples, the chassis portion 3210 may comprise folded portions which contribute to its stiffness. For example, portions of the textile may be folded and joined to other portions, thereby creating a structure which can maintain a three-dimensional shape throughout the patient's respiratory cycle.

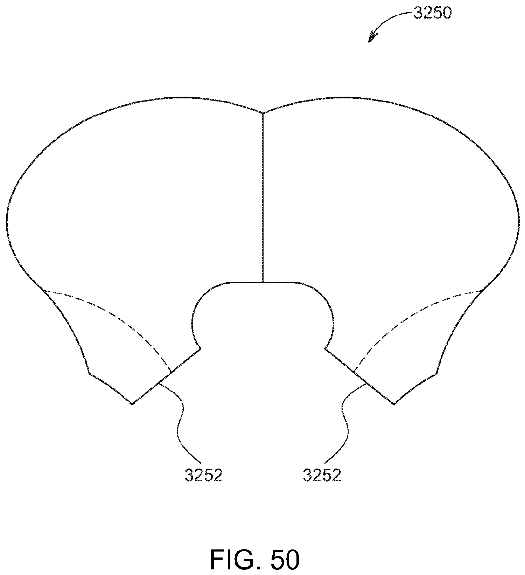

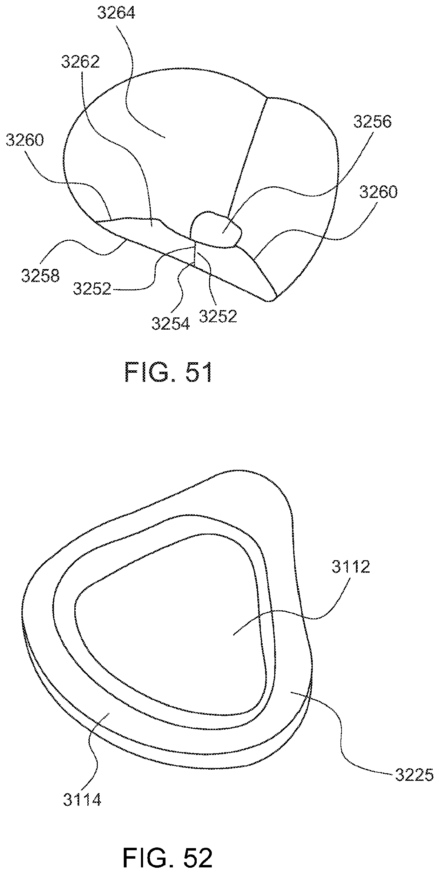

Referring next to Fig. 50, one example of a blank 3250 for a textile chassis portion 3210 is shown. Fig. 51 shows the blank 3250 with two lower edges 3252 of the blank 3250 joined to each other such that the blank 3250 adopts a generally conical or frusto-conical form, the joint 3254 between the two edges 3252 being located on an inferior portion of the chassis portion 3210. In the example shown, the chassis portion 3210 has no seams or joins superior to an opening 3256 through which an inlet port connector 3605 of the patient interface 3000 extends, in use. In some examples the chassis portion 3210 may appear substantially circular or elliptical when viewed from the front.

As shown in Fig. 51, an inferior portion of the blank 3250 is folded inwardly along a laterally extending fold line 3258, and lateral edges 3260 of a flap 3262 formed by this fold 3258 are connected to an inner surface 3264 of the chassis portion 3210. In this way the chassis portion 3210is made sufficiently stiff or rigid to maintain its three-dimensional shape throughout the patient's respiratory cycle.

In examples in which the textile chassis portion 3210 is thermoformed, it may not be necessary to include the flap 3262 shown in Fig. 51 and the shape of the blank 3250 may be varied accordingly.

In examples, a plastically deformable element 3266, for example a thin steel wire or ribbon, may extend laterally across the chassis portion 3210, as best seen in Fig. 48. The plastically deformable element 3266 may be deformed by the patient to make chassis portion 3210 wider or narrower, as required.

Referring next to Fig. 47 in particular, in some examples the chassis portion 3210 has a non-zero negative first principal curvature PC1 and a substantially zero second principal curvature PC2, wherein the second principal curvature PC2 is substantially parallel, in use, to the patient's sagittal plane.

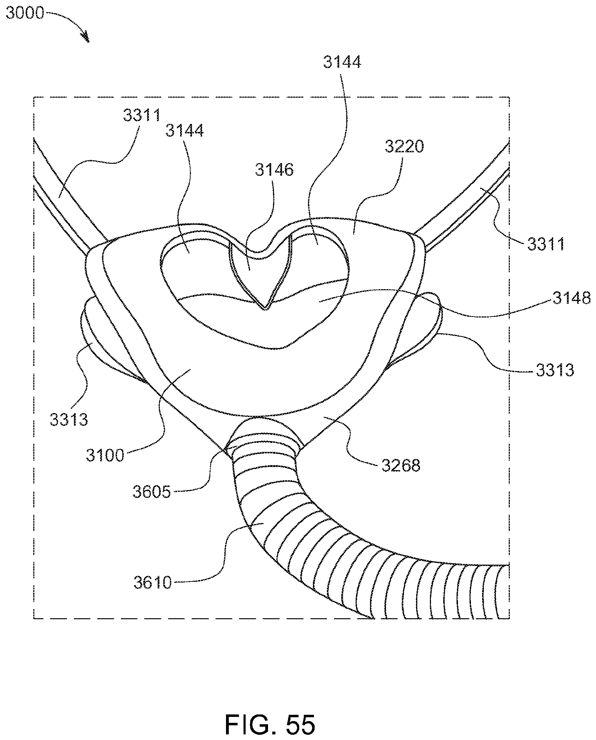

In examples, the patient interface 3000 is provided with an inlet port connector 3605 which extends through an inferior wall 3268 of the chassis portion 3210, as best seen in Fig. 55. In examples, the inlet port connector 3605 has a central axis A-A which extends in a substantially inferior direction in use, shown in Fig. 48 for example.

The inlet port connector 3605 may be configured to fluidly connect to a short tube 3610 as shown in Figs. 47-49, for example, or as described in any other example elsewhere herein. The short tube 3610 may connect to the inlet port connector 3605 at a proximal end of the short tube 3610 and may comprise a connection port 3600 for connection to an RPT device 4000, for example via a longer conduit of an air circuit 4170. In other examples no short tube 3610 is provided and the inlet port connector 3605 provides a connection port 3600 of the patient interface 3000 to connect to an RPT device 4000 via a conduit of an air circuit 4170.

In some examples, the patient interface 3000 may be very compact, such that an exterior of the inlet port connector 3605 is in contact with, or is very close to, a foam undercushion 3225, and is contact with an anterior wall of the chassis portion 3210. In some examples, for example as shown in Fig. 48, the anterior wall 3270 of the chassis portion 3210may have a cutout 3272 to allow the inlet port connector 3605 to protrude therethrough. In examples, the depth (e.g. distance from textile membrane portion 3220 to the anterior wall 3270 of the chassis portion 3210) of the most inferior portion of the cushion module 3150 may be substantially equal to the diameter of the inlet port connector 3605 plus the thickness of the foam undercushion 3225. The depth of the superior-most portion of the cushion module 3150 may be substantially equal to the thickness of the foam undercushion 3225 plus the thickness of the textile chassis portion 3210.

In some examples, an exterior surface of the chassis portion 3210, at a location 3274 immediately adjacent the inlet port connector 3605, has a radius which is substantially equal to a radius of an immediately adjacent portion of the exterior of the inlet port connector 3605. In examples, a central anterior portion 3276 (indicated in Fig. 47) of the chassis portion 3210, extending from the top of the chassis portion 3210 to the bottom of chassis portion 3210, may have substantially the same radius as a radius of the inlet port connector 3605.

The patient interfaces 3000 shown in Fig. 47 and 48 comprise a vent 3400, which may be as described elsewhere herein. In one form the patient interface 3000 may comprise a vent module 3410 comprising the vent 3400, the vent module 3410 being releasably connectable to the chassis portion 3210 by the patient, such that the vent 3400 may be disconnected from the chassis portion 3210 and reconnected, for example for cleaning or replacement. The vent may take any form described herein.

### 5.3.5.8.1 Headgear connectors

In examples, the patient interface 3000 comprises a pair of superior headgear connectors 3301 and a pair of inferior headgear connectors 3302. In the examples shown in Figs. 47 and 48 for example, the superior headgear connectors 3301 comprise upper arms 3311, which may be rigidised so as to be resiliently deformable but able to resist bending in one or more directions. Each upper arm 3311 may be provided with a strap connector portion 3340 at a distal end thereof. The strap connector portions 3340 may comprise loops, slots or the like.

The inferior headgear connectors 3302 in the Fig. 47 example comprise lower arms 3313, which may be rigidised as described in relation to the upper arms 3311. Any suitable type of headgear connector may be used for the inferior headgear connectors 3302, for example magnetic connectors provided to ends of the lower arms 3313.

In some examples, one or more of the headgear connectors 3301, 3302 may be releasably connected to the chassis portion 3210. That is, the connector itself may be repeatedly connected and disconnected from the chassis portion 3210 (rather than the connector simply being repeatedly connectable and disconnectable from the headgear). In examples the headgear connectors may be connected to the chassis portion 3210 by means of snap fasteners.

This method of connection between the headgear connectors and the chassis portion 3210 means that the chassis portion 3210 can be replaceable and the headgear connectors 3301, 3302 can be removed and reused with a new chassis portion 3210. For example, the headgear connectors 3301 and 3302 may be removed to enable replacement of the chassis portion 3210 and seal-forming structure 3100 together.

### 5.3.5.8.2 Foam Undercushion

Referring next to Fig. 52 in particular, the seal-forming structure 3100 comprises a foam undercushion 3225. In one form of the technology the foam undercushion 3225 is shaped to extend around the patient's mouth and beneath the anterior and lateral portions of a periphery of the alar base of the patient's nose when connected to the chassis portion 3210. In use, the foam undercushion 3225 supports the textile membrane portion 3220. The undercushion 3225 has at least one opening 3112 to allow air to flow to the patient's airways.

In examples the foam undercushion 3225 is made from a polyurethane, for example a thermoplastic polyurethane, or a soft thermoplastic elastomer. In examples the undercushion material is soft enough to cause substantially no discomfort when engaging the face.

In examples the foam from which the undercushion 3225 is made is substantially air impermeable, for example, the foam may be a skinned foam.

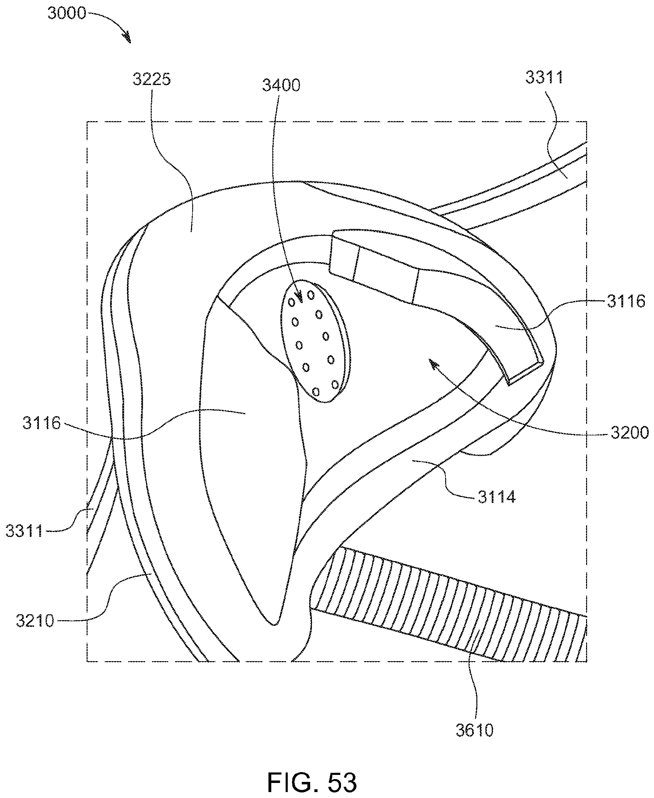

As shown in Fig. 53, the undercushion 3225 may have a substantially triangular form (e.g., prior to mounting to the chassis portion 3210) with one side 3114 of the triangle (e.g. corresponding to a below-the-mouth portion of the seal-forming structure) having a somewhat convex shape.

In other examples, for example as shown in Fig. 53, the substantially triangular form foam undercushion 3225 may comprise support portions 3116 which extend laterally inward to, in use, engage the corners of the patient's nose where the nose meets the face and/or to ensure the textile membrane portion 3220 is adequately supported in the area where the patient's nose meets the patient's face (e.g. to engage the patient's face at the corners of the patient's nose). In examples, the support portions 3116 may be compressed against the corners of the patient's nose in use, or against the patient's face at or proximate the corners of the nose, such as between the nasal alae and nasolabial sulci.

### 5.3.5.8.3 Textile membrane

As described elsewhere herein, the seal-forming structure 3100 may include a sealing flange utilizing a pressure assisted sealing mechanism. The sealing flange may comprise a textile membrane portion 3220, although in other examples alternative materials such as silicone may be used. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In the example shown in Fig. 55, for example, the patient interface 3000 comprises a textile membrane portion 3220 connected at its outer periphery 3140 (e.g. around the entire periphery) to a patient facing side of the foam undercushion 3225. In examples, the only connection between the textile membrane portion 3220 and the foam undercushion 3225 is at the outer periphery 3140 of the textile membrane portion 3220. When subject to treatment pressure inside the plenum chamber 3200, the textile membrane portion 3220 may "balloon" under the pressure, moving away from the undercushion 3225 towards the patient's face. This may cause the textile membrane portion 3220 to conform closely to the shape of the patient's face.

In examples, the textile membrane portion 3220 is configured to form a seal around at least anterior and lateral portions of a periphery of the alar base of the patient's nose and/or the sides of nose portion immediately adjacent the inferior periphery. In some examples, the membrane portion 3220 may engage a pronasale region of the user's nose, the nasal alae, and the lip superior. In some examples, the membrane portion 3220 may engage regions of the patient's face between the nasal alae and the nasolabial sulci.

In examples, the entire textile membrane portion 3220 is integrally formed from a single sheet of textile. For example, the textile membrane portion 3220 may comprise no seams.

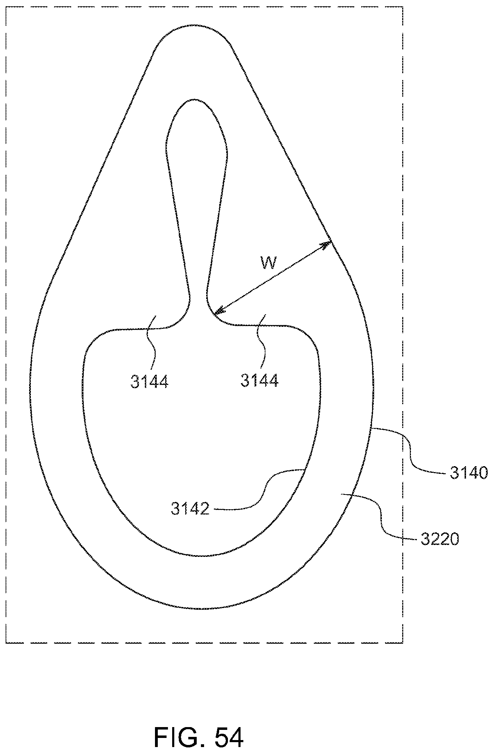

Referring next to Fig. 54 in particular, in one form, the textile membrane portion 3220 may comprise an outer periphery 3140 and an inner periphery 3142 which defines the edge of a hole. In examples a width W of the textile membrane portion 3220, measured between the outer periphery 3140 and the inner periphery 3142, may be greatest at the portions of the membrane intended to seal in the corners of the patient's nose where the patient's nose meets the face. In some examples the membrane portion 3220 may form inwardly extending "wings" 3144 in this area.

In some examples, in order to avoid blowout of the textile membrane portion 3220 at these portions, a connecting portion or "bridge" may be provided between them.

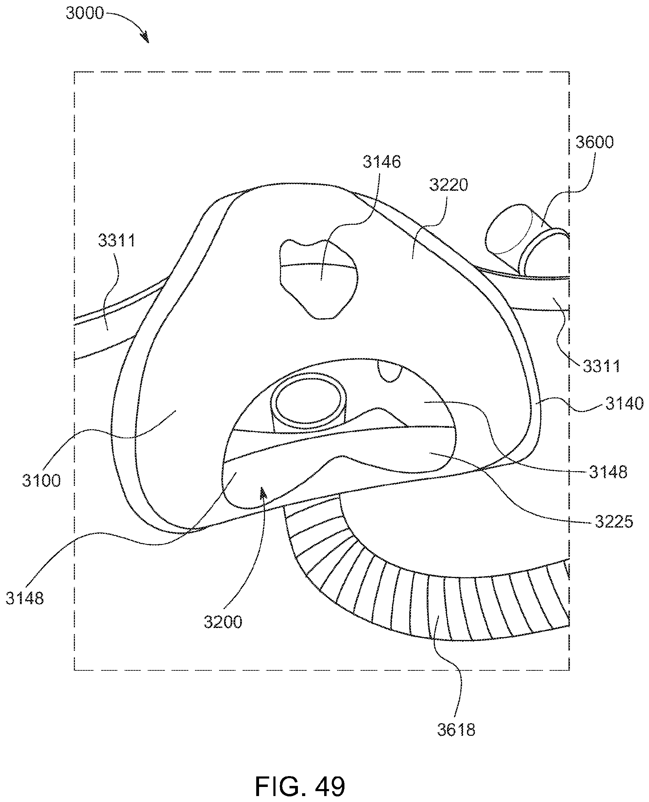

In other examples, for example as shown in Fig. 49, the textile membrane portion 3220 may comprise a first hole 3146 through which air can flow from the plenum chamber 3200 to both the patient's nares, and a second hole 3148 through which air can flow from the plenum chamber 3200 to the patient's mouth. The membrane portion 3220 may extend between the first and second hole 3146, 3148. In examples, only two holes 3146, 3148 are provided.

In examples having two holes, such as that shown in Fig. 49, the textile membrane portion 3220 may form a seal around the entrance to the patient's nares and a separate seal around the patient's mouth.

The textile membrane portion 3220 may be coated with an air impermeable coating, for example a silicone coating. In one example the silicone coating is substantially 0.05 mm thick and the textile membrane portion 3220, including the coating, is substantially 0.3 mm thick. The coating may be on a non-patient contacting side of the textile membrane portion 3220.

The textile membrane portion in the examples shown in Fig. 49 and 55 may comprise a knitted fabric. The membrane portion 3220 may be highly flexible so as to readily conform to the shape of the patient's face.

The highly compliant textile membrane portion 3220 that is activated with pressure allows for a robust seal. The treatment pressure when applied to the compliant textile membrane portion 3220 may allow it to form readily to varying face shapes.

In other examples of the present technology, the membrane portion 3220 may be as described elsewhere herein. In some examples the membrane portion 3220 may be formed from silicone. In some examples the membrane portion 3220 may be formed from silicone sheet. In some examples the membrane portion 3220 is formed from silicone that is unmoulded so as to not have a predetermined three-dimensional shape. More generally, the membrane portion 3220 may be formed from a process other than moulding and may be formed as a sheet.

Any feature of the textile membrane portion 3220 described with reference to Figs. 47-55 may be applied to a patient interface 3000 according to another example of the present technology, such as any of the examples disclosed herein. Likewise any feature of a membrane portion 3220 of any other patient interface 3000 described herein may be applied to the patient interface 3000 of Figs. 47-55.

### 5.3.5.9 Frame formed from resiliently flexible material

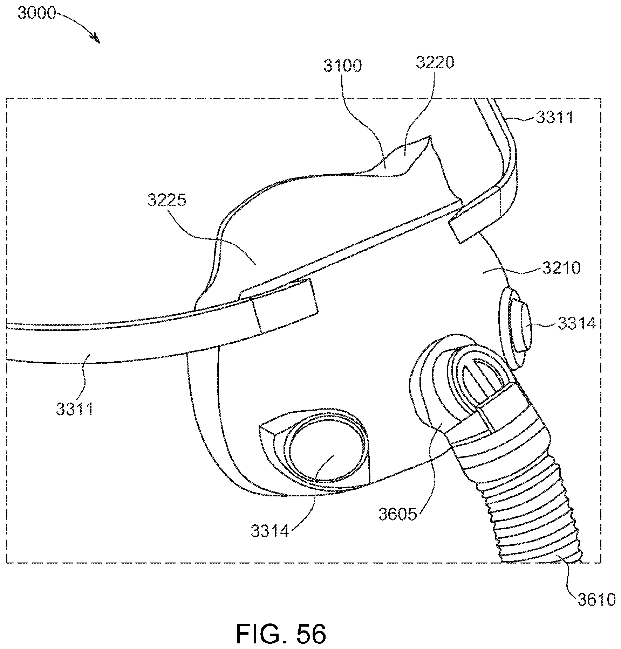

Examples of the patient interface 3000 of the present technology may be particularly light weight in comparison to some other patient interfaces of the prior art. For example, patient interfaces of the present technology may weigh less than 50 grams, more preferably less than 45 grams. For example, the patient interface 3000 of Figs. 56 to 58, including the chassis portion 3210, foam undercushion 3225, textile membrane portion 3220, upper arms 3311, lower headgear connection points 3314, and integrated short tube 3610, may weigh substantially 44.6 grams.

In one form of the technology, the chassis portion 3210 is formed from a resiliently flexible material. In examples the chassis portion 3210 is formed from a polymer, for example a biaxially-oriented polyethylene terephthalate, for example Mylar^{™}. The material may be less than 0.5 mm thick, for example 0.25 mm thick. In other examples the chassis portion 3210 may be formed from silicone. The chassis portion 3210 may alternatively be as described elsewhere herein according to another example of the present technology.

In examples, the chassis portion 3210 is curved such that it has a non-zero first principal curvature PC1 and a substantially zero second principal curvature PC2, as shown schematically in Fig. 48.

In examples, the patient interface 3000 is configured such that the second principal curvature PC2 is substantially parallel, in use, to a sagittal plane of the patient (e.g. it lies in the sagittal plane of the patient's head in use).

The chassis portion 3210 may be formed from a flat sheet of material which is held in the curved configuration by the seal-forming structure 3100 or the chassis portion 3210 may be inherently curved, for example it may be thermoformed, moulded or otherwise manufactured with an inherent curve. In examples the chassis portion 3210 may be stamped from a sheet of material, although other methods of forming the chassis portion 3210 are possible such as any other method or manner described herein.

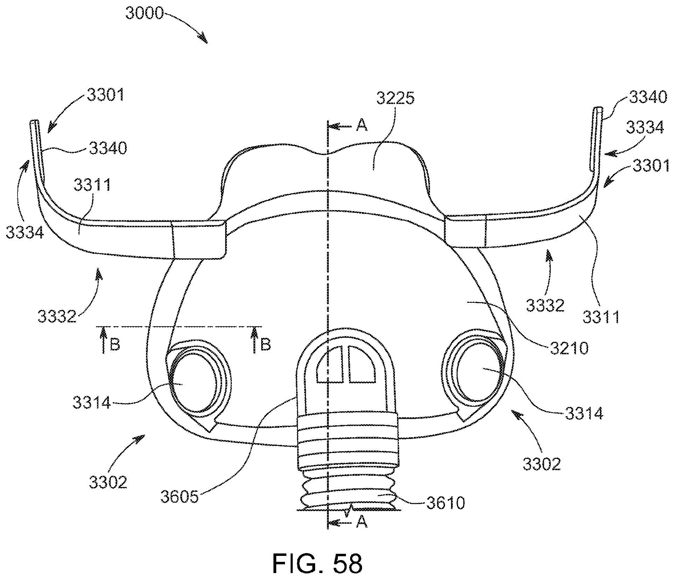

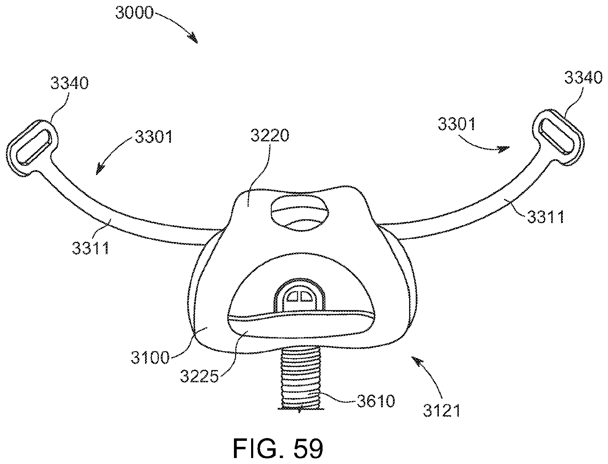

As shown in Figs. 58 and 59, in some forms of the technology the chassis portion 3210 is provided with a pair of superior headgear connectors 3301 and a pair of inferior headgear connectors 3302. In examples, the chassis portion 3210 is also provided with a inlet port connector 3605 for connection (for example releasable connection) to an air delivery tube, in use. In examples the frame 3240 is provided with at least one vent 3400 for gas washout, which may be as described elsewhere herein. The patient interface 3000 may also comprise an AAV, for example integrated in the inlet port connector 3605.

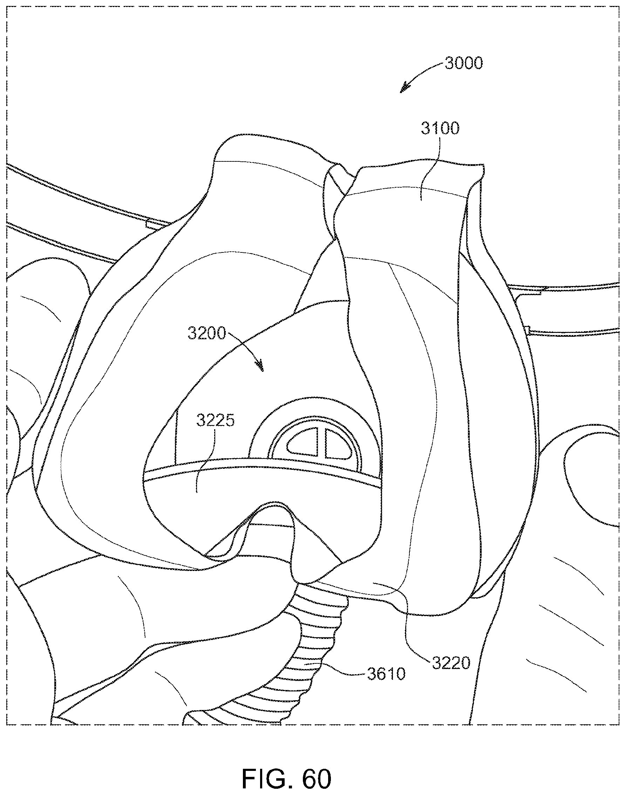

In examples in which the chassis portion 3210 is relatively thin and flexible, and has a substantially zero principal curvature substantially aligned with a sagittal plane, the chassis portion 3210 may flex relatively easily such that the magnitude of the first principal curvature PC1 can be varied when the patient interface 3000 is donned. For example, the magnitude of the first principal curvature PC1 may be relatively large (e.g. the chassis portion 3210 may be more curved - as shown in exaggerated form in Fig. 60, which depicts the patient interface 3000 being squeezed into a narrow configuration by the patient) when the patient interface 3000 is donned by a person having a relatively narrow face, but may be smaller (e.g. the chassis portion 3210 may be relatively "flatter" - for example, as seen in Fig. 59) when the patient interface 3000 is donned by a person having a relatively broad face. In this way, the patient interface 3000 may be adaptable to fit patients having a range of different size faces.

### 5.3.5.9.1 Foam Undercushion

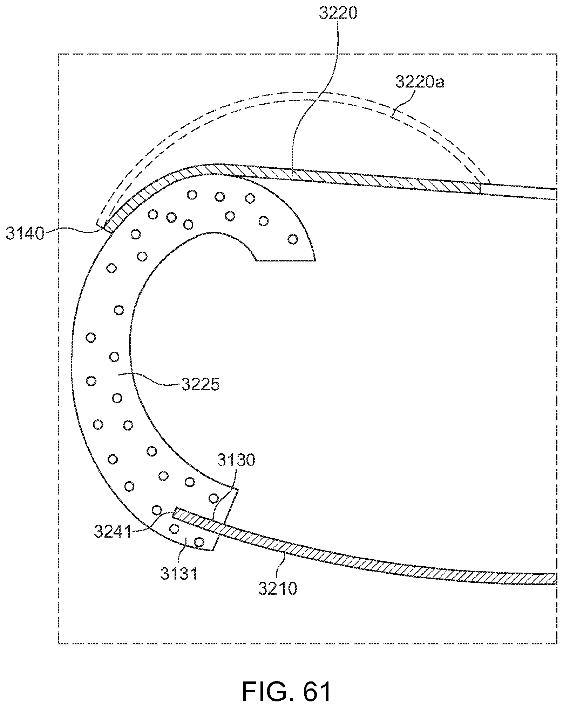

Referring next to Figs. 59 and 61, the seal-forming structure 3100 comprises an undercushion 3225 formed from foam. The foam undercushion 3225 is shaped to extend around the patient's mouth and around the anterior and lateral portions of a periphery of the alar base of the patient's nose. Examples of patient interfaces 3000 made in accordance with the present technology may be referred to as ultra-compact full face masks.

The foam undercushion 3225 is connected, preferably removably, to the chassis portion 3210. In one example the foam undercushion 3225 comprises a channel 3130, shown in Figs 61, 62 and 63, which is configured to engage an outer edge of the chassis portion 3210. In examples, the channel 3130 is configured to engage the entire outer periphery 3241 of the chassis portion 3210. When the patient wishes to separate the seal-forming structure 3100 from the chassis portion 3210 (for example to wash the seal-forming structure 3100 or to replace it), the foam undercushion 3225 can be peeled away from the chassis portion 3210.

The connection between the foam undercushion 3225 and the chassis portion 3210 is substantially airtight, that is, a seal is formed between the foam undercushion 3225 and the chassis portion 3210. In one example the channel 3130 is defined, in part, by a flexible lip 3131 at an edge of the undercushion 3225 which extends over the chassis portion 3210 and seals against the chassis portion 3210. In examples the lip 3131 is moulded into the undercushion 3225, utilising the undercushion material (e.g. foam). This may enable the connection between the chassis portion 3210 and undercushion 3225 to remain flexible.

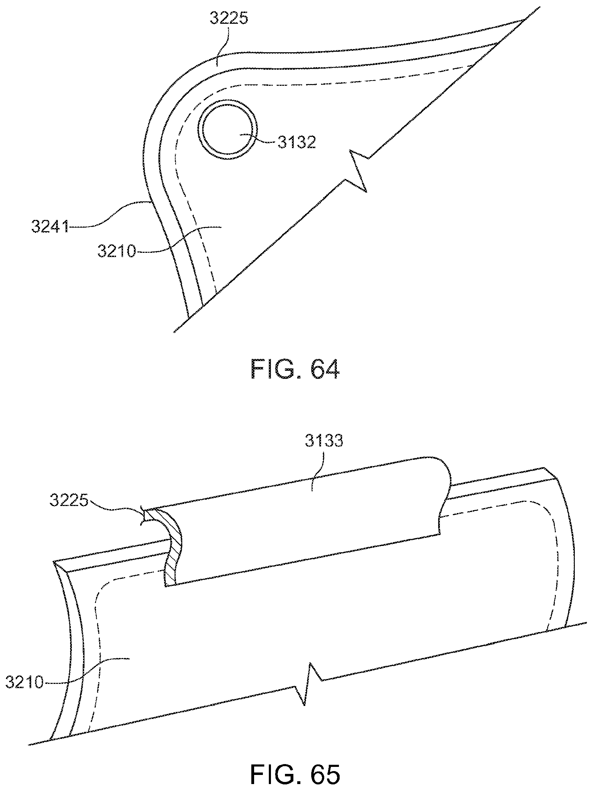

In examples, other connector mechanisms may be provided in addition to, or as an alternative to, the channel 3130 engaging portion referred to above. For example, in one embodiment one or more snap fasteners 3132 are provided to connect the foam undercushion 3225 to the chassis portion 3210, as shown schematically in Fig. 64. In another example, the foam undercushion 3225 may be provided with one or more substantially rigid clipping portions 3133 to engage the frame 3240, as shown schematically in Fig. 65.

In examples, the foam undercushion 3225 is shaped and configured, at least in some regions, to decrease its resistance to compression by the patient's face (e.g. to be less resistant to compression than in other regions). For example, as seen in Fig. 61, the foam undercushion 3225 may have a substantially "C" shape cross-section which is relatively easily compressed, although other suitable cross-section shapes may be used. In examples the cross-section shape may vary around the perimeter of the foam undercushion 3225, such that some portions of the foam undercushion 3225 (for example those at the nose) are more easily compressed than others (for example, those around the mouth).

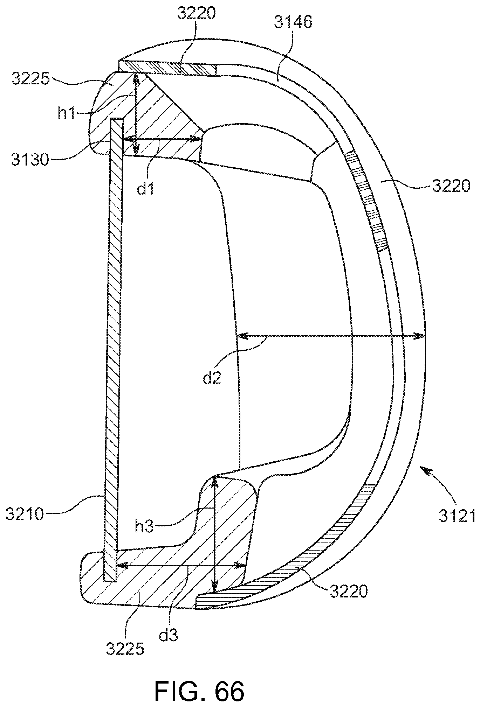

Referring next to Fig. 66, in one example the foam undercushion 3225 has a depth d1 of 14mm and a height h1 of 12mm in the area which receives the tip of the patient's nose. The undercushion 3225 in this particular example has a maximum depth d2 of 21mm at portions which engage the patient's cheeks at the sides of the patient's mouth. The width at this area may be 13mm. The undercushion 3225 may have a depth d3 of 15mm and a height h3 of 15mm at a portion below the patient's mouth. In examples the height, width and or depth of the undercushion 3225 may vary between the chassis portion 3210 and the textile membrane portion 3220. That is, the undercushion 3225 may not have a constant cross-section.

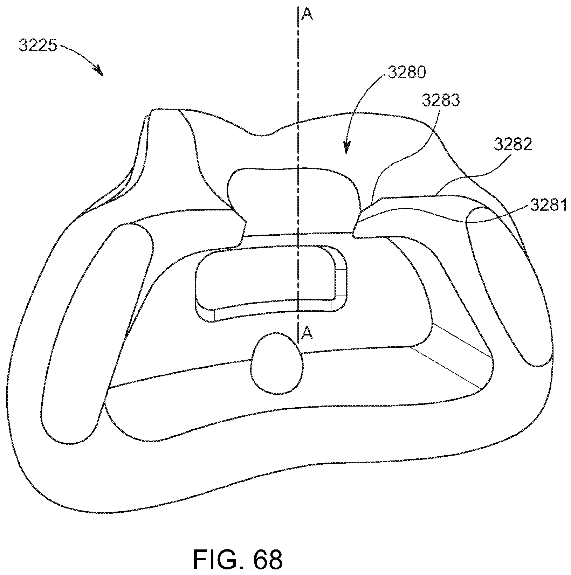

With reference to Fig. 68, which shows one example of a foam undercushion 3225 in isolation, an upper portion 3280 of the undercushion 3225, configured to engage the underside of the patient's nose, comprises a vertical portion 3281, a horizontal portion 3282 and chamfered or angled portion 3283 between the vertical and horizontal portions, and is formed as a surface of rotation about a superior-inferior axis A.

In examples the foam undercushion 3225 is made from a polyurethane, for example a thermoplastic polyurethane, or from a soft thermoplastic elastomer. Foam having the following properties may be suitable:
▪ Force Deflection: 95 +/- 20N @40% (Test spec: AS 2282.8 Method A)
   - Density: 54.5 +/- 2.5 kg/m3 (Test spec: AS 2282.3)
   - Air Permeability: <1.5 1/min @ 20cm H2O (R630-602 Permeability Test (Annular) Procedure).

Foam material having properties different to those above may be used in other examples of the present technology. In one form of the technology a polyethylene foam may be used.

In examples the foam may be substantially water impermeable so that the mask can dry adequately before use at night when washed earlier in the day.

In examples the undercushion material is soft enough to cause substantially no discomfort when engaging the face, but is firm enough to retain the frame and hold it in sealed manner when the patient interface is worn.

In examples the foam from which the undercushion is made is substantially air impermeable, for example, the foam may be a skinned foam.

In use, the foam undercushion 3225 supports the textile membrane portion 3220.

### 5.3.5.9.2 Textile membrane

Referring next to Figs. 59 and 61 in particular, in one form, the seal-forming structure 3100 includes a textile membrane portion 3220 which functions as a pressure assisted sealing mechanism. In use, the textile membrane portion 3220 can readily respond to a system positive pressure in the interior of the plenum chamber 3200 acting on its underside to urge it into tight sealing engagement with the face. The pressure assisted mechanism may act in conjunction with elastic tension in the positioning and stabilising structure 3300.

In examples the textile membrane portion 3220 is connected at its outer periphery 3140 to a patient facing side of the foam undercushion 3225, such that the membrane is substantially taut when not in use. In examples, the only connection between the textile membrane portion 3220 and the foam undercushion 3225 is at the outer periphery 3140 of the textile membrane portion 3220. When subject to treatment pressure inside the plenum chamber 3200, the textile membrane portion 3220 may "balloon" under the pressure, as indicated by 3220a. This may cause the textile membrane to conform closely to the shape of the patient's face.

In examples, the textile membrane portion 3220 is formed from a single sheet of textile. The textile membrane portion 3220 may comprise a first hole 3146 through which air can flow from the plenum chamber 3200 to both the patient's nares, and a second hole 3148 through which air can flow from the plenum chamber 3200 to the patient's mouth. The membrane portion 3220 may extend between the first and second holes 3146, 3148. In other examples (not shown), there may be a single hole in the membrane portion 3220 for both the patient's mouth and nares. In examples the membrane portion 3220 may effectively have only a single hole, but opposed lateral sides of the membrane portion 3220 at the edge of the hole may be connected by a bridging portion, for example a textile bridging portion. In other examples the membrane portion 3220 may comprise two nasal holes and an oral hole, each of the nasal holes being configured to fluidly communicate with a respective nare of the patient's nose in use.

The textile membrane portion 3220 may have a coating and thickness as described elsewhere herein, for example as described with reference to the examples shown in Figs. 47-55 or with reference to any other example herein.

The textile membrane portion 3220 may comprise a knitted fabric. The membrane portion 3220 may be highly flexible so as to readily conform to the shape of the patient's face. In examples the textile may comprise polyamide (e.g. nylon), polyester and/or elastane (e.g. spandex).

In one example a textile with the following properties may be used:
- Single jersey knit;
- Content: polyamide 80%, elastane 20%;
- Density: 136 course per inch, 75 wale per inch;
- Weight: 105 g/m2; and
- Elongation at 1.5kg: Length - 80-108%, Width 93-126%.

In other examples the textile material may have properties that differ from the above properties.

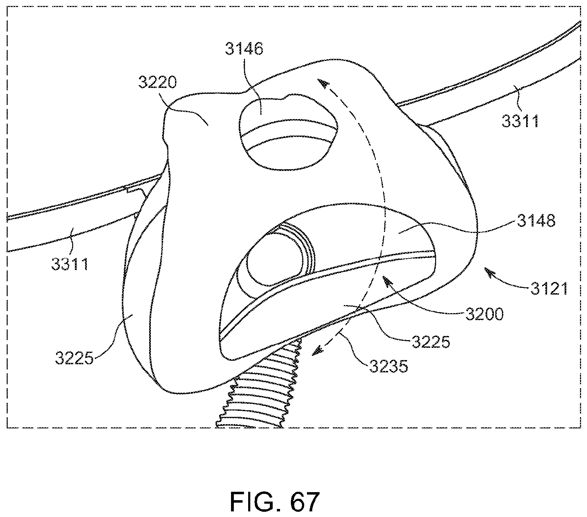

In examples the chassis portion 3210 and foam undercushion 3225 are shaped such that the patient facing side 3121 of the textile membrane portion 3220 lies on a continuously convex curve 3235 when viewed in cross-section through a sagittal plane (for example a central sagittal plane) as indicated in Fig. 67.

For example, the textile membrane portion 3220 may have no secondary geometrical features which could create creases. This allows the membrane to be held in a configuration in which no creases are created when the patient interface 3000 is not in use. When the patient interface 3000 is applied to the face, the membrane portion 3220 flexes and forms to the face, particularly around the peripheries of the nose and mouth holes, as these may be the first points of engagement.

The highly compliant textile membrane portion 3220 that is activated with pressure may allow for a robust seal. The treatment pressure when applied to the compliant textile membrane portion 3220 may allow it to form readily to varying face shapes. The compliant nature of the textile membrane and/or the large area of the membrane in contact with the patient's face may assist in reducing areas of increased pressure between the seal-forming structure 3100 and the patient's face, as may be present with less compliant seal-forming structures of the prior art. In some examples the textile may be of a fine knit. The surface of the textile may be low friction in order to allow the patient to easily position the cushion module 3150 when donning the patient interface 3000, and to ensure that the fabric does not remain creased against the patient's skin.

### 5.3.5.9.3 Connectors for positioning and stabilising structure

As shown in Figs. 58 and 59, in examples, connectors for connecting the positioning and stabilising structure 3300 to the patent interface 3000 are provided to the chassis portion. In examples the chassis portion 3210 is provided with a pair of superior headgear connectors 3301 and a pair of inferior headgear connectors 3302. The superior headgear connectors 3301 and inferior headgear connectors 3302 may be as described above with reference to the example shown in Figs. 47-55.

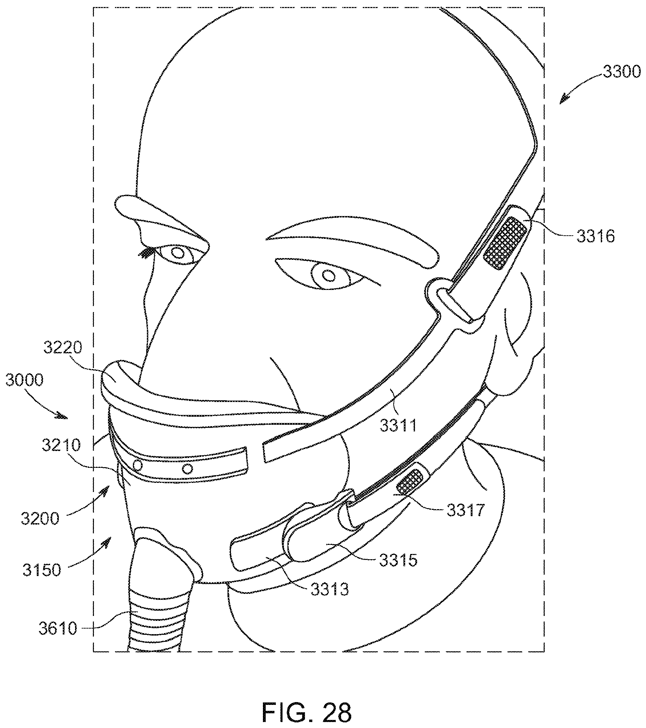

While in the example shown in Figs. 47-55 the patient interface 3000 comprises lower arms 3313 having magnetic connection points at ends thereof, in the example shown in Figs. 56-67 the chassis portion 3210 comprises lower headgear connection points 3314 in the form of magnets attached directly to the chassis portion 3210. The lower headgear connection points 3314 in this example are configured to magnetically connect to headgear connectors provided to lower straps of a positioning and stabilising structure 3300. The patient interfaces 3000 shown in Figs. 47-68 may comprise a positioning and stabilising structure 3300 as shown in Figs. 27 and 28, by way of example only.

The upper arms 3311 may comprise laterally extending portions 3332 and posteriorly extending portions 3334. The posteriorly extending portions 3334 may also extend in a superior direction, in use.

### 5.3.6 Decoupling structure(s)

In one form the patient interface 3000 includes at least one decoupling structure, for example, a swivel or a ball and socket.

### 5.3.7 Connection port

Connection port 3600 allows for connection to the air circuit 4170.

### 5.3.8 Forehead support

In one form, the patient interface 3000 includes a forehead support 3700.

### 5.3.9 Anti-asphyxia valve

In one form, the patient interface 3000 includes an anti-asphyxia valve.

### 5.3.10 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller, a therapy device controller, a pressure generator 4140, one or more protection circuits, memory, transducers 4270, a data communication interface and one or more output devices. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications : U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller.

In other forms, a pressure generator 4140 may be a piston-driven pump, a pressure regulator connected to a high pressure source (e.g. compressed air reservoir), or a bellows.

### 5.4.1.4 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device algorithms

As mentioned above, in some forms of the present technology, a central controller of the RPT device 4000 may be configured to implement one or more algorithms expressed as computer programs stored in a non-transitory computer readable storage medium, such as memory. The algorithms are generally grouped into groups referred to as modules.

In other forms of the present technology, some portion or all of the algorithms may be implemented by a controller of an external device such as the local external device or the remote external device. In such forms, data representing the input signals and / or intermediate algorithm outputs necessary for the portion of the algorithms to be executed at the external device may be communicated to the external device via the local external communication network or the remote external communication network. In such forms, the portion of the algorithms to be executed at the external device may be expressed as computer programs, such as with processor control instructions to be executed by one or more processor(s), stored in a non-transitory computer readable storage medium accessible to the controller of the external device. Such programs configure the controller of the external device to execute the portion of the algorithms.

In such forms, the therapy parameters generated by the external device via the therapy engine module (if such forms part of the portion of the algorithms executed by the external device) may be communicated to the central controller to be passed to the therapy control module.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

### 5.6 HUMIDIFIER

### 5.6.1 Humidifier overview

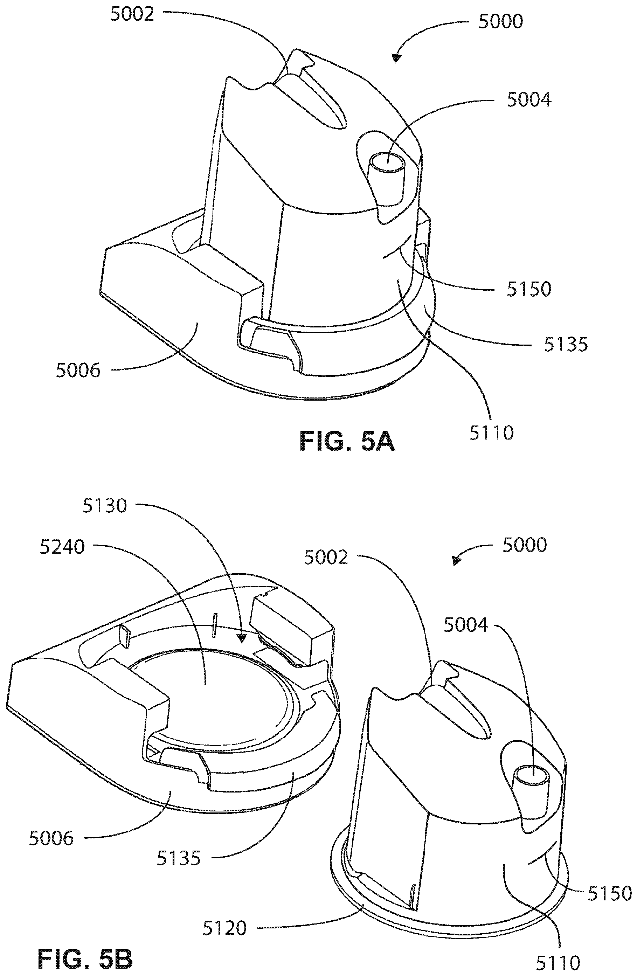

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.6.2 Humidifier components

### 5.6.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.2.5 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072.

In some forms, the heating element 5240 may be provided in the humidifier base 5006 where heat may be provided to the humidifier reservoir 5110 primarily by conduction as shown in Fig. 5B.

### 5.7 BREATHING WAVEFORMS

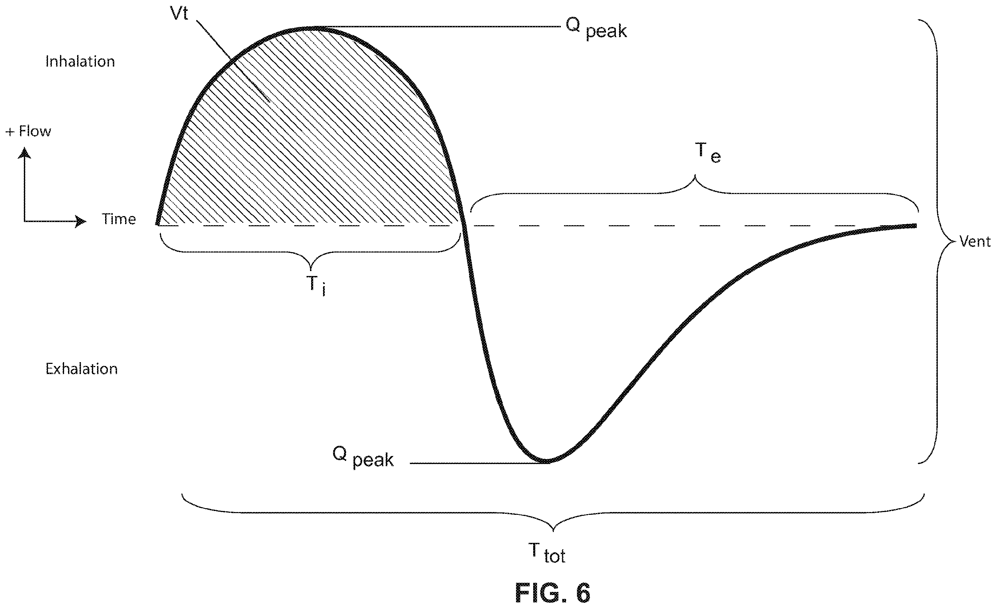

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.8 GLOSSARY

For the purposes of the present technology disclosure, in certain forms of the present technology, one or more of the following definitions may apply. In other forms of the present technology, alternative definitions may apply.

### 5.8.1 General

*Air*: In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted (acoustic)*: Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic)*: Radiated noise in the present document refers to noise which is carried to the patient by the ambient air. In one form, radiated noise may be quantified by measuring sound power/pressure levels of the object in question according to ISO 3744.

*Noise, vent (acoustic)*: Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen*: Medical oxygen is defined as oxygen enriched air with an oxygen concentration of 80% or greater.

*Patient:* A person, whether or not they are suffering from a respiratory condition.

*Pressure*: Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy*: The application of a supply of air to an entrance to the airways at a treatment pressure that is typically positive with respect to atmosphere.

*Ventilator*: A mechanical device that provides pressure support to a patient to perform some or all of the work of breathing.

### 5.8.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 5.8.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness* (or *rigidity)* of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

*Floppy* structure or component: A structure or component that will change shape, e.g. bend, when caused to support its own weight, within a relatively short period of time such as 1 second.

*Rigid* structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH2O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.8.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Expiratory portion* of a breathing cycle: The period from the start of expiratory flow to the start of inspiratory flow.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(3272) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion* of a breathing cycle: The period from the start of inspiratory flow to the start of expiratory flow will be taken to be the inspiratory portion of a breathing cycle.

### 5.8.3 Anatomy

### 5.8.3.1 Anatomy of the face

Ala: the external outer wall or "wing" of each nostril (plural: alar)

Alar angle:
Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### 5.8.3.2 Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 5.8.3.3 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.8.4 Patient interface

*Anti-asphyxia valve (AAV)*: The component or sub-assembly of a mask system that, by opening to atmosphere in a failsafe manner, reduces the risk of excessive CO2 rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

### Tie (noun): A structure designed to resist tension.

*Vent:* (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.8.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

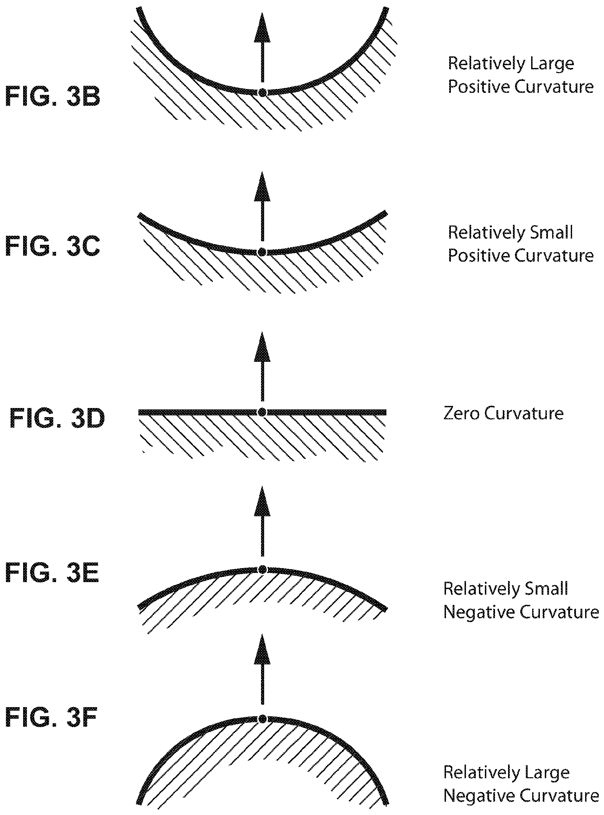

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.8.5.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 5.8.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.8.5.3 Space curves

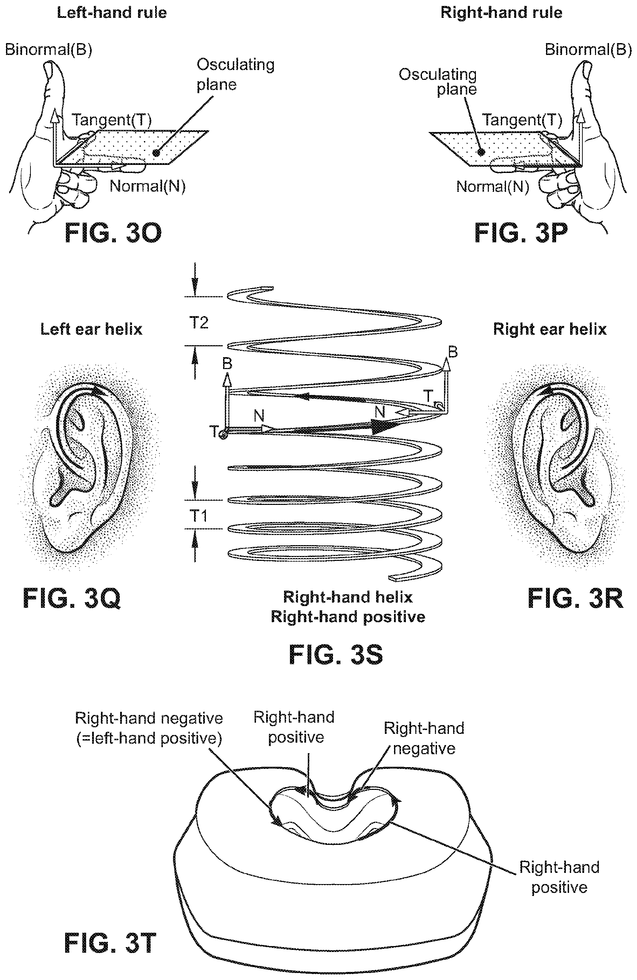

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the Tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.8.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.9 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.10 SELECTED REFERENCE SIGNS LIST

- 3000: Patient interface
- 3100: Seal-forming structure
- 3101: Nasal portion of seal-forming structure
- 3102: Oral portion of seal-forming structure
- 3112: Opening
- 3114: Side
- 3116: Support portions
- 3130: Channel
- 3131: Lip
- 3140: Outer periphery of membrane portion
- 3142: Inner periphery of membrane portion
- 3144: Wing
- 3146: First hole
- 3148: Second hole
- 3150: Cushion module
- 3160: Nasal cushion module
- 3165: Oronasal connector
- 3170: Oral cushion module
- 3200: Plenum Chamber
- 3210: Chassis portion
- 3212: Laterally projecting connection portions
- 3214: Connectors
- 3216: Nasal chassis portion
- 3217: Oral chassis portion
- 3218: Cavity
- 3220: Membrane portion
- 3225: Undercushion
- 3241: Outer periphery of chassis portion
- 3250: Blank
- 3252: Lower edge of blank
- 3256: Opening
- 3258: Fold line
- 3260: Lateral edges
- 3262: Flap
- 3264: Inner surface
- 3266: Plastically deformable element
- 3268: Inferior wall
- 3270: Anterior wall
- 3272: Cutout
- 3274: Location
- 3276: Central portion
- 3280: Upper portion of undercushion
- 3281: Vertical portion
- 3282: Horizontal portion
- 3283: Angled portion
- 3300: Positioning and stabilising structure
- 3301: Superior headgear connector
- 3302: Inferior headgear connector
- 3311: Upper arm
- 3312: Connector
- 3313: Lower arm
- 3314: Lower headgear connection point
- 3315: Headgear clip
- 3316: Upper strap
- 3317: Lower strap
- 3318: Crown strap
- 3319: Backstrap
- 3310: Headgear strap portions
- 3320: Frame
- 3340: Strap connection portion
- 3350: Gas delivery tube
- 3400: Vent
- 3410: Vent module
- 3600: Connection port
- 3605: Inlet port connector
- 3610: Short tube

## Claims

1. A patient interface (3000) comprising:
a chassis portion (3210) at least partially forming a plenum chamber (3200) pressurisable to a therapeutic pressure of at least 4 cmH2O above ambient air pressure, said plenum chamber (3200) including a plenum chamber inlet port sized and structured to receive a flow of air at the therapeutic pressure for breathing by a patient;
a seal-forming structure (3100) comprising a pair of nasal pillows, each nasal pillow being constructed and arranged to form a seal with a respective naris of the nose of a patient, each nasal pillow having a hole therein such that the flow of air at said therapeutic pressure is delivered to at least the patient's nares, the seal-forming structure (3100) constructed and arranged to maintain said therapeutic pressure in the plenum chamber (3200) throughout the patient's respiratory cycle in use;
a membrane portion (3220) connected to the chassis portion (3210) and at least partially forming the plenum chamber (3200), wherein the nasal pillows are supported on the membrane portion (3220) and the membrane portion (3220) is constructed and arranged to be flexible to allow for relative movement between the nasal pillows and the chassis portion (3210) in use, and wherein the membrane portion (3220) does not have a predetermined three-dimensional shape in the absence of positive pressure with respect to atmospheric pressure in the plenum chamber (3200), optionally wherein the membrane portion (3220) is stretchable; and
a vent (3400) to allow a continuous flow of gases exhaled by the patient from an interior of the plenum chamber (3200) to ambient, said vent (3400) being sized and shaped to maintain the therapeutic pressure in the plenum chamber (3200) in use;
wherein the patient interface (3000) is configured to allow the patient to breathe from ambient through their mouth in the absence of a flow of pressurised air through the plenum chamber inlet port, or the patient interface (3000) is configured to leave the patient's mouth uncovered.

2. The patient interface (3000) of claim 1, wherein the membrane portion (3220) is constructed and arranged to stretch during inflation upon pressurisation of the plenum chamber (3200) to the therapeutic pressure in use.

3. The patient interface (3000) of claim 1 or claim 2, wherein the membrane portion (3220) is constructed and arranged to inflate to conform to one or more portions of the patient's nose in use.

4. The patient interface (3000) of any one of claims 1-3, wherein the membrane portion (3220) is constructed and arranged to inflate to urge each of the nasal pillows towards a respective one of the patient's nares in use.

5. The patient interface (3000) of any one of claims 1-4, wherein the membrane portion (3220) is configured to engage the patient's lip superior in use.

6. The patient interface (3000) of any one of claims 1-5, wherein the membrane portion (3220) is formed at least partially from a textile material, optionally wherein the membrane portion (3220) comprises a textile layer and an air impermeable layer.

7. The patient interface (3000) of any one of claims 1-5, wherein the membrane portion (3220) is formed from an elastomer, optionally wherein the membrane portion is formed from silicone.

8. The patient interface (3000) of claim 7, wherein the membrane portion (3220) is in the form of a sheet connected to the chassis portion (3210) at edges of the membrane portion (3220).

9. The patient interface (3000) of any one of claims 1-8, wherein the chassis portion (3210) is formed from an elastomeric material.

10. The patient interface (3000) of any one of claims 1-9, wherein each of the nasal pillows is stalkless.

11. The patient interface (3000) of any one of claims 1-10, wherein each of the nasal pillows is formed from an elastomeric material.

12. The patient interface (3000) of any one of claims 1-10, wherein each of the nasal pillows is formed at least partially from a textile material.

13. The patient interface (3000) of any one of claims 1-12, wherein each of the nasal pillows is formed from a single wall.

14. The patient interface (3000) of any one of claims 1-12, wherein each of the nasal pillows comprises a double wall structure.

15. The patient interface (3000) of any one of claims 1-14, wherein the seal-forming structure (3100) further comprises an oral portion (3102) configured to form a seal around the patient's mouth such that the flow of air at said therapeutic pressure is delivered to the patient's mouth, optionally wherein the membrane portion (3220) forms the oral portion (3102) of the seal-forming structure (3100).

## Patentansprüche

1. Patientenschnittstelle (3000), aufweisend:
einen Chassisabschnitt (3210), der mindestens teilweise eine Luftkammer (3200) bildet, die auf einen Behandlungsdruck von mindestens 4 cmH2O über dem Umgebungsluftdruck gebracht werden kann, wobei die Luftkammer (3200) eine Luftkammereinlassöffnung aufweist, die zum Aufnehmen eines Luftstroms mit dem Behandlungsdruck zum Atmen durch einen Patienten bemessen und ausgebildet ist,
eine dichtungsbildende Struktur (3100), aufweisend ein Paar von Nasenpolstern, wobei die Nasenpolster jeweils zum Bilden einer Dichtung mit einem jeweiligen Nasenlochbereich eines Patienten aufgebaut und angeordnet sind, wobei die Nasenpolster jeweils eine Öffnung aufweisen, sodass der Luftstrom mit dem Behandlungsdruck mindestens an die Nasenlöcher des Patienten abgegeben wird, wobei die dichtungsbildende Struktur (3100) so aufgebaut und angeordnet ist, dass sie im Gebrauch den Behandlungsdruck in der Luftkammer (3200) während des gesamten Atemzyklus des Patienten aufrechterhält,
einen Membranabschnitt (3220), der mit dem Chassisabschnitt (3210) verbunden ist und mindestens teilweise die Luftkammer (3200) bildet, wobei die Nasenpolster auf dem Membranabschnitt (3220) gelagert sind und der Membranabschnitt (3220) flexibel aufgebaut und angeordnet ist, um im Gebrauch eine Relativbewegung zwischen den Nasenpolstern und dem Chassisabschnitt (3210) zu ermöglichen, und wobei der Membranabschnitt (3220) in Abwesenheit eines Überdrucks gegenüber dem Atmosphärendruck in der Luftkammer (3200) keine vorbestimmte dreidimensionale Form aufweist, wobei der Membranabschnitt (3220) gegebenenfalls dehnbar ist; und
eine Abzugsöffnung (3400), um einen kontinuierlichen Strom der vom Patienten ausgeatmeten Gase aus dem Inneren der Luftkammer (3200) in die Umgebung zu ermöglichen,
wobei die Abzugsöffnung (3400) im Gebrauch zum Aufrechterhalten des Behandlungsdrucks in der Luftkammer (3200) bemessen und geformt ist.
wobei die Patientenschnittstelle (3000) dazu konfiguriert ist, dem Patienten zu ermöglichen, bei Abwesenheit eines Stroms von unter Druck stehender Luft durch eine Luftkammereinlassöffnung durch den Mund aus der Umgebung zu atmen oder die Patientenschnittstelle (3000) dazu konfiguriert ist, den Mund des Patienten unbedeckt zu lassen.

2. Patientenschnittstelle (3000) nach Anspruch 1, wobei der Membranabschnitt (3220) so aufgebaut und angeordnet ist, dass er sich beim Aufblasen dehnt, wenn die Luftkammer (3200) im Gebrauch auf den Behandlungsdruck gebracht wird.

3. Patientenschnittstelle (3000) nach Anspruch 1 oder Anspruch 2, wobei der Membranabschnitt (3220) so aufgebaut und angeordnet ist, dass er sich aufbläst, um sich im Gebrauch an einen oder mehrere Bereiche der Nase des Patienten anzupassen.

4. Patientenschnittstelle (3000) nach einem der Ansprüche 1 bis 3, wobei der Membranabschnitt (3220) so aufgebaut und angeordnet ist, dass er sich aufbläst, um die Nasenpolster im Gebrauch jeweils zu einem jeweiligen Nasenloch des Patienten zu drücken.

5. Patientenschnittstelle (3000) nach einem der Ansprüche 1 bis 4, wobei der Membranabschnitt (3220) dazu konfiguriert ist, im Gebrauch die Oberlippe des Patienten in Eingriff zu nehmen.

6. Patientenschnittstelle (3000) nach einem der Ansprüche 1 bis 5, wobei der Membranabschnitt (3220) mindestens teilweise aus einem Textilmaterial gebildet ist, wobei der Membranabschnitt (3220) gegebenenfalls eine Textilschicht und eine luftundurchlässige Schicht aufweist.

7. Patientenschnittstelle (3000) nach einem der Ansprüche 1 bis 5, wobei der Membranabschnitt (3220) aus einem Elastomer gebildet ist, wobei der Membranabschnitt gegebenenfalls aus Silikon gebildet ist.

8. Patientenschnittstelle (3000) nach Anspruch 7, wobei der Membranabschnitt (3220) die Form einer Folie aufweist, die an den Rändern des Membranabschnitts (3220) mit dem Chassisabschnitt (3210) verbunden ist.

9. Patientenschnittstelle (3000) nach einem der Ansprüche 1 bis 8, wobei der Chassisabschnitt (3210) aus einem Elastomermaterial gebildet ist.

10. Patientenschnittstelle (3000) nach einem der Ansprüche 1 bis 9, wobei die Nasenpolster jeweils aufsitzend sind.

11. Patientenschnittstelle (3000) nach einem der Ansprüche 1 bis 10, wobei die Nasenpolster jeweils aus einem Elastomermaterial gebildet sind.

12. Patientenschnittstelle (3000) nach einem der Ansprüche 1 bis 10, wobei die Nasenpolster jeweils mindestens teilweise aus einem Textilmaterial gebildet sind.

13. Patientenschnittstelle (3000) nach einem der Ansprüche 1 bis 12, wobei die Nasenpolster jeweils aus einer einzelnen Wand gebildet sind.

14. Patientenschnittstelle (3000) nach einem der Ansprüche 1 bis 12, wobei die Nasenpolster jeweils eine Doppelwandstruktur aufweisen.

15. Patientenschnittstelle (3000) nach einem der Ansprüche 1 bis 14, wobei die dichtungsbildende Struktur (3100) ferner einen Mundabschnitt (3102) aufweist, der zum Bilden einer Abdichtung um den Mund des Patienten konfiguriert ist, sodass der Luftstrom mit dem Behandlungsdruck in den Mund des Patienten geleitet wird, wobei der Membranabschnitt (3220) gegebenenfalls den Mundabschnitt (3102) der dichtungsbildenden Struktur (3100) bildet.

## Revendications

1. Interface patient (3000) comprenant:
une partie châssis (3210) formant au moins partiellement une chambre de distribution (3200) pouvant être mise sous pression à une pression thérapeutique d'au moins 4 cmH2O au-dessus de la pression de l'air environnant, ladite chambre de distribution (3200) incluant un orifice d'entrée de chambre de distribution dimensionné et structuré pour recevoir un flux d'air à la pression thérapeutique pour la respiration par un patient;
une structure formant joint d'étanchéité (3100) comprenant une paire de coussinets narinaires, chaque coussinet narinaire étant construit et agencé pour former un joint d'étanchéité avec une narine respective du nez d'un patient, chaque coussinet narinaire ayant un orifice de sorte que le flux d'air à ladite pression thérapeutique est délivré à au moins les narines du patient, la structure formant joint d'étanchéité (3100) construite et agencée pour maintenir ladite pression thérapeutique dans la chambre de distribution (3200) pendant tout le cycle respiratoire du patient lors de l'utilisation;
une partie membrane (3220) reliée à la partie châssis (3210) et formant au moins partiellement la chambre de distribution (3200), dans laquelle les coussinets narinaires sont supportés sur la partie membrane (3220) et la partie membrane (3220) est construite et agencée pour être flexible pour permettre un mouvement relatif entre les coussinets narinaires et la partie châssis (3210) lors de l'utilisation, et dans laquelle la partie membrane (3220) ne présente pas de forme tridimensionnelle prédéterminée en l'absence de pression positive par rapport à la pression atmosphérique dans la chambre de distribution (3200), éventuellement dans laquelle la partie membrane (3220) est extensible; et
un orifice d'évent (3400) pour permettre un flux continu des gaz expirés par le patient d'un intérieur de la chambre de distribution (3200) à l'environnement, ledit orifice d'évent (3400) étant dimensionné et façonné pour maintenir la pression thérapeutique dans la chambre de distribution (3200) lors de l'utilisation;
dans laquelle l'interface patient (3000) est configurée pour permettre au patient de respirer à partir de l'environnement par la bouche en l'absence d'un flux d'air sous pression à travers l'orifice d'entrée de la chambre de distribution, ou l'interface patient (3000) est configurée pour laisser la bouche du patient non couverte.

2. Interface patient (3000) selon la revendication 1, dans laquelle la partie membrane (3220) est construite et agencée pour s'étirer pendant le gonflage lors de la mise sous pression de la chambre de distribution (3200) jusqu'à la pression thérapeutique lors de l'utilisation.

3. Interface patient (3000) selon la revendication 1 ou la revendication 2, dans laquelle la partie membrane (3220) est construite et agencée pour se gonfler pour épouser la forme d'une ou plusieurs parties du nez du patient lors de l'utilisation.

4. Interface patient (3000) selon l'une quelconque des revendications 1 à 3, dans laquelle la partie membrane (3220) est construite et agencée pour se gonfler pour pousser chacun des coussinets narinaires vers l'une respective des narines du patient lors de l'utilisation.

5. Interface patient (3000) selon l'une quelconque des revendications 1 à 4, dans laquelle la partie membrane (3220) est configurée pour entrer en contact avec la lèvre supérieure du patient lors de l'utilisation.

6. Interface patient (3000) selon l'une quelconque des revendications 1 à 5, dans laquelle la partie membrane (3220) est formée, au moins partiellement, d'un matériau textile, éventuellement dans laquelle la partie membrane (3220) comprend une couche textile et une couche imperméable à l'air.

7. Interface patient (3000) selon l'une quelconque des revendications 1 à 5, dans laquelle la partie membrane (3220) est formée d'un élastomère, éventuellement dans laquelle la partie membrane est formée de silicone.

8. Interface patient (3000) selon la revendication 7, dans laquelle la partie membrane (3220) se présente sous la forme d'une feuille reliée à la partie châssis (3210) au niveau de bords de la partie membrane (3220).

9. Interface patient (3000) selon l'une quelconque des revendications 1 à 8, dans laquelle la partie châssis (3210) est formée d'un matériau élastomère.

10. Interface patient (3000) selon l'une quelconque des revendications 1 à 9, dans laquelle chacun des coussinets narinaires est sans tige.

11. Interface patient (3000) selon l'une quelconque des revendications 1 à 10, dans laquelle chacun des coussinets narinaires est formé d'un matériau élastomère.

12. Interface patient (3000) selon l'une quelconque des revendications 1 à 10, dans laquelle chacun des coussinets narinaires est formé, au moins partiellement, d'un matériau textile.

13. Interface patient (3000) selon l'une quelconque des revendications 1 à 12, dans laquelle chacun des coussinets narinaires est formé d'une seule paroi.

14. Interface patient (3000) selon l'une quelconque des revendications 1 à 12, dans laquelle chacun des coussinets narinaires comprend une structure à double paroi.

15. Interface patient (3000) selon l'une quelconque des revendications 1 à 14, dans laquelle la structure formant joint d'étanchéité (3100) comprend en outre une partie buccale (3102) configurée pour former un joint d'étanchéité autour de la bouche du patient de sorte que le flux d'air à ladite pression thérapeutique est délivré à la bouche du patient, éventuellement dans laquelle la partie membrane (3220) forme la partie buccale (3102) de la structure formant joint d'étanchéité (3100).
